# EUROPEAN PATENT SPECIFICATION

(11) **EP 1 585 727 B1**
(45) Date of publication and mention of the grant of the patent: **26.05.2010**
(21) Application number: 03810458.4
(22) Date of filing: 03.11.2003
(51) Int. Cl.: C07D 209/30, C07D 401/06, C07D 405/06, C07D 413/06, C07D 401/12, A61K 31/404, C07D 417/06, C07D 409/12, C07D 405/12, C07D 403/12, C07D 403/06, A61P 5/24

(54) **INDOLES USEFUL IN THE TREATMENT OF ANDROGEN-RECEPTOR RELATED DISEASES**
INDOLE, DIE SICH ZUR BEHANDLUNG VON MIT DEM ANDROGENREZEPTOR IN ZUSAMMENHANG STEHENDEN KRANKHEITEN EIGNEN
INDOLES UTILES DANS LE TRAITEMENT DE MALADIES LIEES AU RECEPTEUR D'ANDROGENE

(30) Priority: 07.11.2002 US 424579 P; 07.11.2002 EP 02079648
(43) Date of publication of application: 19.10.2005
(73) Proprietor: N.V. Organon, 5349 AB Oss (NL)
(72) Inventor: Hermkens, Pedro Harold Han, 5340 BH Oss (NL); Stock, Herman Thijs, 5340 BH Oss (NL); Teerhuis, Neeltje Miranda, 5340 BH Oss (NL); Lommerse, Johannes Petrus Maria, 5340 BH Oss (NL); Van der Louw, Jaap, 5340 BH OSS (NL)
(74) Representative: Kraak, Hajo
(86) International application number: PCT/EP2003/050783
(87) International publication number: WO 2004/041782

(56) References cited:
- WO-A-03/064387
- DE-A- 2 559 211
- US-A- 5 767 139

## Description

The invention relates to indole derivatives, their preparation and their use for the preparation of medicaments for the treatment of androgen-receptor related conditions, disorders or diseases and other androgen related treatments.

US 5 767 139 relates to indole derivates and their use as testosterone-5α-reductose inbihitors.

Compounds possessing androgenic activity are useful in the treatment of men with low endogenous levels circulating androgenic hormones or men with suppressed androgenic effects. Such treatments are prescribed to older men, to hypogonadal men or men treated with progestagens for male contraception. In addition, potent androgens suppress spermatogenesis and can be used as male contraceptives.

It is thus important to obtain compounds with high affinity for the androgen receptor. Non-steroidal compounds with high affinity for the androgen receptor are particularly useful since they may have different tissue distribution characteristics than steroidal androgens and can be designed by proper choice of substituents to be more or less selective for certain tissues. For example, an action in the brain is usually prevented when compounds are strongly hydrophilic or carry an ionic charge.

The subject invention provides non-steroidal compounds with affinity for the androgen receptor. These compounds are potentially useful for the treatment of androgen-receptor related disorders or disorders which can be treated with androgens. The compounds of the subject invention have a structure according to formula I: 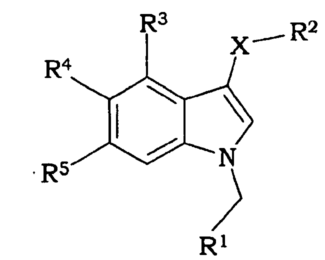 wherein
X is S, SO or SO₂;
R¹ is a 5- or 6-membered monocyclic, hetero- or homocyclic, saturated or unsaturated ring structure optionally substituted with one or more substituents selected from the group consisting of halogen, CN, (1 C-4C)fluoroalkyl, nitro, (1C-4C)alkyl, (1C-4C)alkoxy or (1C-4C)fluoroalkoxy;
R² is 2-nitrophenyl, 2-cyanophenyl, 2-hydroxymethyl-phenyl, pyridin-2-yl, pyridin-2-yl-N-oxide, 2-benzamide, 2-benzoic acid methyl ester or 2-methoxyphenyl;
R³ is H, halogen or (1C-4C)alkyl;
R⁴ is H, OH, (1C-4C)alkoxy, or halogen;
R⁵ is H, OH, (1C-4C)alkoxy, NH₂, CN, halogen, (1C-4C)fluoroalkyl, NO₂, hydroxy(1C-4C)alkyl, CO₂H, CO₂(1C-6C)alkyl, or R⁵ is NHR⁶, wherein R⁶ is (1C-6C)acyl optionally substituted with one or more halogens, S(O)₂(1C-4C)alkyl, or S(O)₂aryl optionally substituted with (1C-4C)alkyl or one or more halogens, or R⁵ is C(O)N(R⁸,R⁹), wherein R⁸ and R⁹ each independently are H, (3C-6C)cycloalkyl, or CH₂R¹⁰, wherein R¹⁰ is H, (1C-5C)alkyl, (1C-5C)alkenyl, hydroxy(1C-3C)alkyl, (1C-4C)alkylester of carboxy(1C-4C)alkyl, (1C-3C)alkoxy(1C-3C)alkyl, (mono- or di(1C-4C)alkyl)aminomethyl, (mono- or di(1C-4C)alkyl)aminocarbonyl, or a 3-, 4-, 5- or 6-membered monocyclic, homo- or heterocyclic, aromatic or non-aromatic ring, or
R⁸ and R⁹ form together with the N a heterocyclic 5- or 6-membered saturated or unsaturated ring optionally substituted with (1C-4C)alkyl;
or a salt or hydrate form thereof.

In one embodiment R¹ is a 5- or 6-membered monocyclic, hetero- or homocyclic, saturated or unsaturated ring structure optionally substituted with one or more substituents selected from the group consisting of halogen, CN, CF₃, nitro, methoxy, trifluoromethoxy or methyl; R² is 2-nitrophenyl, 2-cyanophenyl, 2-hydroxymethyl-phenyl, pyridin-2-yl, pyridin-2-yl-N-oxide, 2-benzamide, 2-benzoic acid methyl ester or 2-methoxyphenyl; R³ is H, halogen or (1C-2C)alkyl; R⁴ is H or F.

In another embodiment R⁵ is H, OH, (1C-4C)alkoxy, CN, halogen, (1C-4C)fluoroalkyl, NO₂, hydroxy(1C-4C)alkyl, CO₂(1C-6C)alkyl, or R⁵ is NHR⁶, wherein R⁶ is (1C-6C)acyl optionally substituted with one or more halogens, S(O)₂(1C-4C)alkyl, or S(O)₂aryl optionally substituted with (1C-4C)alkyl or one or more halogens, or R⁵ is C(O)N(R⁸,R⁹), wherein R⁸ and R⁹ each independently are H, (3C-6C)cycloalkyl, or CH₂R¹⁰, wherein R¹⁰ is H, (1C-5C)alkyl, (1C-5C)alkenyl, hydroxy(1C-3C)alkyl, (1C-4C)alkylester of carboxy(1C-4C)alkyl, (1C-3C)alkoxy(1C-3C)alkyl, (mono- or di(1C-4C)alkyl)aminomethyl, (mono- or di(1C-4C)alkyl)-aminocarbonyl, or a 3-, 4-, 5- or 6-membered monocyclic, homo- or heterocyclic, aromatic or non-aromatic ring, or R⁸ and R⁹ form together with the N a heterocyclic 5- or 6-membered saturated or unsaturated ring optionally substituted with (1C-4C)alkyl.

In yet another embodiment R³ is H or halogen; R⁴ is H; R⁵ is H, OH, (1C-4C)alkoxy, CN, F, Cl, CF₃, NO₂, hydroxy(1C-4C)alkyl, CO₂(1C-6C)alkyl, or R⁵ is NHR⁶, wherein R⁶ is (1C-3C)acyl optionally substituted with one or more halogens or R⁵ is C(O)N(R⁸,R⁹), wherein R⁸ and R⁹ each independently are H, (3C-5C)cycloalkyl, or CH₂R¹⁰, wherein R¹⁰ is H, (1C-5C)alkyl, (1C-5C)alkenyl, hydroxy(1C-3C)alkyl, (1C-2C)alkylester of carboxy(1C-2C)alkyl, (1C-3C)alkoxy(1C-3C)alkyl, (mono- or di(1C-4C)alkyl)aminomethyl, (mono- or di(1C-4C)alkyl)aminocarbonyl, (3C-5C)cycloalkyl, or a 5-membered heterocyclic ring.

In yet another embodiment X is S or SO₂; R² is 2-nitrophenyl, 2-hydroxymethyl-phenyl, 2-benzamide, 2-methoxyphenyl, 2-cyanophenyl or pyridin-2-yl; R³ is H or F; R⁵ is H, OH, (1C-2C)alkoxy, CN, F, Cl, CF₃, NO₂, hydroxy(1C-4C)alkyl, CO₂(1C-4C)alkyl, or R⁵ is NHR⁶, wherein R⁶ is formyl, acetyl, fluoroacetyl, difluoroacetyl, or trifluoroacetyl, or R⁵ is C(O)N(R⁸,R⁹), wherein R⁸ is H, and R⁹ is H, cyclopropyl or R⁹ is CH₂R¹⁰, wherein R¹⁰ is H, (1C-2C)alkyl, hydroxy(1C-2C)alkyl, methoxy(1C-2C)alkyl, cyclopropyl.

In yet another embodiment X is S; R¹ is 3,5-difluorophenyl, pyridin-2-yl, pyridin-3-yl, pyrimidin-5-yl, pyrimidin-4-yl, pyrazin-2-yl, 3-fluorophenyl, 3-cyanophenyl, or 3-nitrophenyl; R² is 2-nitrophenyl, 2-hydroxymethyl-phenyl, 2-methoxyphenyl, 2-cyanophenyl or pyridin-2-yl; R³ is H; R⁵ is OH, (1C-2C)alkoxy, CN, CF₃, NO₂, hydroxy(1C-4C)alkyl, CO₂(1C-4C)alkyl, or NHR⁶, wherein R⁶ is formyl, acetyl, fluoroacetyl, difluoroacetyl, or trifluoroacetyl.

In yet another embodiment R¹ is 3,5-difluorophenyl, pyridin-2-yl, pyridin-3-yl, pyrimidin-5-yl, pyrimidin-4-yl, or pyrazin-2-yl; R² is 2-nitrophenyl, or 2-hydroxymethyl-phenyl; R⁵ is OH, (1C-2C)alkoxy, CN, hydroxy(1C-4C)alkyl, or NHR⁶, wherein R⁶ is formyl, acetyl, fluoroacetyl, difluoroacetyl, or trifluoroacetyl.

In yet another embodiment R¹ is 3,5-difluorophenyl, pyridin-2-yl, pyridin-3-yl, pyrimidin-5-yl, or pyrimidin-4-yl; R² is 2-nitrophenyl; R⁵ is OH, (1C-2C)alkoxy, CN, or NHR⁶, wherein R⁶ is formyl, acetyl, fluoroacetyl, difluoroacetyl, or trifluoroacetyl.

In a specific embodiment the subject invention provides the compounds 6-Methoxy-3-(2-nitro-phenylsulfanyl)-1-pyrimidin-5-ylmethyl-1*H*-indole, 3-(2-Nitrophenylsulfanyl)-1-pyridin-2-ylmethyl-1*H*-indole-6-carbonitrile, 3-(2-Nitrophenylsulfanyl)-1-pyridin-2-ylmethyl-1*H*-indole-6-carbonitrile-hydrochloride, 3-(2-Nitro-phenylsulfanyl)-1-pyrimidin-5-ylmethyl-1*H*-indole-6-carbonitrile, 3-(2-Nitrophenylsulfanyl)-1-pyrimidin-4-ylmethyl-1*H*-indole-6-carbonitrile, *N*-[1-(3,5-Difluorobenzyl)-3-(2-nitro-phenylsulfanyl)-1*H*-indol-6-yl]-2-fluoro-acetamide, and *N*-[3-(2-Nitro-phenylsulfanyl)-1-pyrimidin-5-ylmethyl-1*H*-indol-6-yl]-formamide.

In another embodiment X is S; R¹ is 3,5-difluorophenyl, pyridin-2-yl, pyridin-3-yl, 3-fluorophenyl, 3-cyanophenyl, or 3-nitrophenyl; R² is 2-nitrophenyl, 2-hydroxymethyl-phenyl, 2-methoxyphenyl, 2-cyanophenyl or pyridin-2-yl; R³ is H; R⁵ is C(O)N(R⁸,R⁹), wherein R⁸ is H, and R⁹ is H, or CH₂R¹⁰, wherein R¹⁰ is H, (1C-2C)alkyl, hydroxy(1C-2C)alkyl, or methoxy(1C-2C)alkyl.

In yet another embodiment R¹ is 3,5-difluorophenyl, pyridin-2-yl, or pyridin-3-yl; R² is 2-nitrophenyl, or 2-hydroxymethyl-phenyl; R⁵ is C(O)N(R⁸,R⁹), wherein R⁸ is H, and R⁹ is CH₂R¹⁰, wherein R¹⁰ is H, or (1C-2C)alkyl.

In a specific embodiment the subject invention provides the compound 1-(3,5-Difluorobenzyl)-3-(2-nitro-phenylsulfanyl)-1*H*-indole-6-carboxylic acid methylamide.

In yet another embodiment X is S; R¹ is 3,5-difluorophenyl, pyridin-2-yl, pyridin-3-yl, 3-fluorophenyl, 3-cyanophenyl, or 3-nitrophenyl; R² is 2-nitrophenyl, 2-hydroxymethyl-phenyl, 2-methoxyphenyl, 2-cyanophenyl or pyridin-2-yl; R³ is H; R⁵ is C(O)N(R⁸,R⁹), wherein R⁸ and R⁹ each independently are H or CH₂R¹⁰, wherein R¹⁰ is H, (1C-5C)alkyl, (1C-5C)alkenyl, hydroxy(1C-3C)alkyl, (1C-3C)alkoxy(1C-3C)alkyl, or (mono- or di(1C-4C)alkyl)aminomethyl.

In yet another embodiment R¹ is 3,5-difluorophenyl, pyridin-2-yl, or pyridin-3-yl; R² is 2-nitrophenyl, or 2-hydroxymethyl-phenyl; R⁵ is C(O)N(R⁸,R⁹), wherein R⁸ and R⁹ each independently are H, or CH₂R¹⁰, wherein R¹⁰ is H, (1C-5C)alkyl, hydroxy(1C-3C)alkyl, or (1C-3C)alkoxy(1C-3C)alkyl.

In a specific embodiment the subject invention provides the compound 1-(3,5-Difluorobenzyl)-3-(2-nitro-phenylsulfanyl)-1*H*-indole-6-carboxylic acid dimethylamide.

In those cases that a compound of the invention contains a basic amine function, the compound may be used as a free base or as a pharmaceutically acceptable salt such as hydrochloride, acetate, oxalate, tartrate, citrate, phosphate, maleate or fumarate.

A compound according to the invention is a compound as defined above, a salt thereof, a hydrate thereof or a prodrug thereof.

The terms used in this description have the following meaning:
alkyl is a branched or unbranched alkyl group, for example methyl, ethyl, propyl, isopropyl, butyl, sec-butyl, tert-butyl, hexyl, and the like;
fluoroalkyl is an alkyl group substituted with one or more fluorine atoms;
cycloalkyl is a cyclized unbranched alkyl group, such as cyclopropyl, cyclobutyl, cyclopentyl and the like;
alkenyl is a branched or unbranched alkenyl group, such as ethenyl, 2-butenyl, etc.;
alkoxy is a branched or unbranched alkyloxy group, for example methyloxy, ethyloxy, propyloxy, isopropyloxy, butyloxy, sec-butyloxy, tert-butyloxy and the like; fluoroalkoxy is a alkoxy group substituted with one or more fluorine atoms;
aryl is a mono- or polycyclic, homo- or heterocyclic aromatic ring system, such as phenyl, naphtyl or pyridyl; a monocyclic ring with 6 atoms is preferred for use;
acyl is a (substituent-)carbonyl group, such as an aroyl or alkanoyl;
aroyl is arylcarbonyl such as a benzoyl group;
alkanoyl means a formyl or alkylcarbonyl group such as formyl, acetyl and propanoyl; carboxy is a -COOH substituent, making the compound an organic acid;
carboxylate is a salt of a carboxyl substituent.

The prefixes (1C-4C), (2C-4C) etc. have the usual meaning to restrict the meaning of the indicated group to those with 1 to 4, 2 to 4 etc. carbon atoms; halogen refers to fluorine, chlorine, bromine and iodine.

The androgen receptor affinity and efficacy of the compounds according to the invention make them suitable for use in the treatment of androgen-receptor related disorders, disorders which can be treated with androgens, and in diagnostic methods focussed on the amount and/or location of androgen receptors in various tissues. For the latter purpose it can be preferred to make labelled variants of the compounds according to the invention. Typical androgen receptor-related treatments are those for male contraception and male or female hormone replacement therapy.

The subject invention also lies in the use of any of its compounds for the preparation of a medicine for treating androgen insufficiency. In the context of the invention, the term "androgen insufficiency" is to be understood to pertain to all kinds of diseases, disorders, and symptoms in which a male or a female suffers from too low a testosterone level, such as in hypogonadal men or boys. In particular, the androgen insufficiency to be treated by a compound of the invention is the reduction of the testosterone level which a male individual incurs as a result of age (the compound of the invention is then used for male hormone replacement therapy), or when he is subject to male contraception. In the context of male contraception, the compound of the invention especially serves to neutralise the effect of regimens of male hormone contraception in which a sterilitant such as a progestagen or LHRH (luteinizing hormone releasing hormone) is administered regularly, *e.g.* daily, or it is used as the sole male contraceptive substance.

Thus, the subject invention provides any one of the compounds of the subject invention for use in therapy.

The subject invention further encompasses a pharmaceutical composition comprising a compound of the subject invention and a pharmaceutically acceptable carrier. In an embodiment of the subject invention, the pharmaceutical composition is for the treatment of a disorder selected from the group consisting of an androgen-receptor related disorder, an androgen related disorder and androgen insufficiency.

The subject invention further provides a use of a compound of the invention for the manufacture of a medicament for the treatment of androgen-receptor related disorders, androgen related disorders and androgen insufficiency.

The compounds of the invention may further be useful for the treatment of osteoporosis, as well as other bone disorders, bone fraction repair, sarcopenia, frailty, skin aging, male hypogonadism, female sexual dysfunction, postmenopausal symptoms, atherosclerosis, aplastic anemia, muscular atrophy, lipodystrophy, reduced muscle strength and function, side effects of chemotherapy, chronic fatigue syndrome, benign prostate hyperplasia (BPH), cachexia, chronic catabolic state, cognitive impairment, male contraception, and others.

The compounds of the invention may be administered in conjunction with estrogens, progestogen and other androgens.

The compounds of the invention can be produced by various methods known in the art of organic chemistry in general. More specifically the routes of synthesis as illustrated in the following schemes and examples can be used. In the schemes and examples the following abbreviations were used:
DMF = dimethylformamide
mCPBA = *meta* chloro perbenzoic acid
THF = tetrahydofuran
TBTU = 2-(1H-benzotriazol-1-yl)-1,1,3,3-tetramethyluronium tetrafluoroborate
DIPEA = diisopropylethylamine
EDCI = 1-(3-dimethylaminopropyl)-3-ethylcarbodiimide hydrochloride
HOBt = 1-hydroxybenzotriazole
NMM = N-methylmorpholine
SPE = solid phase extraction
RP-SPE = reversed phase solid phase extraction
DMSO = dimethylsulfoxide
DCM = dichloromethane
DHT = 5α-dihydrotestosterone
NMP = 1-methyl-2-pyrrolidinone
DMS = dimethyl sulfide

With regard to nomenclature the following trivial names are used interchangeably for substituents R¹ and R²:

| Trivial name | Official name |
|---|---|
| 2-pyridyl | pyridin-2-yl |
| 3-pyridyl | pyridyl-3-yl |
| 4-pyridyl | pyridyl-4-yl |
| 3,5-pyrimidyl | pyrimidin-5-yl |
| 2,4-pyrimidyl | pyrimidin-4-yl |
| 2,5-pyrazyl | pyrazin-2-yl |

etc.

In each of the Schemes **I-VII** the meanings of the symbols correspond to the definitions given in the previous paragraphs.

Substituted indole compounds of structure **3** were prepared in two steps from the correctly substituted indoles of structure **1**, via two different routes. In the first route a correctly substituted indole of structure **1** is N-alkylated with a halide of type R¹CH₂Y, where Y is a halogen, mesylate or tosylate, with NaH or Cs₂CO₃ as a base in DMF or NMP at 0°C to room temperature, to give a compound of structure **2.** Structure **2** is then sulfanylated at the C-3 position of the indole ring by reaction with a sulfenyl chloride in either CH₂Cl₂ or diethyl ether at room temperature, to give structure **3.** In the second route the two steps of the first route are reversed: the correctly substituted indole of structure **1** is first sulfanylated at the C-3 position of the indole to give structure **4,** followed by N-alkylation with R¹CH₂Y to give structure **3** (Scheme **I**).

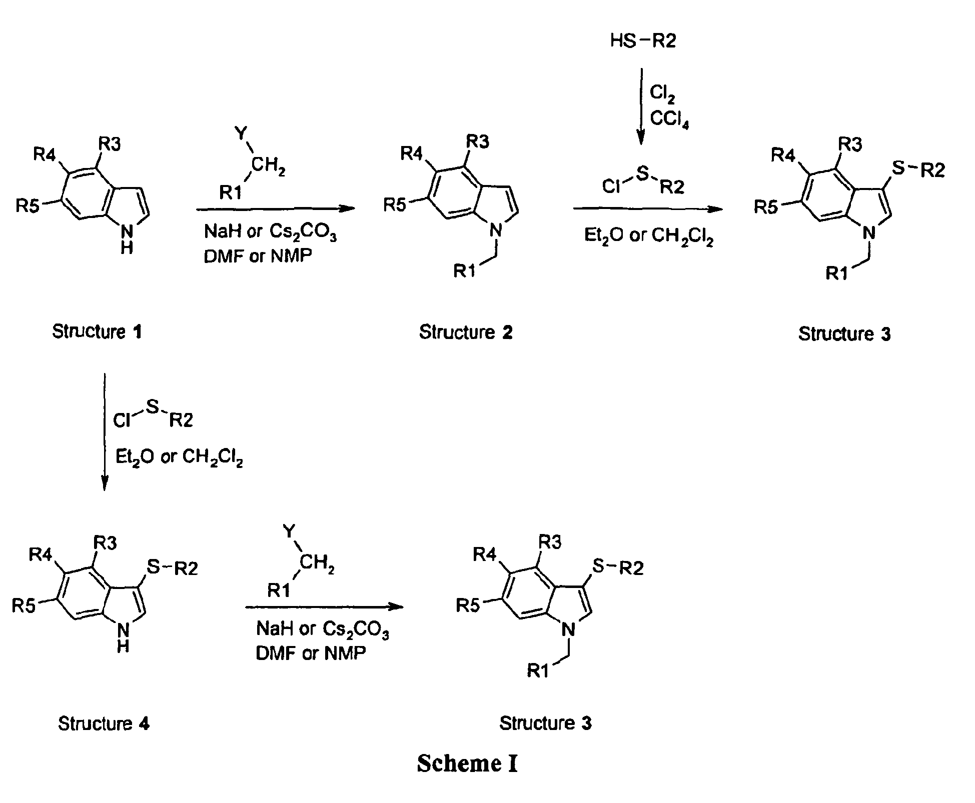

Sulfoxides of structure **5** can be obtained by oxidation of the corresponding sulfide (Structure **3**) by reaction with e.g. 0.9 equivalents of mCPBA. Sulfones of structure **6** can be obtained by oxidation of the corresponding sulfide (Structure **3**) by reaction with e.g. 3 equivalents of mCPBA (Scheme **II**).

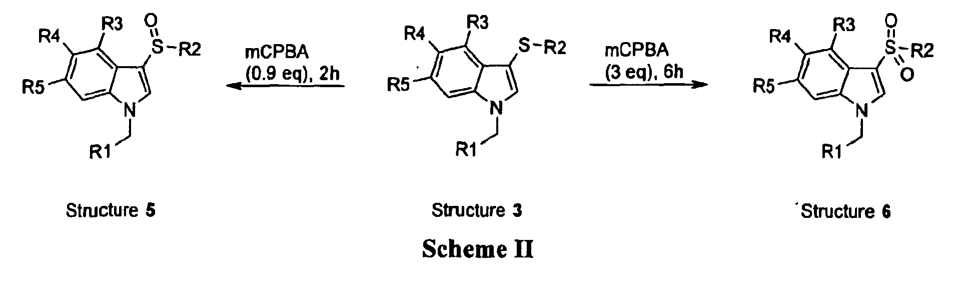

Scheme **III** describes the synthesis of compounds of structure **3,** in which the R² group is a phenyl ring substituted on the 2-position with either CO₂Me (Structure **7**), CH₂OH (Structure **8**), CO₂H (Structure **9**), CONHMe (Structure **10**), CONH₂ (Structure **11**) or CN (Structure **12**).

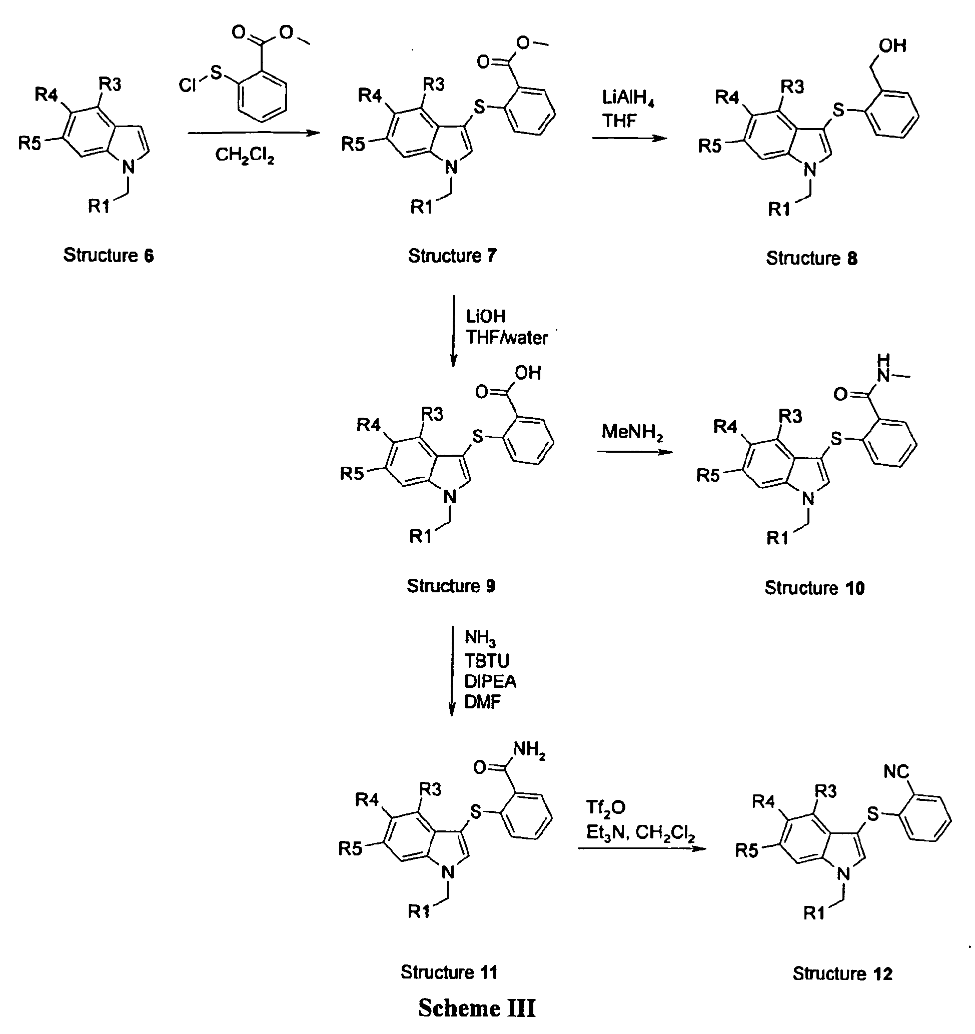

In the first step a substituted indole of structure **6** is sulfanylated at the 3-position with 2-(carboxymethyl)-phenylsulfenyl chloride, which was prepared from methylthiosalicylate and chlorine gas, to give a compound of structure **7**. Reduction of the methyl ester moiety of compounds of structure **7** with LiAlH₄ gave the corresponding hydroxymethyl compounds of structure **8**. Saponification of the methyl ester moiety of compounds of structure **7** with lithium hydroxide gave the corresponding carboxylic acid compounds of structure **9.** The carboxylic acid moiety of compounds of structure **9** can be converted to the corresponding carboxamide by reaction with an amine, TBTU and DIPEA in DMF at room temperature. By this method structure **10** was obtained when methylamine was used as the amine and structure **11** was obtained when NH₃ was used as the amine. Dehydration of the benzamide moiety of structure **11** with Tf₂O and triethylamine in CH₂Cl₂ afforded the corresponding benzonitrile compound of structure **12** (Scheme **III**).

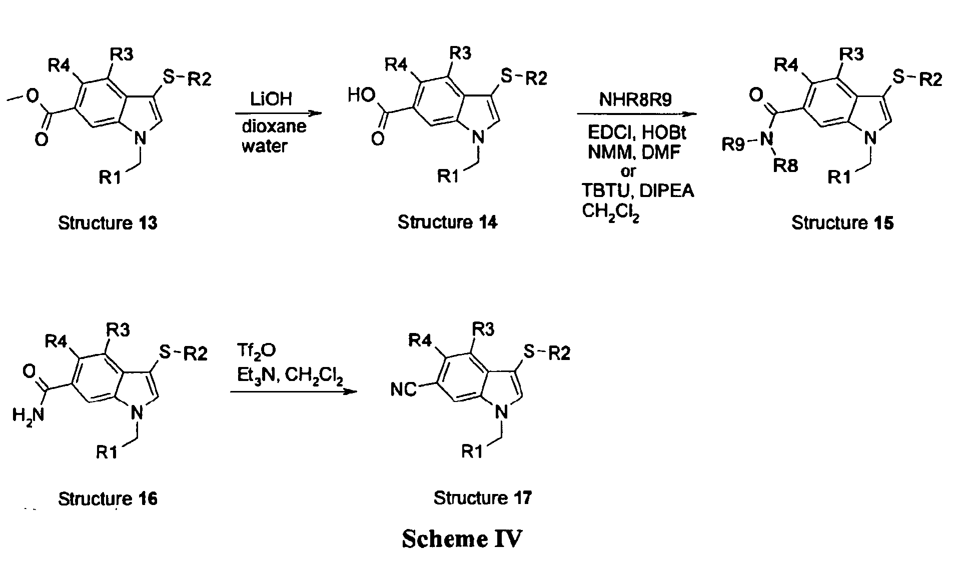

Scheme **IV** describes the synthesis of compounds of structure **3,** in which the indole ring is substituted at the 6-position with either CO₂Me (Structure **13**), CO₂H (Structure **14**), CONR⁸R⁹ (Structure **15**), CONH₂ (Structure **16**) or CN (Structure **17**). Saponification of the methyl ester moiety of structure **13** with lithium hydroxide afforded the corresponding carboxylic acid of structure **14**. The carboxylic acid moiety of structure **14** can be converted to the corresponding carboxamides of structures **15** and **16** by reaction with an amine or amine salt, in the presence of EDCI and HOBt in a mixture of NMM and DMF or in the presence of TBTU and DIPEA in CH₂Cl₂ at room temperature. Dehydration of the benzamide moiety of structure **16** with Tf₂O and triethylamine in CH₂Cl₂ afforded the corresponding benzonitrile compound of structure **17** (Scheme **IV**).

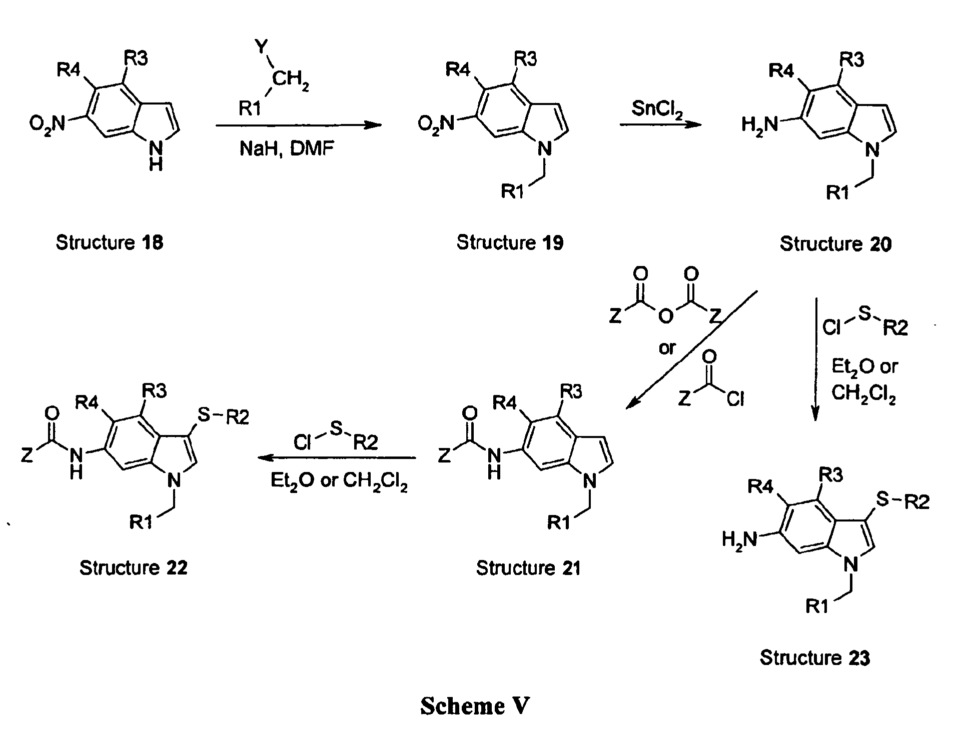

Scheme **V** describes the synthesis of compounds of structure **22** containing an acylated amine functionality on the 6-position of the indole ring. These compounds can be synthesised from 6-nitro indoles of structure **18** in 4 steps. In the first step the indole of structure **18** is alkylated on the nitrogen atom by reaction of a halide of type R¹CH₂Y, in which Y is halogen, mesylate or tosylate, with NaH as a base in DMF to give compounds of structure **19.** In the second step the nitro group of structure **19** is reduced to an amine group by SnCl₂ to give structure **20.** Subsequent acylation of the amine group with an acid chloride of type ZCOCl afforded structure **21,** which was then sulfanylated at the 3-position of the indole ring by reaction with a sulfenyl chloride in either CH₂Cl₂ or Et₂O as a solvent, at room temperature, to give compounds of structure **22.** Direct sulfanylation of a compound of structure **20** with a sulfenyl chloride afforded structure **23**

In Scheme **VI** is decribed how methyl ethers of structure **24** can be cleaved with e.g. BF₃·DMS in THF at room temperature to hydroxy compounds of structure **25.**

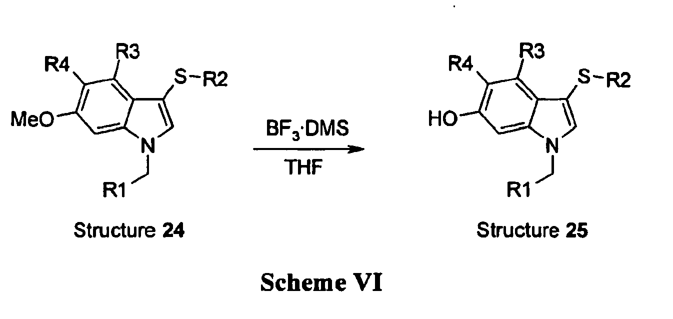

Scheme **VII** describes the synthesis of compounds containing either an acylated amine functionality (structure **28**) or a sulfonamide functionality (structure **29**) at the 6-position of the indole ring. These compounds can be prepared from 6-nitro indoles of structure **18** in 6 steps. In the first step the indole of structure **18** is alkylated on N-1 by reaction of a halide of type R¹CH₂Y, in which Y is a halogen, mesylate or tosylate, with NaH or Cs₂CO₃ as a base in either NMP or DMF to give compounds of structure **19.** In the second step the nitro group of structure **19** is reduced to an amine group with Fe and NH₄Cl in an ethanol/water mixture to give structure **20.** Subsequent acylation of the amine group with di-*tert*-butyl dicarbonate afforded structure **26** which was then sulfanylated at the 3-position of the indole ring by reaction with a sulfenyl chloride in either CH₂Cl₂ or Et₂O to give compounds of structure **27.** Then the amide functionality at the 6-position of the indole was cleaved with trifluoroacetic acid or HCOOH to give amines of structure **23.** These amines can be converted into amides of structure **28** by reaction with either triethylamine and an acid chloride in CH₂Cl₂ (method a) or an acid, TBTU and DIPEA in CH₂Cl₂ (method b). Sulfonamides of structure **29** can be prepared by reaction of amines of structure **23** with sulfonyl chlorides (method c).

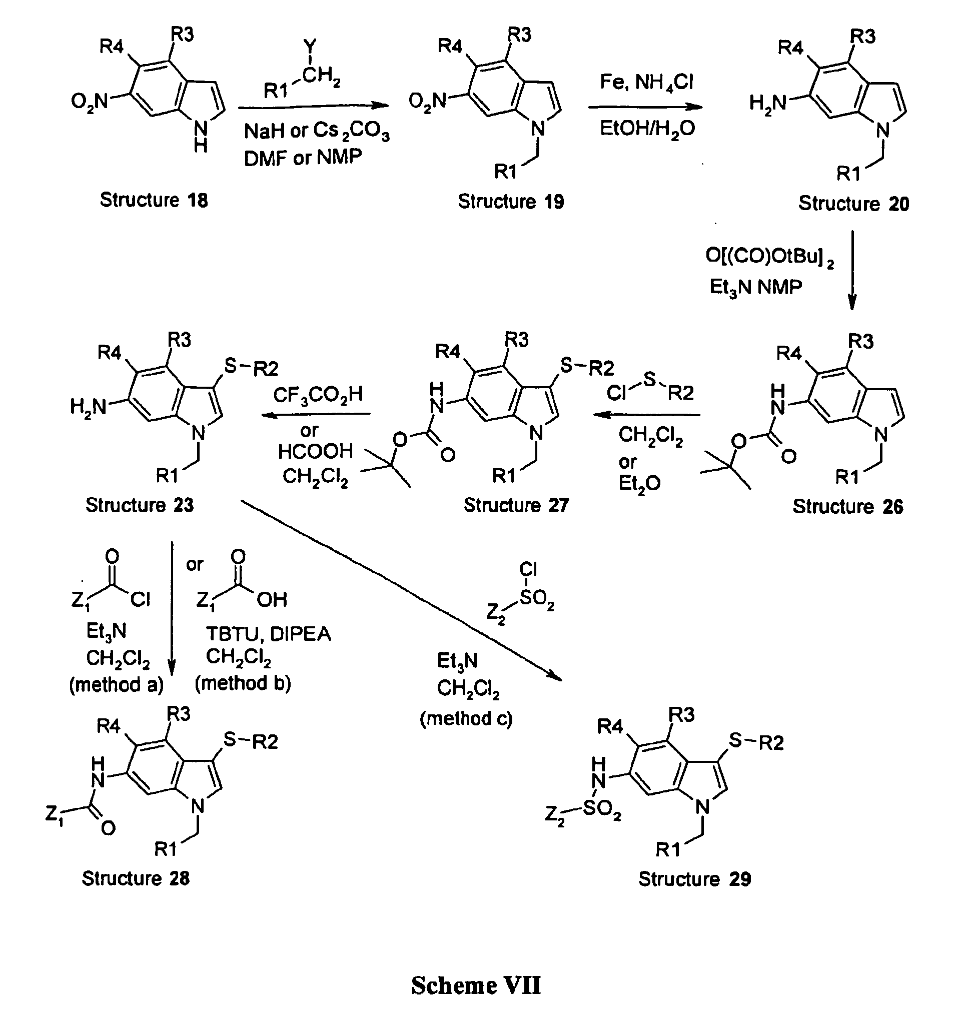

The present invention also relates to a pharmaceutical composition comprising the non-steroidal compound according to the invention mixed with a pharmaceutically acceptable auxiliary, such as described in the standard reference Gennaro et al, Remmington: The Science and Practice of Pharmacy, (20th ed., Lippincott Williams & Wilkins, 2000, see especially Part 5: Pharmaceutical Manufacturing). Suitable auxiliaries are made available in e.g. the Handbook of Pharmaceutical Excipients (2nd Edition, Editors A. Wade and P.J. Weller; American Pharmaceutical Association; Washington; The Pharmaceutical Press; London, 1994). The mixture of a compound according to the invention and a pharmaceutically acceptable auxiliary may be compressed into solid dosage units, such as pills, tablets, or be processed into capsules or suppositories. By means of pharmaceutically suitable liquids the compounds can also be applied as an injection preparation in the form of a solution, suspension, emulsion, or as a spray, e.g. nasal spray. For making dosage units, e.g. tablets, the use of conventional additives such as fillers, colorants, polymeric binders and the like is contemplated. In general any pharmaceutically acceptable additive which does not interfere with the function of the active compounds can be used. The compounds of the invention may also be included in an implant, a vaginal ring, a patch, a gel, and any other preparation for sustained release.

Suitable carriers with which the compositions can be administered include lactose, starch, cellulose derivatives and the like, or mixtures thereof used in suitable amounts.

The dosage amounts of the present compounds will be of the normal order for pharmaceutically active compounds, e.g. of the order of 0.001 to 50 mg/kg body weight of the recipient per administration. The recipient can be a human or an animal in need of an androgen receptor-related treatment or an androgen treatment.

The invention is illustrated by the following examples.

### Examples

### Example 1

### 1-(3,5-Difluoro-benzyl)-6-methoxy-3-(2-nitro-phenylsulfanyl)-1H-indole (Compound 63, Structure 3 of Scheme I, where R¹ = 3,5-difluorophenyl, R² = 2-nitrophenyl, R³=R⁴=H, R⁵=OMe)

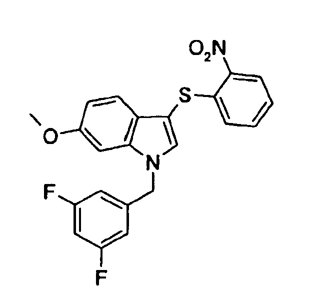

**General method 1:** N-alkylation of a (un)substituted indole of structure **1** to give N-alkylated indole of structure 2, followed by 3-sulfanylation to give substituted indoles of structure **3** (Scheme **I**).

### (a) 1-(3,5-Difluoro-benzyl)-6-methoxy-1H-indole (Structure 2 of Scheme I, where R¹ = 3,5-difluorophenyl, R³=R⁴=H, R⁵=OMe)

Under a nitrogen atmosphere: to a cooled (0°C) solution of 6-methoxyindole (863 mg, 5.86 mmol) in DMF (40 mL) was added NaH (60% in oil; 281 mg, 7.03 mmol) in small portions over a 3 minute period. The resulting green suspension was stirred at 0°C for 10 min. Then 3,5-difluorobenzyl bromide (0.91 mL, 7.03 mmol) was added. The mixture was stirred at 0°C for 1 h and then at room temperature for another 21 h. Ethyl acetate (50 mL) was added and the mixture was washed with 3% aqueous citric acid (3x50 mL) and brine (50 mL). The organic phase was dried (MgSO₄) and concentrated *in vacuo* to give a green oil (1.43 g). The crude product was purified over a 20 g silica SPE cartridge (ethyl acetate/heptane 1:9) to give the title compound as a colourless oil (1.23 g, yield = 77%).
LCMS: 4.01 min (96.3%, MH⁺ = 274); TLC (ethyl acetate/heptane 1:4): R_{f} = 0.46; ¹H NMR (CDCl₃): δ 3.80 (s, 3H, OCH₃), 5.24 (s, 2H, NCH₂Ar), 6.51 (dd, 1H, J1 = 3.5 Hz, J2 = 0.8 Hz), 6.57-6.60 (m, 2H), 6.65 (d, 1H, J = 3.1 Hz), 6.66-6.72 (m, 1H), 6.81 (dd, 1H, J1 = 8.6 Hz, J2 = 3.1 Hz), 7.01 (d, 1H, J = 3.5 Hz), 7.53 (d, 1H, J = 8.6 Hz).

### (b) 1-(35-Difluoro-benzyl)-6-methoxy-3-(2-nitro-phenylsulfanyl)-1H-indole (Compounds 63, Structure 3 of Scheme I, where R¹ = 3,5-difluorophenyl R² = 2-nitrophenyl, R³=R⁴=H, R⁵=OMe)

To a solution of 1-(3,5-Difluoro-benzyl)-6-methoxy-1*H*-indole (900 mg, 3.29 mmol) in diethyl ether (20 mL) was added dropwise at room temperature a suspension of 2-nitrobenzenesulfenyl chloride (627 mg, 3.31 mmol) in diethyl ether (10 mL) over a period of 2 min. After stirring at room temperature for 1h ethyl acetate (50 mL) was added and the mixture was washed with saturated NaHCO₃ solution (2x50 mL) and brine (50 mL). The organic phase was dried (MgSO₄) and concentrated *in vacuo* to give an orange-red oil (1.54 g). The crude product was crystallised from toluene/acetone to give the title compound as orange-red crystalline solid (900 mg, yield = 64%).
LCMS: 4.25 min (100%, MH⁺ = 427); HPLC: 4.86 min (98.7%); ¹H NMR (CDCl₃): δ 3.82 (s, 3H, OCH₃), 5.32 (s, 2H, NCH₂Ar), 6.63-6.69 (m, 2H), 6.72-6.79 (m, 1H), 6.75 (d, 1H, J = 2.7 Hz), 6.85 (dd, 1H, J1 = 8.2 Hz, J2 = 2.7 Hz), 6.98 (dd, 1H, J1 = 6.7 Hz, J2 = 1.2 Hz), 7.16-7.20 (m, 1H), 7.26-7.30 (m, 1H) 7.34 (s, 1H), 7.39 (d, 1H, J = 8.2 Hz), 8.27 (dd, 1H, J1 = 8.2 Hz, J2 = 1.6 Hz).

According to General method 1 the following compounds were prepared:

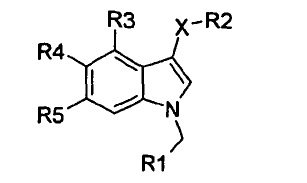

**Table 1 Compounds synthesised according to General Method 1**

| **Compound number** | **X** | **R¹** | **R²** | **R³** | **R⁴** | **R⁵** | **MWt** | **LCMS (MH+)** | **LCMS ret. Time^{a} (min)** |
|---|---|---|---|---|---|---|---|---|---|
| **21** | S | 3,5-difluorophenyl | 2-methoxyphenyl | H | H | OMe | 411.47 | 446^{c} | 5.14^{b} |
| **22** | S | 3,5-difluorophenyl | 2-nitrophenyl | H | H | Br | 475.32 | 476 | 4.89 |
| **23** | S | 3,5-difluorophenyl | 2-nitrophenyl | H | H | CF3 | 464.41 | 465 | 5.34^{b} |
| **24** | S | 3,5-difluorophenyl | 2-nitrophenyl | H | H | CH2OH | 426.44 | Nd | 3.88^{b} |
| **25** | S | 3,5-difluorophenyl | 2-nitrophenyl | H | H | Cl | 430.86 | 431 | 4.89 |
| **50** | S | 3,5-difluorophenyl | 2-nitrophenyl | H | H | F | 414.40 | 415 | 4.79 |
| **51** | S | 3,5-difluorophenyl | 2-nitrophenyl | F | H | H | 414.40 | 415 | 4.72 |
| **52** | S | 3,5-difluorophenyl | 2-nitrophenyl | H | H | H | 396.41 | 397 | 4.22 |
| **53** | S | 3,5-difluorophenyl | 2-nitrophenyl | H | F | H | 414.40 | 415 | 4.75 |
| **54** | S | 3,5-difluorophenyl | 2-nitrophenyl | Cl | H | H | 430.86 | 431 | 5.14^{b} |
| **55** | S | 3,5-difluorophenyl | 2-nitrophenyl | Me | H | H | 410.44 | 411 | 5.24^{b} |
| **56** | S | 3,5-difluorophenyl | 2-nitrophenyl | H | OH | H | 412.41 | 413 | 4.50^{b} |
| **60** | S | 3,5-difluorophenyl | 2-nitrophenyl | H | H | N02 | 441.41 | 442 | 4.7 |
| **61** | S | 3,5-difluorophenyl | 2-nitrophenyl | H | H | OH | 412.41 | 413 | 4.54^{b} |
| **63** | S | 3,5-difluorophenyl | 2-nitrophenyl | H | H | OMe | 426.45 | 274^{d} | 4.01 |
| **66** | S | 3,5-difluorophenyl | 2-pyridyl-N-oxide | H | H | OMe | 398.43 | 399 | 3.96 |
| **90** | S | phenyl | 2-nitrophenyl | H | H | H | 360.43 | 361 | 4.19 |
| **91** | S | phenyl | 2-nitrophenyl | H | OMe | H | 390.46 | 391 | 4.14 |
| **92** | S | phenyl | 2-nitrophenyl | H | H | OMe | 390.46 | 391 | 4.12 |

| | | | | | | | | | |
|---|---|---|---|---|---|---|---|---|---|
| a) 7 min LCMS method was used, unless stated otherwise. b) 10 min LCMS method was used. c) M+Cl. nd = not detected. d) =[M-S(PhNO₂)]H⁺. | | | | | | | | | |

### Example 2

### 1-(3,5-Difluoro-benzyl)-3-(2-nitro-phenylsulfanyl)-1H-indole-6-carboxylic acid methyl ester (Compound 31, Structure 3 of Scheme I, where R¹ = 3,5-difluorophenyl R²=2-nitrophenyl, R³=R⁴=H, R⁵=CO₂Me)

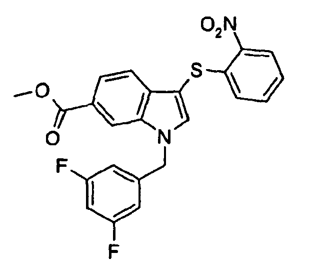

### (a) 1H-Indole-6-carboyylic acid methyl ester (Structure 1 of Scheme 1, where R³=R⁴=H, R⁵=CO₂Me)

To a solution of 1*H*-Indole-6-carboxylic acid (1.500 g, 9.31 mmol) in methanol (50 mL) was added concentrated H₂SO₄ (550 µL, 10.24 mmol). The mixture was stirred overnight at reflux temperature. The mixture was then neutralised to pH 7 by addition of saturated aqueous NaHCO₃ and the mixture was extracted twice with ethyl acetate. The combined organic layers were dried over Na₂SO₄ and concentrated *in vacuo* to give a yellow powder. The product was recrystallised from heptane/ethyl acetate to give the title compound as green/yellow crystals (688 mg, yield = 53%)
¹H NMR (CDCl₃): δ 3.94 (s, 3H, CH₃OCO), 6.60 (m, 1H), 7.37 (t, 1H, J = 4.7 Hz), 7.66 (d, 1H, J = 8.2 Hz), 7.82 (dd, 1H, J1 = 8.2 Hz, J2 = 1.0 Hz), 8.18 (s, 1H), 8.73 (s, 1H, NH)

### (b) 1-(3,5-Difluoro-benzyl)-1H-indole-6-carboxylic acid methyl ester (Structure 2 of Scheme I, where R¹ = 3,5-difluorophenyl, R³=R⁴=H, R⁵=CO₂Me)

Under a nitrogen atmosphere: to a solution of 1*H*-indole-6-carboxylic acid methyl ester (367 mg, 2.09 mmol) in DMF (10 mL) was added NaH (60% in oil, 10 mg, 2.52 mmol) at room temperature. After stirring for 15 min 1-bromomethyl-3,5-difluorobenzene (325 µL, 2.51 mmol) was added and the mixture was stirred at room temperature for 4 h. The reaction was quenched with 3% aqueous citric acid (10 mL) and ethyl acetate (30 mL) was added. The mixture was washed with 3% aqueous citric acid (3x20 mL) and brine (20 mL). The organic phase was dried (MgSO₄) and concentrated *in vacuo* to give a pale yellow oil (702 mg). The crude product was purified over a 20 g silica SPE cartridge (ethyl acetate/heptane 1:9) to give the title compound as a colourless oil, which slowly crystallised on standing (530 mg, yield = 84%).
LCMS: 4.08 min (99%, MH⁺ = 302); ¹H NMR (CDCl₃): δ 3.92 (3, 3H, CO₂CH₃), 5.37 (s, 2H, NCH₂Ar), 6.54-6.60 (m, 2H), 6.64 (d, 1H, J = 3.1 Hz), 6.67-6.74 (m, 1H), 7.27 (d, 1H, J = 3.1 Hz), 7.68 (d, 1H, J = 8.6 Hz), 7.83 (d, 1H, J = 8.6 Hz), 8.01 (s, 1H).

### (c) 1-(3,5-Difluoro-benzyl)-3-(2-nitro-phenylsulfanyl)-1H-indole-6-carboylic acid methyl ester (Compound 31, Structure 3 of Scheme I, where R¹ = 3,5-difluorophenyl, R² = 2-nitrophenyl, R³=R⁴=H, R⁵=CO₂Me)

To a solution of 1-(3,5-Difluoro-benzyl)-1*H*-indole-6-carboxylic acid methyl ester (330 mg, 1.10 mmol) in dichloromethane (30 mL) was added at room temperature a solution of 2-nitrobenzenesulfenyl chloride (210 mg, 1.11 mmol) in dichloromethane (10 mL). The mixture was stirred at room temperature for 4 days. The reaction mixture was concentrated and the crude product was purified over a 20 g silica SPE cartridge (ethyl acetate/heptane 1:5 to 1:2) to give the title compound as a yellow solid (392 mg, yield = 78%).
HPLC: 4.60 min (97.8%); ¹H NMR (CDCl₃): δ 3.93 (s, 3H, CO₂CH₃), 5.45 (s, 2H, NCH₂Ar), 6.63-6.69 (m, 2H), 6.74-6.80 (m, 1H), 6.88 (dd, 1H, J1 = 8.2 Hz, J2 = 1.2 Hz), 7.18-7.23 (m, 1H), 7.26-7.30 (m, 1H), 7.57 (d, 1H, J = 7.8 Hz), 7.58 (s, 1H), 7.89 (dd, 1H, J1 = 7.8 Hz, J2 = 1.2 Hz), 8.12 (s, 1H), 8.28 (dd, 1H, J1 = 7.8Hz, J2 = 1.2 Hz).

### Example 3

### 1-(3,5-Difluoro-benzyl)-3-(2-nitro-phenylsulfanyl)-1H-indole-6-carboxylic acid (Compound 30, Structure 14 of Scheme IV, where R¹ = 3,5-difluorophenyl, R² =2-nitrophenyl, R³=R⁴=H)

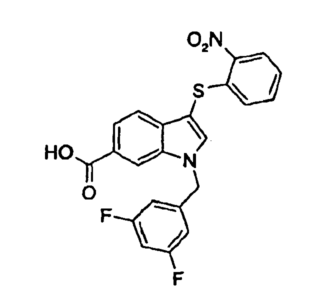

To a solution of 1-(3,5-Difluoro-benzyl)-3-(2-nitro-phenylsulfanyl)-1*H*-indole-6-carboxylic acid methyl ester (150.0 mg, 0.33 mmol) in dioxane (20 mL) and water (15 mL) was added LiOH-H₂O (83.1 mg, 2.0 mmol) in 5 ml water. The reaction mixture was stirred overnight at 60°C. The mixture was then acidified to pH 4 by addition of 15% aqueous HCl and the mixture was extracted twice with ethyl acetate. The combined organic layers were dried over Na₂SO₄ and concentrated *in vacuo* to give a yellow powder. The product was recrystallised from heptane/ethyl acetate to give the title compound as yellow/orange crystals (129.3 mg, y = 89%)
¹H NMR (DMSO): δ 5.62 (s, 2H, CH₂Ar), 6.85 (dd, 1H, J1 = 8.6 Hz, J2 = br), 7.05 (m, 2H, br), 7.20 (m, 1H, br), 7.32-7.38 (m, 1H), 7.40 (d, 1H, J = 8.2 Hz), 7.44-7.50 (m, 1H), 7.68 (dd, 1H, J1 = 8.6 Hz, J2 = br), 7.97 (s, 1H, COOH), 8.20 (s, 1H), 8.26 (s, 1H), 8.28 (d, 1H, J = 8.2)

### Example 4

### 1-(3,5-Difluoro-benzyl)-3-(2-nitro-phenylsulfanyl)-1H-indole-6-carboxylic acid methylamide (Compound 47, Structure 15 of Scheme IV, where R¹ = 3,5-difluorophenyl, R² = 2-nitrophenyl, R³=R⁴=R⁸=H, R⁹=Me)

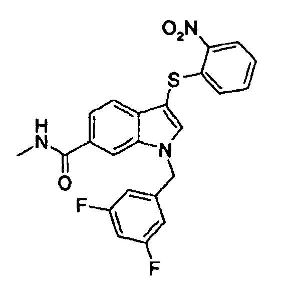

### General method 2: amidation of 6-carboxyl indoles of structure 14 to give 6-carboxamideindoles of structure 15 (Scheme IV).

Under nitrogen atmosphere: to a solution of 1-(3,5-Difluoro-benzyl)-3-(2-nitrophenylsulfanyl)-1*H-*indole-6-carboxylic acid (25.1 mg, 57.0 µmol) in dry DMF (10mL) was added NMM (1 mL), HOBt (9.60 mg, 62.7 µmol), EDCI (12.1 mg, 62.7 µmol), and methylammonium chloride (19.2 mg, 285 µmol). The reaction mixture was stirred overnight at room temperature and then poured into ice water. The resulting precipitate was filtered and the residue was washed with excess water followed by little heptane. The product was dried *in vacuo* at 40°C to give the title compound as a yellow solid (15.2 mg, y = 60%).
LCMS: 4.31 min (100.0%, MH⁺ = 454); ¹H NMR (DMSO): δ 2.80(d, 3H, J = 4.3, CH₃NHCO), 5.63 (s, 2H, CH₂Ar), 6.86 (dd, 1H, J1 = 8.2 Hz, J2 = 1.0 Hz), 7.04 (m, 2H), 7.20 (m, 1H), 7.33-7.37 (m, 1H), 7.41 (d, 1H, 8.6 Hz), 7.46-7.50 (m, 1H), 7.64 (dd, 1H, J1 = 8.2 Hz, J2 = 1.0 Hz), 8.15 (s, 1H), 8.25 (s, 1H), 8.27 (dd, 1H, J1 = 8.6 Hz, J2 = 1.0 Hz), 8.40 (m, 1H, MeNHCO).

According to General method 2 the following compounds were prepared:

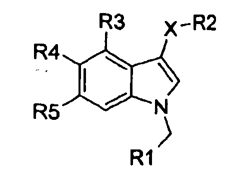

**Table 2 Compounds synthesised according to General Method 2**

| **Compound number** | **X** | **R¹** | **R²** | **R**³ | **R⁴** | **R⁵** | **MWt** | **LCMS (MH+)** | **LCMS ret. Time^{a} (min)** |
|---|---|---|---|---|---|---|---|---|---|
| **27** | S | 3,5-difluorophenyl | 2-nitrophenyl | H | H | CO(1-pyrrolidinyl) | 493.53 | 494 | 4.66 |
| **28** | S | 3,5-difluorophenyl | 2-nitrophenyl | H | H | CO(4-morpholinyl) | 509.53 | 510 | 4.50 |
| **29** | S | 3,5-difluorophenyl | 2-nitrophenyl | H | H | CO[1-(4-methylpiperazinyl)] | 522.57 | 523 | 3.81 |
| **32** | S | 3,5-difluorophenyl | 2-nitrophenyl | H | H | CONEt2 | 495.55 | 496 | 4.82 |
| **34** | S | 3,5-difluorophenyl | 2-nitrophenyl | H | H | CONHCH2(2-furanyl) | 519.53 | 520 | 4.74 |
| **35** | S | 3,5-difluorophenyl | 2-nitrophenyl | H | H | CONHCH2(3-pyridyl) | 530.55 | 531 | 3.94 |
| **36** | S | 3,5-difluorophenyl | 2-nitrophenyl | H | H | CONHCH2CH2NMe2 | 510.56 | 511 | 3.90 |
| **37** | S | 3,5-difluorophenyl | 2-nitrophenyl | H | H | CONHCH2CH2OH | 483.49 | 484 | 4.21 |
| **38** | S | 3,5-difluorophenyl | 2-nitrophenyl | H | H | CONHCH2CH2OMe | 497.52 | 498 | 4.48 |
| **39** | S | 3,5-difluorophenyl | 2-nitrophenyl | H | H | CONHCH2CO2Me | 511.50 | 512 | 4.51 |
| **40** | S | 3,5-difluorophenyl | 2-nitrophenyl | H | H | CONHCH2CONMe2 | 524.55 | 525 | 4.33 |
| **41** | S | 3,5-difluorophenyl | 2-nitrophenyl | H | H | CONHCH2cPr | 493.53 | 494 | 4.75 |
| **42** | S | 3,5-difluorophenyl | 2-nitrophenyl | H | H | CONHCH2iPr | 495.55 | 496 | 4.85 |
| **43** | S | 3,5-difluorophenyl | 2-nitrophenyl | H | H | CONHCH2Ph | 529.56 | 530 | 4.90 |
| **44** | S | 3,5-difluorophenyl | 2-nitrophenyl | H | H | CONHcPr | 479.50 | 480 | 4.57 |
| **45** | S | 3,5-difluorophenyl | 2-nitrophenyl | H | H | CONHEt | 467.49 | 468 | 4.56 |
| **46** | S | 3,5-difluorophenyl | 2-nitrophenyl | H | H | CONHiPr | 481.52 | 482 | 4.71 |
| **47** | S | 3,5-difluorophenyl | 2-nitrophenyl | H | H | CONHMe | 453.47 | 454 | 4.31 |
| **48** | S | 3,5-difluorophenyl | 2-nitrophenyl | H | H | CONHnPr | 481.52 | 482 | 4.75 |
| **49** | S | 3,5-difluorophenyl | 2-nitrophenyl | H | H | CONMe2 | 467.49 | 468 | 4.30^{b} |

| | | | | | | | | | |
|---|---|---|---|---|---|---|---|---|---|
| a) 7 min LCMS method was used, unless stated otherwise. b) 10 min LCMS method was used. | | | | | | | | | |

### Example 5

### 1-(3,5-Difluoro-benzyl)-3-(2-nitro-phenylsulfanyl)-1H-indole-6-carboxylic acid amide (Compound 33, Structure 16 of Scheme IV where R¹ = 3,5-difluorphenyl, R² = 2-nitrophenyl, R³=R⁴=H)

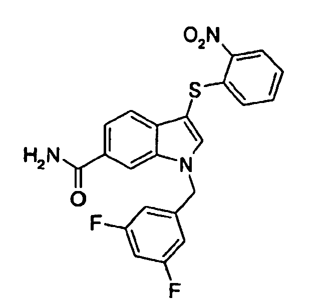

The title compound was prepared according to General method 2, using 51.1 mg (116 µmol) carboxylic acid, 19.5 mg (128 µmol) HOBt, 24.6 mg (128 µmol) EDCI and 31.1 mg (581 µmol) ammonium chloride. The title compound was obtained as a yellow solid (9,0 mg; 18%).
LCMS: 4.16 min (100.0%, MH⁺= 440); ¹H NMR (CDCl₃): δ 5.46 (s, 2H, CH₂Ar), 6.65 (m, 2H), 6.77 (m, 1H), 6.88 (dd, 1H, J1 = 8.2 Hz, J2 = 1.0 Hz)), 7.21 (m, 1H), 7,29 (m, 1H), 7.51 (dd, 1H, J1 = 7.8 Hz, J2 = 1.0 Hz), 7.57 (d, 1H, J = 7.7 Hz), 7.58 (s, 1H), 8.05 (s, 1H), 8.29 (dd, 1H, J1 = 8.2 Hz, J2 = 1.0 Hz).

### Example 6

### 6-Methoxy-3-(2-nitro-phenylsulfanyl)-1-pyridin-2-ylmethyl-1H-indole (Compound 9, Structure 3 of Scheme I, where R¹ = 2-pyridyl, R² = 2-nitrophenyl R³=R⁴=H, R⁵=OMe)

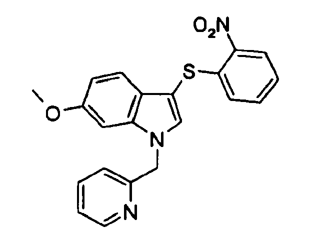

### General method 3: 3-sulfanylation of a (un)substituted indole of structure 1 to give substituted indoles of structure 4, followed by indole-N-alkylation to give N-alkylated indole structure 3 (Scheme I).

### (a) 6-Methoxy-3-(2-nitro-phenylsulfanyl)-1H-indole (Structure 4 of Scheme I, where R² =2-nitrophenyl, R¹=R³=R⁴=H, R⁵=OMe)

To a solution of 6-methoxyindole (1.5 g, 10.2 mmol) in Et₂O (100 mL) was added a solution of 2-nitrobenzenesulfenyl chloride (1.93 g, 10.2 mmol) in 50 mL Et₂O dropwise, over a 4 minute period. The resulting yellow solution was stirred at room temperature for 1 h. The solvent was evaporated and the crude product was purified over a silica column (heptane/ethyl acetate 9:1) to give the title compound (3.054 g, yield = 97%).
HPLC : 3.72 min. purity 96.7%, TLC (heptane/ethyl acetate 1:1): R_{f}= 0.6;
¹H NMR (CDCl₃): δ 3.87 (s, 3H, OCH3), 6,84 (dd, 1H, J1 = 7.8 Hz, J2 = 3,13 Hz), 6.97 (m, 2H), 7.16 (t, 1H, J = 7.8Hz), 7.25 (t, 1H, J = 7.8Hz), 7.36 (d, 1H, J = 7.8 Hz), 7.44 (d, 1H, J = 2.0 Hz), 8.26 (d, 1H, J = 7.8 Hz), 8.45 (s, 1H, NH).

### (b) 6-Methoxy-3-(2-nitro-phenylsulfanyl)-1-pyridin-2-ylmethyl-1H-indole (Compound 9, Structure 3 of Scheme I, where R¹ = 2-pyridyl, R² = 2-nitrophenyl, R³=R⁴=H, R⁵=OMe

To a solution of 6-Methoxy-3-(2-nitro-phenylsulfanyl)-1*H*-indole (1.5 g, 5 mmol) in DMF (150 mL) was added NaH (60% in oil; 500 mg, 12.5 mmol) in small portions, over a 4 minute period. The resulting dark solution was stirred for 5 min. at room temperature. Then 2-picolyl chloride hydrochloride (984 mg, 6 mmol) was added in small portions, over a 2 minute period. During stirring at room temperature (2h) the colour of the solution slightly changed from dark to yellow. Ethyl acetate (100 mL) was added and the mixture was washed with 2% aqueous citric acid (2x100 mL) and water (100 mL). The organic phase was dried (MgSO4) and the solvent was evaporated. The crude product was purified by crystallisation (ethyl acetate/heptane) to give the title compound (1.437 g, yield = 74%).
HPLC : 10.3 min, purity 99.6%, TLC (heptane/ethyl acetate 1:1): E_{f}= 0.3;
¹H NMR (CDCl₃): δ 3.80 (s, 3H, OCH3), 5.47 (s, 2H, NCH₂), 6.82 (m, 2H), 6.88 (d, 1H, J = 7.8 Hz), 7.01 (dd, 1H, J1 = 7.8 Hz, J2 = 1,6 Hz), 7.15-7.19 (m, 1H), 7.25 (m, 2H), 7.37 (d, 1H, j = 8.6 Hz), 7.60-7.64 (m, 1H), 8.26 (dd, 1H, J1 = 7.8 Hz, J2 = 1.6 Hz), 8.63 (d, 1H, J = 5.88 Hz).

According to General method 3 the following compounds were prepared:

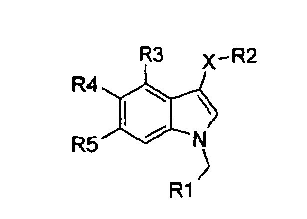

**Table 3 Compounds synthesised according to General Method 3**

| **Compound number** | **X** | **R¹** | **R²** | **R³** | **R⁴** | **R⁵** | **MWt** | **LCMS (MH+)** | **LCMS ret. Time^{a} (min)** |
|---|---|---|---|---|---|---|---|---|---|
| **1** | S | 2,5-difluorophenyl | 2-nitrophenyl | H | H | OMe | 426.44 | 427 | 4.07 |
| **2** | S | 2,fluoro,3-methylphenyl | 2-nitrophenyl | H | H | OMe | 422.48 | 423 | 4.23 |
| **3** | S | 2-chlorophenyl | 2-nitrophenyl | H | H | OMe | 424.91 | 425 | 4.17 |
| **4** | S | 2-cyanophenyl | 2-nitrophenyl | H | H | OMe | 415.47 | 416 | 3.97 |
| **5** | S | 2-fluorophenyl | 2-nitrophenyl | H | H | OMe | 408.45 | 409 | 4.10 |
| **6** | S | 2-methyl,3-nitrophenyl | 2-nitrophenyl | H | H | OMe | 449.48 | 450 | 4.10 |
| **7** | S | 2-methylphenyl | 2-nitrophenyl | H | H | OMe | 404.49 | 405 | 4.16 |
| **8** | S | 2-nitrophenyl | 2-nitrophenyl | H | H | OMe | 435.46 | 436 | 4.08 |
| **9** | S | 2-pyridyl | 2-nitrophenyl | H | H | OMe | 391.45 | | 10.30^{b} |
| **11** | S | 2-tetrahydropyranyl | 2-nitrophenyl | H | H | OMe | 398.48 | 399 | 4.68 |
| **12** | S | 2-trifluoromethylphenyl | 2-nitrophenyl | H | H | OMe | 458.46 | 459 | 4.29 |
| **13** | S | 3-(5-methylisoxazolyl) | 2-nitrophenyl | H | H | OMe | 395.44 | 396 | 4.66 |
| **14** | S | 3,4-dichlorophenyl | 2-nitrophenyl | H | H | OMe | 459.35 | 459 | 4.33 |
| **15** | S | 3,5-dichlorophenyl | 2-nitrophenyl | H | H | OMe | 459.35 | 459 | 4.36 |
| **67** | S | 3-chlorophenyl | 2-nitrophenyl | H | H | OMe | 424.91 | 425 | 4.34 |
| **68** | S | 3-cyanophenyl | 2-nitrophenyl | H | H | OMe | 415.47 | 416 | 3.92 |
| **69** | S | 3-fluoro,5-trifluoromethylphenyl | 2-nitrophenyl | H | H | OMe | 476.45 | 477 | 4.42 |
| **70** | S | 3-fluoro,6-trifluoromethylphenyl | 2-nitrophenyl | H | H | OMe | 476.45 | 477 | 4.27 |
| **71** | S | 3-fluorophenyl | 2-nitrophenyl | H | H | OMe | 408.45 | 409 | 4.22 |
| **72** | S | 3-methoxyphenyl | 2-nitrophenyl | H | H | OMe | 420.49 | 421 | 4.04 |
| **73** | S | 3-methylphenyl | 2-nitrophenyl | H | H | OMe | 404.49 | 405 | 4.16 |
| **74** | S | 3-nitrophenyl | 2-nitrophenyl | H | H | OMe | 435.46 | 436 | 4.00 |
| **75** | S | 3-pyridyl | 2-nitrophenyl | H | H | OMe | 391.45 | 392 | 3.28 |
| **76** | S | 3-trifluoromethyl,4-chlorophenyl | 2-nitrophenyl | H | H | OMe | 492.90 | 493 | 4.33 |
| **77** | S | 3-trifluoromethyloxyphenyl | 2-nitrophenyl | H | H | OMe | 474.46 | 475 | 4.43 |
| **78** | S | 3-trifluoromethylphenyl | 2-nitrophenyl | H | H | OMe | 458.46 | 459 | 4.21 |
| **79** | S | 4-(2-methylthiazolyl) | 2-nitrophenyl | H | H | OMe | 411.50 | 412 | 4.39 |
| **80** | S | 4-(3,5-dimethylisoxazolyl) | 2-nitrophenyl | H | H | OMe | 409.46 | 410 | 4.49 |
| **81** | S | 4-chlorophenyl | 2-nitrophenyl | H | H | OMe | 424.91 | 425 | 4.22 |
| **82** | S | 4-cyanophenyl | 2-nitrophenyl | H | H | OMe | 415.47 | 416 | 3.93 |
| **83** | S | 4-fluorophenyl | 2-nitrophenyl | H | H | OMe | 408.45 | 409 | 4.07 |
| **84** | S | 4-methoxyphenyl | 2-nitrophenyl | H | H | OMe | 420.49 | 421 | 4.03 |
| **85** | S | 4-methylphenyl | 2-nitrophenyl | H | H | OMe | 404.49 | 405 | 4.19 |
| **86** | S | 4-morpholinyl | 2-nitrophenyl | H | H | OMe | 413.50 | 414 | 3.54 |
| **87** | S | 5-(2-chlorothiazolyl) | 2-nitrophenyl | H | H | OMe | 431.92 | 432 | 4.61 |
| **88** | S | 5-(2-chlorothiophenyl) | 2-nitrophenyl | H | H | OMe | 430.93 | 431 | 4.92 |
| **89** | S | cyclohexyl | 2-nitrophenyl | H | H | OMe | 396.51 | 397 | 5.11 |

| | | | | | | | | | |
|---|---|---|---|---|---|---|---|---|---|
| a) 7 min LCMS method was used unless stated otherwise. b) a 20 min run on an HPLC system was used. | | | | | | | | | |

### Example 7

### 1-(3,5-Difluoro-benzyl)-6-methoxy-3-(pyridin-2-ylsulfanyl)-1H-indole (Compound 65, Structure 3 of Scheme I, where R¹ = 3,5-difluorophenyl, R² = 2-pyridyl, R³=R⁴=H, R⁵=OMe)

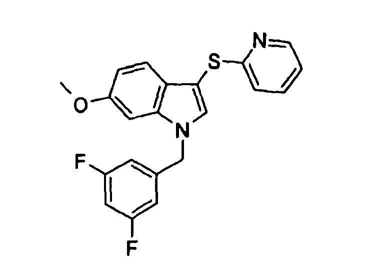

Under a nitrogen atmosphere: to a cooled (-10°C) solution of 2-mercaptopyridine (25 mg, 0.22 mmol) in CCl₄ (2 mL) was passed Cl₂-gas during a period of 1 minute. The solvent was evaporated and a solution of 1-(3,5-Difluoro-benzyl)-6-methoxy-1*H*-indole (25 mg, 0.09 mmol) in Et₂O (2 mL) was added dropwise. The reaction mixture was concentrated and the crude product was purified by preparative LCMS to give the title compound (7.77 mg, yield = 23%).
LCMS : 3.95 min (100%, MH⁺ 383); TLC (heptane/ethyl acetate 1:1): R_{f}= 0.6;
¹H-NMR (CDCl₃) : δ 2.23 (s, 1H, NH), 3.82 (s, 1H, OCH₃), 6.65 (m, 2H), 6.73 (d, 1H, J = 2.7 Hz), 6.73 -6.78 (m, 1H), 6.80 (d, 1H, J = 7.8 Hz), 6.87 (dd, 1H, J1 = 7.8 Hz, J2 = 1.6 Hz), 7.00 (ddd, 1H, J1 = 9.8 Hz, J2 = 4.7 Hz, J3 = 0.8 Hz), 7.35 (s, 1H), 7.42 (ddd, 1H, J1 = J2 = 7.8 Hz, J3 = 1. 6 Hz), 7.50 (d, 1H, 8.6 Hz), 8.46 (d, 1H, J = 4.7 Hz).

### Example 8

### {2-[1-(3,5-Difluoro-benzyl)-6-methoxy-1H-indol-3-ylsulfanyl]-phenyl}-methanol (Compound 16 Structure 8 of Scheme III, where R¹ = 3,5-difluorophenyl, R³=R⁴=H, R⁵=OMe)

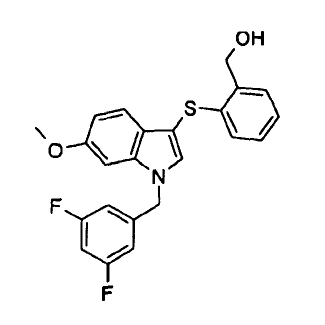

### a) 2-[1-(3,5-Difluoro-benzyl)-6-methoxy-1H-indol-3-ylsulfanyl]-benzoic acid methyl ester Compound 19, Structure 7 of Scheme III, where R¹ = 3,5-difluorophenyl, R³=R⁴=H, R⁵=OMe)

Chlorine gas was bubbled through CCl₄ (5 mL) at - -10°C for 3 min. Then a solution of methylthiosalicylate (91 µL, 0.66 mmol) in CCl₄ (2 mL) was added. The mixture was stirred at -10°C for 5 min and then at room temperature for 15 min. The mixture was concentrated and redissolved in CH₂Cl₂ (5 mL). To this solution a solution of 1-(3,5-Difluoro-benzyl)-6-methoxy-1*H*-indole (121 mg, 0.443 mmol) in CH₂Cl₂ (3 mL) was added dropwise. The mixture was stirred at room temperature for 2 h and was concentrated. The crude product was purified by column chromatography (ethyl acetate/heptane 1:9) to give the title compound as a white solid (161 mg, 83% yield). LCMS: 4.72 min (MH⁺ = 440); ¹H-NMR (CDCl₃) : δ 3.82 (s, 3H, ArOCH₃), 3.99 (s, 3H, CO₂CH₃), 5.30 (s, 2H, CH₂Ar), 6.62-6.69 (m, 2H), 6.71-6.77 (m, 2H), 6.83 (dd, 1H, J1 = 8.6 Hz, J2 = 2.4 Hz), 6.87 (dd, 1H, J1 = 8.4 Hz, J2 = 1.2 Hz), 7.06-7.11 (m, 1H, 7.15-7.20 (m, 1H), 7.30 (s, 1H), 7.44 (d, 1H, J = 8.6 Hz), 8.02 (dd, 1H, J1 = 8.0 Hz, J2 = 1.2 Hz).

### b) {2-[1-(3,5-Difluoro-benzyl)-6-methoxy-1H-indol-3-ylsulfanyl]-phenyl}-methanol (Compound 16, Structure 8 of Scheme III, where R¹ = 3,5-difluorophenyl, R³=R⁴=H, R⁵=OMe)

Under a nitrogen atmosphere: to a solution of 2-[1-(3,5-Difluoro-benzyl)-6-methoxy-1*H*-indol-3-ylsulfanyl]-benzoic acid methyl ester (13.5 mg, 0.031 mmol) in THF (2 mL) was added LiAlH₄ (1.0 M in THF, 0.040 mL, 0.040 mmol). The mixture was stirred at room temperature for 3 h. Then ethyl acetate (25 mL) was added and the mixture was washed with 3% aqueous citric acid (2x25 mL) and brine (25 mL). The organic phase was dried (MgSO₄) and concentrated to give a colourless oil (15 mg). The crude product was purified by prep. HPLC to give the title compound as a colourless oil (5.6 mg, 44% yield).
LCMS: 4.47 min (95.6%, MH⁺= 412); ¹H-NMR (CDCl₃) : δ 3.49 (s, 1H, OH), 3.81 (3, 3H, OCH₃), 4.92 (s, 2H, CH₂OH), 5.28 (s, 2H, CH₂Ar), 6.62-6.66 (m, 2H), 6.70-6.77 (m, 2H), 6.83 (dd, 1H, J1 = 8.8 Hz, J2 = 2.4 Hz), 6.89 (dd, 1H, J1 = 8.0 Hz, J2 = 1.2 Hz), 7.02-7.07 (m, 1H), 7.08-7.13 (m, 1H), 7.29 (s, 1H), 7.38 (dd, 1H, J1 = 8.0 Hz, J2 = 1.2 Hz), 7.44 (d, 1H, J = 8.8 Hz).

### Example 9

### 2-[1-(3,5-Difluoro-benzyl)-6-methoxy-1H-indol-3-ylsulfanyl]-benzamide (Compound 18, Structure 11 of Scheme III, where R¹ = 3,5-difluorophenyl, R³=R⁴=H, R⁵=OMe)

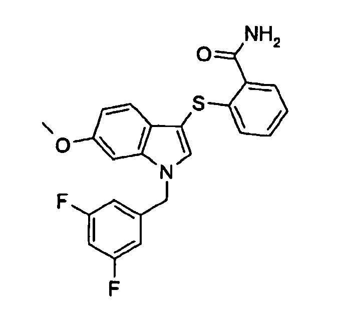

### a) 2-[1-(3,5-Difluoro-benzyl)-6-methoxy-1H-indol-3-ylsulfanyl]-benzoic acid (Structure 9 of Scheme III, where R¹ = 3,5-difluorophenyl. R³=R⁴=H, R⁵=OMe)

To a solution of 2-[1-(3,5-Difluoro-benzyl)-6-methoxy-1*H*-indol-3-ylsulfanyl]-benzoic acid methyl ester (97 mg, 0.221 mmol) in THF (10 mL) was added a solution of LiOH·H₂O (71 mg, 1.69 mmol) in water (10 mL). The mixture was stirred at room temperature for 17 h and then at 60°C for 24 h and was then acidified with 3% aqueous citric acid. Ethyl acetate (50 mL) was added and the mixture was washed with 3% aqueous citric acid (2x50 mL) and brine (50 mL). The organic phase was dried (MgSO₄) and concentrated to give a yellow solid (104 mg). The crude product was recrystallised from ethyl acetate/heptane to give the title compound as a yellow powder (49 mg, 52% yield).
LCMS: 4.37 min (MH⁺= 426); ¹H-NMR (CDCl₃) : δ 3.77 (s, 3H, OCH₃), 5.51 (s, 2H, CH₂Ar), 6.69 (d, 1H, J = 8.0 Hz), 6.76 (dd, 1H, J1 = 8.4 Hz, J2 = 2.4 Hz), 6.98-7.04 (m, 2H), 7.11-7.15 (m, 1H), 7.16-7.25 (m, 4H), 7.83 (s, 1H), 7.92 (dd, 1H, J1 = 8.0Hz, J2 = 1.6 Hz).

**General method 4:** Reaction of an amine with a carboxylic acid of structure **9,** with TBTU and DIPEA, to give amides of structure **3,** in which R² = phenyl-2-carboxamide , exemplified by compounds of structure **10** and **11** (Scheme **III**).

### b) 2-[1-(3,5-Difluoro-benzyl)-6-methoxy-1H-indol-3-ylsulfanyl]-benzamide (Compound 18, Structure 11 of Scheme III, where R¹ = 3,5-difluorophenyl R³=R⁴=H, R⁵=OMe)

Through a solution of 2-[1-(3,5-Difluoro-benzyl)-6-methoxy-1*H*-indol-3-ylsulfanyl]-benzoic acid (36 mg, 0.085 mmol), TBTU (28 mg, 0.087 mmol) and DIPEA (30 µL, 0.172 mmol) in DMF (1 mL) was bubbled NH₃ gas for 10 min. The mixture was then stirred at room temperature for 3 days. Then 3% aqueous citric acid (0.4 mL) was added and the crude reaction mixture was purified over a RP-SPE cartridge (2g sorbent; 25% aqueous methanol to 100% methanol) to give a white solid (34 mg). The crude product was purified by column chromatography (ethyl acetate) to give the title compound as a colourless oil, which solidifies on standing (31 mg, yield: 86%).
LCMS: 4.20 min (MH⁺ = 425); ¹H-NMR (CDCl₃) : δ 3.81 (s, 3H, OCH₃), 5.39 (s, 2H, CH₂Ar), 6.05 (s, br, 1H, NH), 6.28 (s, br, 1H, NH), 6.61-6.67 (m, 2H), 6.70-6.72 (m, 1H), 6.73-6.77 (m, 1H), 6.83 (dd, 1H, J1 = 8.8 Hz, J2 = 2.0 Hz), 6.92 (dd, 1H, J1 = 8.0 Hz, J2 = 1.2 Hz), 7.07-7.17 (m, 2H), 7.31 (s, 1H), 7.45 (d, 1H, J = 8.8 Hz), 7.60 (dd, 1H, J1 = 8.0 Hz, J2 = 1.2 Hz).

### Example 10

### 2-[1-(3,5-Difluoro-benzyl)-6-methoxy-1H-indol-3-ylsulfanyl]-N-methyl-benzamide (Compound 17, Structure 10 of Scheme III, where R¹ = 3,5-difluorophenyl, R³=R⁴=H, R⁵=OMe)

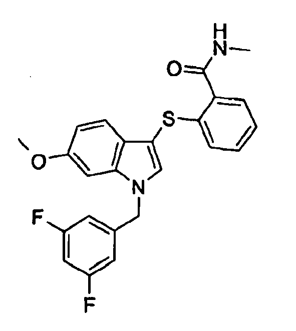

According to general **method 4** compound **17** was synthesised from methylamine. LCMS: 4.07 min (MH⁺ = 439).

### Example 11

### 2-[1-(3,5-Difluoro-benzyl)-6-methoxy-1H-indol-3-ylsulfanyl]-benzonitrile_(Compound 20, Structure 12 of Scheme III, where R¹ = 3,5-difluorophenyl, R³=R⁴=H, R⁵=OMe)

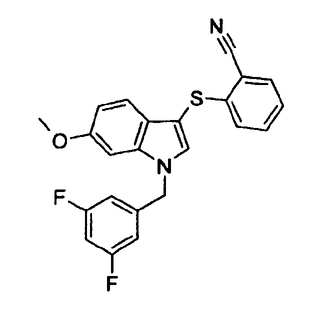

To a cooled (0°C) solution of 2-[1-(3,5-Difluoro-benzyl)-6-methoxy-1*H*-indol-3-ylsulfanyl]-benzamide (8 mg, 0.019 mmol) and Et₃N (10 µL, 0.072 mmol) in CH₂Cl₂ (1.5 mL) was added Tf₂O (6 µL, 0.036 mmol). The mixture was stirred at 0°C for 2 h and then at room temperature for 22 h. Water (25 mL) was added and the mixture was extracted with CH₂Cl₂ (2x25 mL). The combined organic phases were dried (Na₂SO₄) and concentrated to give a brown oil (25 mg). The crude product was purified by column chromatography (ethyl acetate/heptane 1:2) to give the title compound as a pale pink oil (3 mg, yield: 39%).
LCMS: 4.57 min (MH⁺ = 407); ¹H-NMR (CDCl₃): δ 3.82 (s, 3H. OCH₃), 5.30 (s, 2H, CH₂Ar), 6.62-6.67 (m, 2H), 6.71-6.73 (m, 1H), 6.74-6.78 (m, 1H), 6.86 (dd, 1H, J1 = 8.8 Hz, J2 = 2.0 Hz), 6.91 (d, 1H, J = 8.4 Hz), 7.11-7.15 (m, 1H), 7.26-7.30 (m, 1H), 7.37 (s, 1H), 7.46 (d, 1H, J = 8.8 Hz), 7.57-7.60 (m, 1H).

### Example 12

### 1-(3,5-Difluoro-benzyl)-3-(2-nitro-phenylsulfanyl)-1H-indole-6-carbonitrile (Compound 26, Structure 17 of Scheme IV, where R¹ = 3,5-difluorophenyl, R² = 2 - nitrophenyl, R³=R⁴=H)

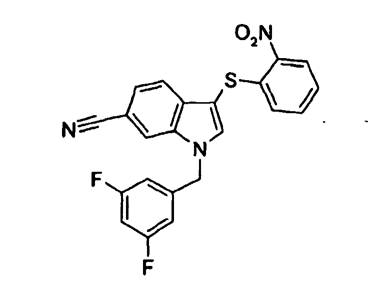

To a cooled (0°C) suspension of 1-(3,5-Difluoro-benzyl)-3-(2-nitro-phenylsulfanyl)-1*H-*indole-6-carboxylic acid amide (15 mg, 0.034 mmol) and Et₃N (10 µL, 0.072 mmol) in CH₂Cl₂ (1.5 mL) was added Tf₂O (13 µL, 0.077 mmol). The mixture was stirred at 0°C for 2 h and then at room temperature for 20 h. Water (25 mL) was added and the mixture was extracted with CH₂Cl₂ (2x25 mL). The combined organic phases were dried (Na₂SO₄) and concentrated to give the title compound as a yellow solid (13 mg, yield: 91%).
LCMS: 4.55 min (MH⁺= not detectable); ¹H-NMR (CDCl₃): δ 5.42 (s, 2H, CH₂Ar), 6.63-6.69 (m, 2H), 6.78-6.85 (m, 2H), 7.21-7.25 (m, 1H), 7.28-7.33 (m, 1H), 7.44 (dd, 1H, J1 = 8.4 Hz, J2 = 1.2 Hz), 7.62 (d, 1H, J = 8.4 Hz), 7.66 (s, 1H), 8.29 (dd, 1H, J1 = 8.4 Hz, J2 = 1.6 Hz).

### Example 13

### 1-(3,5-Difluoro-benzyl)-6-methoxy-3-(2-nitro-benzenesulfinyl)-1H-indole (Compound 64, Structure 5 of Scheme II, where R¹ = 3,5-difluorophenyl, R² = 2-nitrophenyl, R³=R⁴= H, R⁵=OMe)

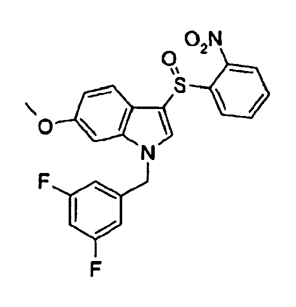

To a solution of 1-(3,5-Difluoro-benzyl)-6-methoxy-3-(2-nitro-phenylsulfanyl)-*1*H-indole (40 mg, 0.09 mmol) in CH₂Cl₂ (4 mL) was added meta-chloroperbenzoic acid (14.5, 0.08 mmol). The solution was stirred at room temperature for 2 h. The solvent was evaporated and the crude product was purified by prep LC-MS to give the title compound (14.3 mg, yield = 34%).
HPLC : 4.13 min, purity 100%; TLC (heptane/ethyl acetate 1:1) : Rf = 0.4; ¹H NMR (CDCl₃): δ 3.66 (s, 3H, OCH3), 5.12 (s, 2H), 6.57 (m, 3H), 6.75 (m, 2H), 7.47 (d, 1H, J = 7.8 Hz), 7.52 (s, 1H), 7.66-7.70 (m, 1H), 8.28-8.32 (m, 1H), 8.23 (dd, 1H, J1 = 7.8 Hz, J2 = 1.9 Hz), 8.80 (dd, 1H, J1 = 7.8 Hz, J2 = 3.1 Hz).

### Example 14

### 1-(3,5-Difluoro-benzyl)-6-methoxy-3-(2-nitro-benzenesulfonyl)-1H-indole (Compound 62, Structure 6 of Scheme II, where R¹ = 3,5-difluorophenyl, R² = 2-nitrophenyl, R³=R⁴=H, R⁵=OMe)

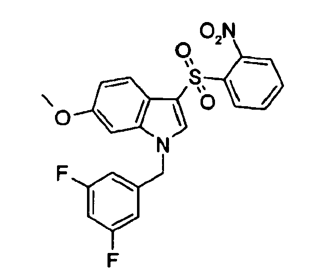

To a solution of 1-(3,5-Difluoro-benzyl)-6-methoxy-3-(2-nitro-phenylsulfanyl)-1*H-*indole (40 mg, 0.09 mmol) in CH₂Cl₂ (4 mL) was added meta-chloroperbenzoic acid (46.5 mg, 0.27 mmol). The solution was stirred at room temperature for 6 h. The solvent was evaporated and the crude product was purified by LC-MS to give the title compound (14.5 mg, yield = 34%).
HPLC: 4.38 min. purity 100%; TLC (heptane/ethyl acetate 1:1) : Rf = 0.6; ¹H NMR (CDCl₃): δ 3.71 (s, 3H, OCH3), 5.25 (s, 2H), 6.58 (d, 1H, J = 3.1), 6.62 (m, 1H), 6.07-6.11 (m, 1H), 6.88 (dd, 1H, J1 = 7.8 Hz, J2 = 3.1 Hz), 7.63 (m, 4H), 7.72 (d, 1H, J = 7.8 Hz), 7.83 (s, 1H), 8.37 (dd, 1H, J1 = 7.8 Hz, J2 = 1.9 Hz).

### Example 15

### 6-Methoxy-3-(2-nitro-phenylsulfanyl)-1-pyridin-2 ylmethyl-1H-indole monohydrochloride (Compound 10).

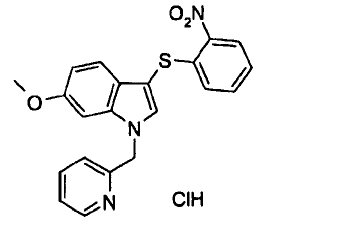

To a solution of 6-Methoxy-3-(2-nitro-phenylsulfanyl)-1-pyridin-2-ylmethyl-1*H*-indole in methylene chloride was passed HCl-gas till the organic salt crystallised. The title compound was isolated by filtration.
LC-MS : 4.04 min (MH⁺= 392); ¹H NMR (DMSO): δ 3.78 (s, 3H, OCH3), 5.78 (s, 2H, NCH₂), 6.77 (dd, 1H, J1 = 7.8 Hz, J2 = 2.0 Hz), 6.93 (dd, 1H, J1 = 7.8 Hz, J2 = 0.8 Hz), 7.22 (d, 1H, J = 7.8 Hz), 7.26 (d, 1H, J = 2.0 Hz), 7.34 (m, 2H), 7.48-7.52 (m, 1H), 7.59 (t, 1H, J = 7.0 Hz), 7.96 (s, 1H), 8.08 (t, 1H, J = 7.0 Hz), 8.27 (dd, 1H, J1 = 7.8 Hz, J2 = 1.6 Hz), 8.74 (d, 1H, J = 6.7 Hz).

### Example 16

### 1-(3,5-Difluoro-benzyl)-3-(2-nitro-phenylsulfanyl)-1H-indol-6-ylamine (Compound 57, Structure 23 of Scheme V, where R¹ = 3,5-difluorophenyl, R² = 2-nitrophenyl R³=R⁴=H)

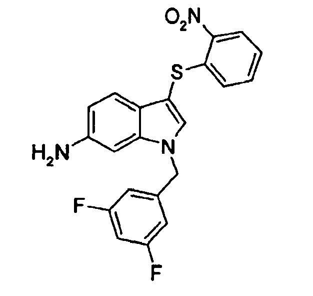

### a) 1-(3,5-Difluoro-benzyl)-6-nitro-1H-indole (Structure 19 of (Scheme V, where R¹= 3,5-difluorophenyl, R³=R⁴=H)

To a solution of 6-nitroindole (162 mg, 1.0 mmol) in DMF (4 mL) was added NaH (60% dispersion on oil; 80 mg, 2.0 mmol). The resulting dark solution was stirred at room temperature for 15 minutes. 3,5-difluorobenzyl bromide (129 µL, 1,1 mmol) was added. The reaction mixture was stirred overnight, poured into acidified water and extracted twice with ethyl acetate. The combined organic layers were dried over Na₂SO₄ and concentrated *in vacuo.*
The product was purified over a SiO₂ column (heptane/ethyl acetate 9:1) to give the title compound as a yellow solid (270 mg, yield = 94%).
LCMS: 5.54 min (100.0%, MH⁺= 289); ¹H NMR (CDCl₃): δ 5.40 (s, 2H, CH₂Ar), 6.58 (m, 2H), 6.71 (d, 1H, J = 3.5 Hz), 6.75 (m, 1H), 7.26 (s, 1H), 7,41 (d, 1H, J = 3.5 Hz), 7.71 (d, 1H, J = 8.3 Hz), 8.05 (dd, 1H, J1 = 8.3 Hz, J2 = 2.0 Hz), 8.21 (d, 1H, J = 2.0 Hz).

### b) 1-(3,5-Difluoro-benzyl)-1H-indol-6-ylamine (Structure 20 of Scheme V, where R¹ = 3,5-difluorophenyl, R³=R⁴=H)

To a solution of 1-(3,5-Difluoro-benzyl)-6-nitro-1*H*-indole (144 mg, 0.5 mmol) in ethanol (20 mL) was added hydrochloric acid 37% (80 µL) and SnCl₂.2H₂O (600 mg). The reaction mixture was stirred at 60 °C for 40 h and was then concentrated *in vacuo.* The residue was poured into ethyl acetate and a concentrated NaHCO₃-solution was added. The two-layer phase system was filtered over decalite to get rid of the tin salts and the filtrate was twice extracted with ethyl acetate. The combined organic layers were dried over Na₂SO₄ and concentrated *in vacuo.* The product was purified over a SiO₂ column (heptane/ethyl acetate 9:1) to give the title compound as a colourless oil (89 mg, yield = 69%).
LCMS: 3.20 min (97.7%, MH⁺= 259); ¹H NMR (CDCl₃): δ 5.12 (s, 2H, CH₂ Ar), 6.52 (m, 2H), 6.56 (d, 1H, J = 3.5 Hz), 6.63 (m, 1H), 7.97 (dd, 1H, J1 = 8.3 Hz, J2 = 2.0 Hz), 7.13 (d, 1H, J = 3.5 Hz), 7,17 (d, 1H, J = 2.0 Hz), 7.55 (d, 1H, J = 8.3 Hz).

### c) 1-(3,5-Difluoro-benzyl)-3-(2-nitro-phenylsulfanyl)-1H-indol-6-ylamine (Compound 57, Structure 23 of Scheme V, where R¹ = 3,5-difluorophenyl, R² = 2-nitrophenyl, R³=R⁴=H)

To a solution of 1-(3,5-Difluoro-benzyl)-1*H*-indol-6-ylamine (25 mg, 0.1 mmol) in DCM (4 mL) was added 2-nitrobenzenesulfenyl chloride. The resulting yellow solution was stirred at room temperature for 1 hour. The mixture was concentrated *in vacuo* and purified over a SiO₂ column (heptane/ethyl acetate 9:1) to give the title compound as an orange solid (11.6 mg, yield = 28%).
LCMS: 5.11 min (100.0%, MH⁺= 412); ¹H NMR (CDCl₃): δ 5.22 (s, 2H, CH₂ Ar), 6.56 (m, 2H), 6.65 (m, 1H), 6.84 (dd, 1H, J1 = 8.2 Hz, J2 = 1.2 Hz), 7.00 (dd, 1H, J1 = 8.2 Hz, J2 = 1.2 Hz), 7.19 (m, 1H), 7.3 0 (d, 1H, J =1.2 Hz), 7.31 (s, 1H), 7.37 (d, 1H, J = 8.2 Hz) 7.49 (m, 1H), 8.27 (dd, 1H, J1 = 8.2 Hz, J2 = 1.2 Hz).

### Example 17

### N-[1-(3,5-Difluoro-benzyl)-3-(2-nitro-phenylsulfanyl)-1H-indol-6-yl]-acetamide (Compound 58, Structure 22 of Scheme V, where R¹ = 3,5-difluorophenyl, R² = 2-nitrophenyl, R³=R⁴=H, Z = methyl)

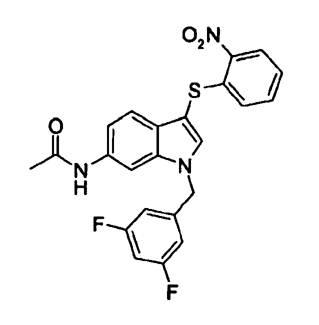

### General method 5: Reaction of an acid anhydride with a 6-aminoindole of structure 20 to give compounds of structure 21, followed by sulfanylation of the indole 3-position to give compounds of structure 22 (Scheme V).

### a) N-[1-(3,5-Difluoro-benzyl)-1H-indol-6-yl]-acetamide (Structure 21 of Scheme V, where R¹ = 3,5-difluorophenyl, R³=R⁴=H, Z = methyl)

To a solution of 1-(3,5-Difluoro-benzyl)-1*H*-indol-6-ylamine (25 mg, 0.1 mmol) in DCM (4 mL) was added pyridine (25 µL) and acetic anhydride (9.8 µL, 0.11 mmol) and stirred overnight at room temperature. The reaction mixture was poured into 10 mL of water and neutralised with NaHCO₃ and extracted twice with DCM. De combined organic layers were dried over Na₂SO₄ and concentrated *in vacuo* to give the crude compound as a off white solid (23.3 mg, yield = 84%). The product was used without further purification.
LCMS: 4.10 min (97.4%, MH⁺ = 301); ¹H NMR (CDCl₃): δ 2.18 (s, 3H, CH₃CON), 5.28 (s, 2H, CH₂Ar), 6.53 (d, 1H, J = 3.1), 6.58 (m, 2H), 6.68 (m, 1H), 6.87 (dd, 1H, J1 = 8.3 Hz, J2 = 1.6 Hz), 7.07 (d, 1H, J = 3.1 Hz), 7,55 (d, 1H, J = 8.3 Hz), 7.90 (s, 1H).

### b) N-[1-(3,5-Difluoro-benzyl)-3-(2-nitro-phenylsulfanyl)-1H-indol-6-yl]-acetamide (Compound 58, Structure 22 of Scheme V, where R¹ = 3,5-difluorophenyl, R² = 2-nitrophenyl, R³=R⁴=H, Z = methyl)

To a solution of *N*-[1-(3,5-Difluoro-benzyl)-1*H*-indol-6-yl]-acetamide (23.3 mg, 0.08 mmol) in diethyl ether (4 mL) was added 2-nitrobenzenesulfenyl chloride (14.7 mg, 0.09 mmol). The resulting yellow solution was stirred overnight at room temperature. The mixture was concentrated *in vacuo* and purified over a SiO₂ column (heptane/ethyl acetate 9:1) to give the title compound as a yellow solid (35.0 mg, yield = 96%). LCMS: 4.26 min (98.6%, MH⁺ = 454); ¹H NMR (DMSO): δ 2.03 (s, 3H, CH₃CON), 5.51 (s, 2H, CH₂Ar), 6.89 (dd, 1H, J = 8.3 Hz, J2 = 1.2 Hz), 6.95 (m, 2H), 7.17 (dd, 1H, J1 = 8.3 Hz, J2 = 2.0 Hz), 7.19 (m, 1H), 7.27 (d, 1H, J = 8.3 Hz), 7,34 (m, 1H), 7.49 (m, 1H), 7.97 (d, 1H, J = 1.2 Hz), 8.02 (s, 1H), 8.27 (dd, 1H, J1 = 8.3 Hz, J2 = 1.2 Hz).

### Example 18

### N-[1-(3,5-Difluoro-benzyl)-3-(2-nitro-phenylsulfanyl)-1H-indol-6-yl]-2,2,2-trifluoroacetamide (Compound 59, Structure 22 of Scheme V, where R¹ = 3,5-difluorophenyl, R² = 2-nitrophenyl, R³=R⁴=H, Z = trifluoromethyl)

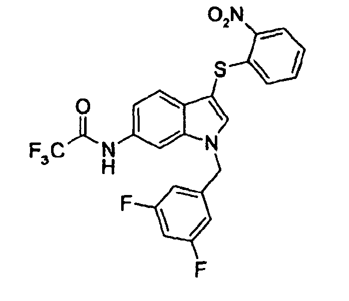

According to General method 5, step a): *N*-[1-(3,5-Difluoro-benzyl)-1*H*-indol-6-yl]-2,2,2-trifluoro-acetamide was prepared using 1-(3,5-Difluoro-benzyl)-1*H*-indol-6-ylamine (17.7 mg, 69 µmol), pyridine (17 µL) and trifluoroacetic anhydride (9,68 µL, 70 µmol). The compound was purified over a SiO₂ column (heptane/ethyl acetate 9:1) to give the title compound as a white solid (10.9 mg, yield = 61%).
LCMS:4.41 min (82.1%, MH⁺ = 355)
According to General method 5, step b): *N*-[1-(3,5-Difluoro-benzyl)-3-(2-nitrophenylsulfanyl)-1*H*-indol-6-yl]-2,2,2-trifluoro-acetamide was prepared using *N*-[1-(3,5-Difluoro-benzyl)-1*H*-indol-6-yl]-2,2,2-trifluoro-acetamide (10.9 mg, 31 µmol) and 2-nitrobenzenesulfenyl chloride (5.7 mg, 37 µmol). The compound was purified over an HPLC column (MeCN/H₂O) to give the title compound as a yellow solid (10.2 mg, yield = 65%).
LCMS: 4.65 min (89.0%, MH⁺ = 508); ¹H NMR (DMSO): δ 5.56 (s, 2H, CH₂Ar), 6.87 (dd, 1H, J = 8.3 Hz, J2 = 1.2 Hz), 7.01 (m, 2H), 7.20 (m, 1H), 7.35 (m, 1H), 7.37 (dd, 1H, J1 = 8.3 Hz, J2 = 2.0 Hz), 7.39 (d, 1H, J = 8.3 Hz), 7.49 (m, 1H), 7,50 (d, 1H, J = 2.0 Hz), 8.15 (s, 1H), 8.28 (dd, 1H, J1 = 8.3 Hz, J2 = 2.0 Hz).

### Example 19

### Compound 97: N-[1-(3,5-Difluoro-benzyl)-3-(2-nitro-phenylsulfanyl)-1H-indol-6-yl]-2-fluoro-acetamide, structure 28 of Scheme VII, where R¹ = 3,5-difluorophenyl, R² = 2-nitrophenyl. R³=R⁴=H, Z¹= monofluoromethyl.

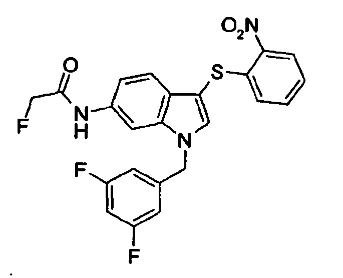

### a) 1-(3,5-Difluoro-benzyl)-6-nitro-1H-indole, structure 19 of Scheme VII, where R¹ = 3,5-difluorophenyl, R²=R³=R⁴=H.

To a solution of 6-nitro-1*H*-indole (19.5 g, 120 mmol) in NMP (500 mL) was added at 0 °C cesium carbonate (39.1 g, 120 mmol). After stirring for 30 min at 0 °C 3,5-difluorobenzyl bromide (30.0 g, 144 mmol) was added and the mixture was allowed to warm to room temperature and stirred for 18 hrs. The mixture was poured into saturated aqueous ammonium chloride (1 L) and extracted with ethyl acetate (2 x 500 mL). The combined organic layers were washed with H₂O (2 x 500 mL) and brine (500 mL) and dried (Na₂SO₄) and concentrated to give the title compound (65 g, yellow solid) which was used without furter purification.

### b) 1-(3,5-Difluoro-benzyl)-1H-indol-6-ylamine, structure 20 of Scheme VII, where R¹ = 3,5-difluorophenyl, R²=R³=R⁴=H.

EtOH 96% (1L) was added to 1-(3,5-difluoro-benzyl)-6-nitro-1*H*-indole and the mixture was stirred at 60 °C until all material was dissolved. 37% HCl (37 mL) was added followed by tin(II)chloride dihydrate (268 g, 1.19 mol) and stirring was continued at 60 °C for 18 hrs. After the mixture was cooled to room temperature the solvent was removed under reduced pressure. Ethyl acetate (800 mL) was added and the mixture was poured into saturated aqueous NaHCO₃ (1 L) and filtered over kieselguhr. The layers were separated and the organic layer was washed with H₂O (2 x 500 mL) and brine (500 mL), dried (Na₂SO₄) and concentrated to afford the the title compound (54 g, brown oil/solid) which was used without further purification.

### c) [1-(3,5-Difluoro-benzyl)-1H-indol-6-yl]-carbamic acid tert-butyl ester, structure 26 of Scheme VII, where R¹ = 3,5-difluorophenyl, R²=R³=R⁴=H.

1-(3,5-Difluoro-benzyl)-1*H*-indol-6-ylamine was dissolved in NMP (1 L) and triethylamine (34 mL, 242 mmol) was added followed by di-*tert*-butyl dicarbonate (53 g, 242 mmol) and the mixture was stirred at room temperature for 18 hrs. The mixure was poured into saturated aqueous NaHCO₃ (1.5 L) and extracted with ethyl acetate (3 x 750 mL). The combined organic layers were washed with H₂O (1 L) and brine (1 L), dried (Na₂SO₄) and concentrated. The crude product was purified using column chromatography (heptane/ethyl acetate 4:1) to afford the title compound (39 g, 109 mmol, 91% (three steps), white solid).

### d) [1-(3,5-Difluoro-benzyl)-3-(2-nitro-phenylsulfanyl)-1H-indol-6-yl]-carbamic acid tert-butyl ester, structure 27 of Scheme VII where R¹ = 3,5-difluorophenyl, R² = 2-nitrophenyl, R³=R⁴=H.

To a solution of [1-(3,5-difluoro-benzyl)-1*H*-indol-6-yl]-carbamic acid *tert*-butyl ester (39 g, 109 mmol) in diethylether (750 mL) was added 2-nitrobenzenesulfenyl chloride (20.4 g, 109 mmol) and the reaction mixture was stirred at room temperature for 18 hrs. The mixture was poured into saturated aqueous NaHCO₃ (1 L) and extracted with ethyl acetate (2 x 300 mL). The combined organic layers were washed with H₂O (400 mL) and brine (400 mL), dried and concentrated to afford the title compound (56 g, yellow solid) which was used without further purification.

### e) 1-(3,5-Difluoro-benzyl)-3-(2-nitro-phenylsulfanyl)-1H-indol-6-ylamine structure 23 of Scheme VII, where R¹ = 3,5-difluorophenyl, R² = 2-nitrophenyl, R³=R⁴=H.

[1-(3,5-Difluoro-benzyl)-3-(2-nitro-phenylsulfanyl)-1*H*-indol-6-yl]-carbamic acid *tert-*butyl ester was dissolved in CH₂Cl₂ (1 L) and under nitrogen atmosphere trifluoroacetic acid (48 mL, 624 mmol) was added dropwise at room temperature. After stirring for 1 h another portion of trifluoroacetic acid (48 mL, 624 mmol) was added and the reaction mixture was stirred for an additional hour. The mixture was diluted with CH₂Cl₂ (500 mL) and H₂O (1 L) and 3N NaOH solution was added until the pH of the mixture was 11-12. The layers were separated and the organic layer was washed with H₂O (500 mL) and brine (500 mL), dried (Na₂SO₄) and concentrated to afford the crude product. The product was purified using column chromatography (heptane/ethylacetate 1:1) to give the title compound (28 g, 68 mmol, 63 % (two steps), red/brown solid).

**General method 6:** Reaction of an acid chloride (method a), carboxylic acid (method b) or sulfonyl chloride (method c) with a 6-aminoindole of structure **23** to give compounds of structure **28** and **29** (Scheme **VII**).
Method a, b and c can all be carried out using the same procedure which is examplified below for method a.

To a solution of 1-(3,5-difluoro-benzyl)-3-(2-nitro-phenylsulfanyl)-1*H*-indol-6-ylamine (300 mg, 0.73 mmol) in CH₂Cl₂ (7 mL) was added at 0 °C triethylamine (0.12 mL, 0.87 mmol). Subsequently monofluoroacetyl chloride (50 µL, 0.73 mmol) was added and the mixture was stirred at room temperature for 18 hrs. The mixture was concentrated and purified using flash column chromatography (CH₂Cl₂/MeOH 99:1) to afford compound **97** (317 mg, 0.67 mmol, 92%, yellow solid).
¹H NMR (DMSO) δ 4.82 (s, 1H), 5.03 (s, 1H), 5.52 (s, 2H), 6.89 (dd, 1H, J1 -= 7.6 Hz, J2 = 1.1 Hz), 6.98-7.01 (m, 2H), 7.20 (tt, 1H, J1 = 9.5 Hz, J2 = 3.0 Hz), 7.28-7.32 (m, 2H), 7.33-7.37 (m, 1H), 7.46-7.51 (m, 1H), 7.98 (s, 1H), 8.08 (s, 1H), 8.27 (dd, 1H, J1 = 7.6 Hz, J2 = 1.1 Hz), 10.15 (s, 1H).

According to **general method 6** the following compounds were prepared:

| Compound **98:** *N*-[1-(3,5-Difluoro-benzyl)-3-(2-nitro-phenylsulfanyl)-1*H*-indol-6-yl]-2,2-difluoro-acetamide, structure **28** of Scheme **VII,** where R¹ = 3,5-difluorophenyl, R² = 2-nitrophenyl, R³=R⁴=H, Z¹= difluoromethyl. Method a was used. | |
|---|---|
| 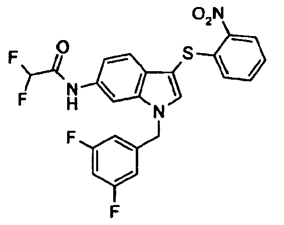 | ¹H-NMR (DMSO): δ 5.55 (s, 2H), 6.23-6.50 (m, 1H), 6.88 (dd, 1H, J1 = 7.6 Hz, J2 = 1.1 Hz), 6.97-7.03 (m, 2H), 7.20 (tt, 1H, J1 = 9.5 Hz, J2 = 3.0 Hz), 7.30-7.38 (m, 3H), 7.46-7.51 (m, 1H), 7.98 (d, 1H, J = 1.1 Hz), 8.12 (s, 1H), 8.28 (dd, 1H, J1 = 7.6 Hz, J2 = 1.1 Hz), 10.80 (s, 1H). |

| Compound **99**: *N-*[1-(3,5-Difluoro-benzyl)-3-(2-nitro-phenylsulfanyl)-1*H*-indol-6-yl]-propionamide, structure **28** of Scheme **VII,** where R¹ = 3,5-difluorophenyl, R² = 2-nitrophenyl, R³=R⁴=H, Z¹= ethyl. Method b was used. | |
|---|---|
| 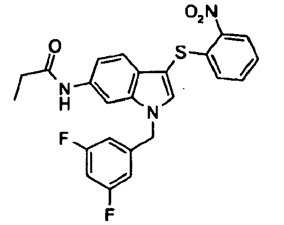 | ¹H-NMR (DMSO): δ 1.07 (t, 3H, J = 7.6 Hz), 2.31 (q, 2H, J = 7.6 Hz), 5.51 (s, 2H), 6.89 (dd, 1H, J1 = 7.6 Hz, J2 = 1.1 Hz), 6.93-6.97 (m, 1H), 7.16-7.23 (m, 3H), 7.27 (d, 1H, J = 8.4 Hz), 7.32-7.37 (m, 1H), 7.46-7.51 (m, 1H), 8.01 (d, 1H, J = 1.1 Hz), 8.02 (s, 1H), 8.27 (dd, 1H, J 1 = 7.6 Hz, J2 = 1.1 Hz), 9.92 (s, 1H). |

| Compound **100:** 2-Fluoro-*N*-[3-(2-nitro-phenylsulfanyl)-1-pyridin-2-ylmethyl-1*H*-indol-6-yl]-acetamide, structure **28** of Scheme **VII,** where R¹ = 2-pyridyl, R² = 2-nitrophenyl, R³=R⁴=H, Z¹= monofluoromethyl. Method a was used. | |
|---|---|
| 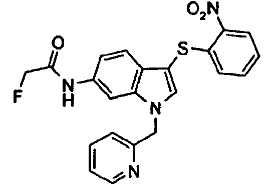 | ¹H-NMR (CDCl₃): δ 4.88 (s, 1H), 5.00 (s, 1H), 5.51 (s, 2H), 6.94-6.99 (m, 2H), 7.06 (dd, 1H, J1 = 7.6 Hz, J2 = 1.5 Hz), 7.18 (td, 1H, J1 = 8.4 Hz, J2 = 1.5 Hz), 7.22-7.29 (m, 2H), 7.46 (d, 1H, J = 8.4 Hz), 7.55 (s, 1H), 7.64 (td, 1H, J1 = 8.4 Hz, J2 = 1.5 Hz), 8.02 (s, 1H), 8.05 (d, 1H, J = 2.2 Hz), 8.27 (dd, 1H, J1 = 7.6 Hz, J2 = 1.5 Hz), 8.62 (d, 1H, J = 4.6 Hz). |

| Compound **101:** *N*-[3-(2-Nitro-phenylsulfanyl)-1-pyridin-2-ylmethyl-1*H*-indol-6-yl]-acetamide, structure **28** of Scheme **VII,** where R¹ = 2-pyridyl, R² = 2-nitrophenyl, R³=R⁴=H, Z¹= methyl. Method a was used. | |
|---|---|
| 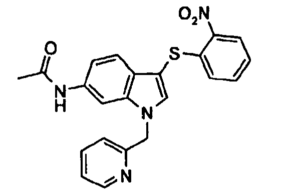 | ¹H-NMR (CDCl₃): δ 1.80 (s, 3H), 5.48 (s, 2H), 6.92-6.98 (m, 3H), 7.15-7.30 (m, 4H), 7.40 (d, 1H, J = 7.6 Hz), 7.51 (s, 1H), 7.63 (td, 1H, J1 = 7.6 Hz, J2 = 2.2 Hz), 8.04 (d, 1H, J = 1.5 Hz), 8.26 (dd, 1H, J1 = 7.6 Hz, J2 = 1.5 Hz), 8.61 (d, 1H, J = 4.6 Hz). |

| Compound **102:** *N*-[3-(2-Nitro-phenylsulfanyl)-1-pyridin-2-ylmethyl-1*H*-indol-6-yl]-propionamide, structure **28** of Scheme **VII,** where R¹ = 2 -pyridyl, R² = 2 -nitrophenyl, R³=R⁴=H, Z¹= ethyl). Method b was used. | |
|---|---|
| 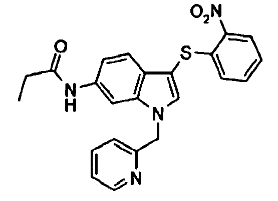 | ¹H-NMR (CDCl₃): δ 1.25 (t, 3H, J = 7.6 Hz), 2.41 (q, 2H, J = 7.6 Hz), 5.49 (s, 2H), 6.92-6.98 (m, 3H), 7.15-7.27 (m, 4H), 7.40 (d, 1H, J = 8.4 Hz), 7.51 (s, 1H), 7.63 (td, 1H, J1 = 7.6 Hz, J2 = 1.5 Hz), 8.12 (d, 1H, J = 1.5 Hz), 8.26 (dd, 1H, J1 = 7.6 Hz, J2 = 1.5 Hz), 8.61 (d, 1H, J = 4.6 Hz). |

| Compound **103:** *N*-[3-(2-Nitro-phenylsulfanyl)-1-pyridin-3-ylmethyl-1*H*-indol-6-yl]-acetamide, structure **28** of Scheme **VII,** where R¹ = 3-pyridyl, R² = 2-nitrophenyl, R³=R⁴=H, Z¹= methyl. Method a was used. | |
|---|---|
| 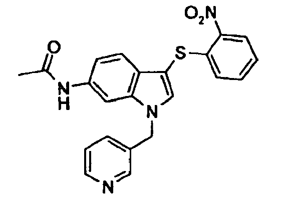 | ¹H NMR (CDCl₃) δ 2.20 (s, 3H), 5.40 (s, 2H), 6.86 (dd, 1H, J1 = 7.6 Hz, J2 = 2.2 Hz), 6.72 (dd, 1H, J1 = 7.6 Hz, J2 = 1.1 Hz), 7.16-7.20 (m, 1H), 7.24-7.31 (m, 3H), 7.40 (d, 1H, J = 8.4 Hz), 7.42 (s, 1H), 7.49-7.52 (m, 1H), 8.15 (d, 1H, J = 2.2 Hz), 8.26 (dd, 1H, J1 = 8.4 Hz, J2 = 2.2 Hz), 8.55 (d, 1H, J = 3.0 Hz), 8.58 (dd, 1H, J1 = 8.4 Hz, J2 = 2.2 Hz). |

| Compound **104**: 2-Fluoro-*N*-[3-(2-nitro-phenylsulfanyl)-1-pyridin-3-ylmethyl-1*H*-indol-6-yl]-acetamide, structure **28** of Scheme **VII,** where R¹ = 3-pyridyl, R² = 2-nitrophenyl, R³=R⁴=H, Z¹= monofluoromethyl. Method a was used. | |
|---|---|
| 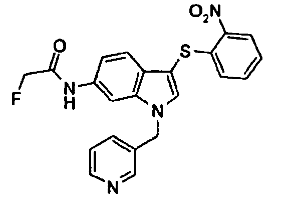 | ¹H NMR (CDCl₃) δ 4.88 (s, 1H), 5.00 (s, 1H), 5.42 (s, 2H), 6.92 (dd, 1H, J1 = 7.6 Hz, J2 = 1.1 Hz), 7.00 (dd, 1H, J1 = 8.4 Hz, J2 = 1.8 Hz), 7.17-7.21 (m, 1H), 7.25-7.32 (m, 2H), 7.44-7.47 (d, 1H, J = 7.6 Hz), 7.45 (s, 1H), 7.49-7.53 (m, 1H), 8.04 (d, 1H, J = 4.6 Hz), 8.15 (d, 1H, J = 1.8 Hz), 8.27 (dd, 1H, J1 = 8.4 Hz, J2 = 1.8 Hz), 8.56 (d, 1H, J = 3.0 Hz), 8.58 (dd, 1H, J1 = 4.6 Hz, J2 = 1.2 Hz). |

| Compound **105**: *N-*[3-(2-Nitro-phenylsulfanyl)-pyridin-3-ylmethyl-1*H-*indol-6-yl]-propionamide, structure **28** of Scheme **VII,** where R¹ = 3 -pyridyl, R² = 2 -nitrophenyl, R³=R⁴=H, Z¹= ethyl. Method b was used. | |
|---|---|
| 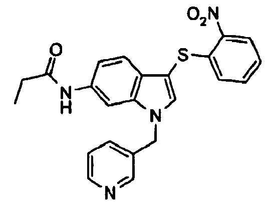 | ¹H NMR (CDCl₃) δ 1.26 (t, 3H, J = 7.6 Hz), 2.42 (q, 2H, J = 7.6 Hz), 5.39 (s, 2H), 8.68 (dd, 1H, 11 = 8.4 Hz, J2 = 1.8 Hz), 6.91 (dd, 1H, J1 = 8.4 Hz, J2 = 1.1 Hz), 7.15-7.20 (m, 1H), 7.24-7.30 (m, 3H), 7.38-7.41 (m, 2H), 7.48-7.52 (m, 1H), 8.23 (d, 1H, J = 1.8 Hz), 8.27 (dd, 1H, J1 = 8.4 Hz, J2 = 1.8 Hz), 8.55 (d, 1H, J = 3.0 Hz), 8.57 (dd, 1H, J1 = 4.6 Hz, J2 = 1.1 Hz). |

| Compound **106**: *N-*[1-(3,5-Difluoro-benzyl)-3-(2-nitro-phenylsulfanyl)-1*H-*indol-6-yl]-methanesulfonamide, structure **29** of Scheme **VII,** where R¹ = 3,5-difluorophenyl, R² = 2-nitrophenyl, R³=R⁴=H, Z²= Me. Method c was used. | |
|---|---|
| 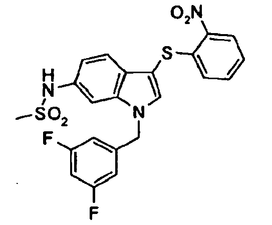 | ¹H-NMR (DMSO): δ 2.95 (s, 3H), 5.54 (s, 2H), 6.90 (dd, 1H, J1 = 7.6 Hz, J2 = 1.1 Hz), 6.98-7.04 (m, 3H), 7.20 (tt, 1H, J = 9.5 Hz, J2 = 3.0 Hz), 7.31-7.37 (m, 2H), 7.41 (d, 1H, J = 1.5 Hz), 7.47-7.52 (m, 1H), 8.08 (s, 1H), 8.27 (dd, 1H, J1 = 7.6 Hz, J2 = 1.1 Hz), 9.65 (s, 1H). |

| Compound **107:** Thiophene-3-sulfonic acid [1-(3,5-difluoro-benzyl)-3-(2-nitro-phenylsulfanyl)-1*H-*indol-6-yl]-amide, structure **29** of Scheme **VII,** where R¹ = 3,5-difluorophenyl, R² = 2-nitrophenyl, R³=R⁴=H, Z¹= 3-thiophene. Method c was used. | |
|---|---|
| 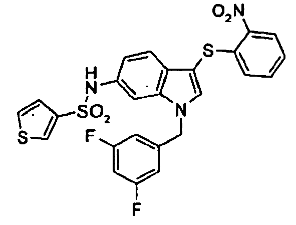 | ¹H-NMR (DMSO): δ 5.50 (s, 2H), 6.85 (m, 2H), 6.92-6.97 (m, 2H), 7.13 (dd, 1H, J1 = 5.0, J2 = 1.1 Hz), 7.19-7.26 (m, 2H), 7.31 (d, 1H, J = 2.2 Hz), 7.32-7.36 (m, 1H), 7.45-7.50 (m, 1H), 7.61 (dd, 1H, J1 = 5.0 Hz, J2 = 3.0 Hz), 7.97 (dd, 1H, J1= 3.0 Hz, J2 = 1.1 Hz), 8.05 (s, 1H), 8.26 (dd, 1H, J1= 7.6 Hz, J2 = 1.1 Hz), 10.20 (s, 1H). |

| Compound **108:** 1-Methyl-1*H-*imidazole-**4**-sulfonic acid [1-(3,5-difluoro-benzyl)-3- (2-nitro-phenylsulfanyl)-1*H-*indol-6-yl]-amide, structure **29** of Scheme **VII**, where R¹ = 3,5-difluorophenyl, R² = 2-nitrophenyl, R³=R⁴=H, Z²= 4-(1-methyl)-imidazole: Method c was used. | |
|---|---|
| 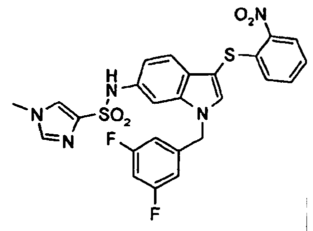 | ¹H-NMR (DMSO): δ 3.60 (s, 3H), 5.46 (s, 2H), 6.86 (dd, 1H, J 1 = 7.6 Hz, J2 = 0.7 Hz), 6.92 (dd, 1H, J 1 = 7.6 Hz, J2 = 1.5 Hz), 6.96-7.01 (m, 2H), 7.19 (d, 1H, J = 7.6 Hz), 7.23 (tt, 1H, J1 = 9.5 Hz, J2 = 3.0 Hz), 7.31-7.36 (m, 2H), 7.45-7.49 (m, 1H), 7.66-7.69 (m, 2H), 8.02 (s, 1H), 8.26 (dd, 1H, J1 = 7.6 Hz, J2 = 0.7 Hz), 10.20 (s, 1H). |

### Example 20

According to **general method 3** the following compounds were prepared:

| Compound **94**:6-Methoxy-3-(2-nitro-phenylsulfanyl)-1-(3,4,5-trifluoro-benzyl)-1*H* indole, structure **3** of scheme **I**, where R¹ = 3,4,5-difluorophenyl, R²= 2 -nitrophenyl, R³ = R⁴ = H, R⁵ = OMe. Cs₂CO₃ and NMP were used. | |
|---|---|
| 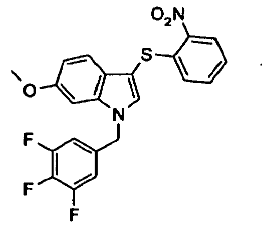 | ¹H-NMR (CDCl₃): δ 3.83 (s, 3H, OCH₃), 5.28 (s, 2H, NCH₂Ar), 6.72 (d, 1H, J = 2.2 Hz), 6.75 (d, 1H, J = 7.6 Hz), 6.77 (d, 1H, J = 7.6 Hz), 6.86 (dd, 1H, J 1 = 8.4 Hz, J2 = 2.2 Hz), 6.97 (dd, 1H, J1 = 8.4 Hz, J2 = 1.1 Hz), 7.19 (td, 1H, J = 7.6 Hz, J2 = 1.1 Hz), 7.28 (td, 1H, J1 = 7.6 Hz, J2 = 1.1 Hz), 7.33 (s, 1H), 7.40 (d, 1H, J = 8.4 Hz), 8.27 (dd, 1H, J 1 = 8.4 Hz, J2 = 2.2 Hz). |

| Compound **95**: 6-Methoxy-3-(2-nitro-phenylsulfanyl)-1-(2,3,5-trifluoro-benzyl)-1*H*-indole, structure **3** of scheme **I**, where R¹ = 2,3,5-difluorophenyl, R² = 2-nitrophenyl, R³ = R⁴ = H, R⁵ = OMe. Cs₂CO₃ and NMP were used. | |
|---|---|
| 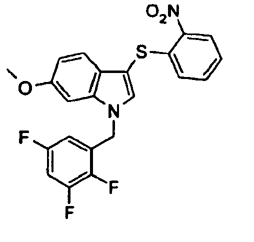 | ¹H-NMR (CDCl₃): δ 3.85 (s, 3H, OCH₃), 5.39 (s, 2H, NCH₂Ar), 6.42 (m, 1H), 6.82 (d, 1H, J = 2.7 Hz), 6.86 (dd, 1H, J1 = 7.6 Hz, J2 = 2.2 Hz), 6.90 (m, 1H), 6.96 (dd, 1H, J 1 = 7.6 Hz, J2 = 2.2 Hz), 7.18 (td, 1H, J1 = 7.6 Hz, J2 = 1.5 Hz), 7.27 (td, 1H, J = 7.6 Hz, J2 = 1.5 Hz), 7.37 (s, 1H), 7.3 9 (d, 1H, J = 7.6 Hz), 8.27 (dd, 1H, J1 = 7.6 Hz, J2 = 1.5 Hz). |

| Compound **96**: 1-(6-Chloro-pyridin-3-ylmethyl)-6-methoxy-3-(2-nitro-phenylsulfanyl)-1*H-*indole, structure **3** of Scheme **I**, where R¹ =4-chloro-3-pyridyl, R² = 2-nitrophenyl, R³=R⁴=H, R⁵=OMe). Cs₂CO₃ and NMP were used. | |
|---|---|
| 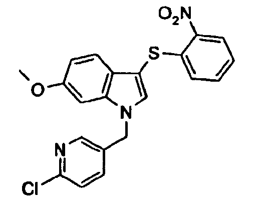 | ¹H-NMR (CDCl₃): δ 3.82 (s, 3H, OCH₃), 5.37 (s, 2H, NCH₂Ar), 6.76 (d, 1H, J = 2.2 Hz), 6.84 (dd, 1H, J1 = 7.6 Hz, J2 = 2.2 Hz), 6.96 (dd, 1H, J1 = 7.6 Hz, J2 = 1.1 Hz), 7.18 (td, 1H, J1 = 8.4 Hz, J2 = 1.1 Hz), 7.27 (td, 1H, J1 = 8.4 Hz, J2 = 1.1 Hz), 7.3 0-7.41 (m. 4H), 8.27 (dd, 1H, J1 = 8.4 Hz, J2 = 1.1 Hz), 8.34 (d, 1H, J = 3.0 Hz). |

| Compound **115:** 6-Methoxy-3-(2-nitro-phenylsulfanyl)-1-pyrimidin-5-ylmethyl-1*H-*indole, structure **3** of Scheme **I,** where R¹ = 3,5-pyrimidyl, R² = 2-nitrophenyl, R³=R⁴=H, R⁵=OMe. NaH and DMF were used. The synthesis of methanesulfonic acid pyrimidin-5-ylmethyl ester is described in example **23a).** | |
|---|---|
| 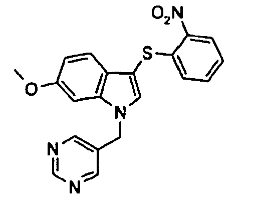 | ¹H NMR (CDCl₃) δ 3.83 (s, 3H), 5.38 (s, 2H), 6.77 (d, 1H, J = 3.0 Hz), 6.86 (dd, 1H, J1 = 8.4 Hz, J2 = 3.0 Hz), 6.96 (dd, 1H, J1 = 8.4 Hz, J2 = 1.1 Hz), 7.17-7.21 (m, 1H), 7.26-7.32 (m, 1H), 7.37 (s, 1H), 7.39 (d, 1H, J = 8.4 Hz), 8.27 (dd, 1H, J1 = 8.4 Hz, J2 = 1.1 Hz), 8.61 (s, 2H), 9.20 (s, 1H). |

| Compound **116**: 6-Methoxy-3-(2-nitro-phenylsulfanyl)-1-pyrimidin-4-ylmethyl-1*H*-indole, structure **3** of Scheme **I,** where R¹ = 2,4-pyrimidyl, R² = 2-nitrophenyl, R³=R⁴=H, R⁵=OMe. Cs₂CO₃ and NMP were used. | |
|---|---|
| 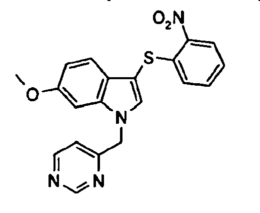 | ¹H NMR (CDCl₃) δ 3.80 (s, 3H), 5.44 (s, 2H), 6.74 (d, 1H, J = 2.2 Hz), 6.80 (dd, 1H, J 1 = 4.6 Hz, J2 = 1.1 Hz), 6.86 (dd, 1H, J1 = 8.4 Hz, J2 = 3.0 Hz), 7.00 (dd, 1H, J1 = 8.4 Hz, J2 = 1.1 Hz), 7.17-7.21 (m, 1H), 7.26-7.33 (m, 1H), 7.40 (d, 1H, J = 8.4 Hz), 7.42 (s, 1H), 8.27 (dd, 1H, J1 = 8.4 Hz, J2 = 1.1 Hz), 8.66 (d, 1H, J = 4.6 Hz), 9.24 (d, 1H, J = 1.1 Hz). |

| Compound 117: 6-Methoxy-3-(2-nitro-phenylsulfanyl)-1-pyrazin-2-ylmethyl-1*H*-indole, structure **3** of Scheme **I,** where R¹ = 2,5-pyrazyl, R² = 2-nitrophenyl, R³=R4=H_{,} R⁵=OMe. Cs₂CO₃ and NMP were used. | |
|---|---|
| 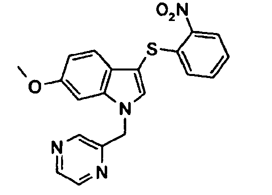 | ¹H NMR (CDCl₃) δ 3.83 (s, 3H), 5.49 (s, 2H), 6.82-6.86 (m, 2H), 6.99 (dd, 1H, J1 = 8.4 Hz, J2 = 1.1 Hz), 7.16-7.20 (m, 1H), 7.25-7.29 (m, 1H), 7.38 (d, 1H, J = 8.4 Hz), 7.46 (s, 1H), 8.26 (dd, 1H, J1 = 8.4 Hz, J2 = 1.1 Hz), 8.35 (d, 1H, J = 1.1 Hz), 8.55 (d, 1H, J = 3.0 Hz), 8.59-8.61 (m, 1H). |

### Compound 61: 1-(3,5-Difluoro-benzyl)-3-(2-nitro-phenylsulfanyl)-1H-indol-6-ol, structure 25 of Scheme VI, where R¹ = 3,5-difluorophenyl, R² =2-nitrophenyl, R³=R⁴=H, R⁵=OH.

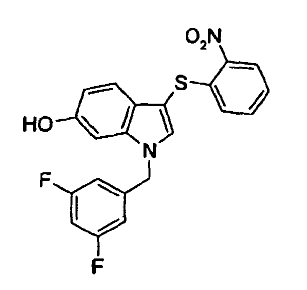

To a solution of compound **63** (240 mg, 0.56 mmol) in CH₂Cl₂ (10 mL) was added at 0 °C boron trifluoride-methyl sulfide complex (2.37 mL, 22.5 mmol). After stirring for 2 hrs at 0 °C the reaction mixture was allowed to warm to room temperature and stirring was continued for another 4 hrs. The reaction mixture was poored into ice water (20 mL) and after addition of ethyl acetate (10 mL) the layers were separated. The organic layer was washed with saturated aqueous NaHCO₃ (20 mL) and brine (20 mL) and dried over Na₂SO_{4.} Concentration gave the crude product which was purified using flash chromatography (heptane/ethylactate 3:2) to afford compound **61** as a yellow oil (87 mg, 0.21 mmol, 38%).
¹H NMR (CDCl₃) δ 4.75 (2, 1H), 5.29 (s, 2H), 4.66 (m, 1H), 6.72-6.79 (m, 3H), 6.98 (dd, 1H, J1 = 8.4 Hz, J2 = 1.5 Hz), 7.17-7.21 (m, 1H), 7.27-7.31 (m, 1H), 7.35-7.37 (m, 3H), 8.27 (dd, 1H, J1 = 8.4 Hz, J2 = 1.5 Hz).

### Example 22

### Compound 109: 3-(2-Nitro-phenylsulfanyl)-1-pyridin-2-ylmethyl-1H-indole-6-carboxylic acid dimethylamide, structure 15 of Scheme IV, where R¹ 2=pyridyl, R²= 2-nitrophenyl, R³=R⁴=H, R⁸=R⁹=Me.

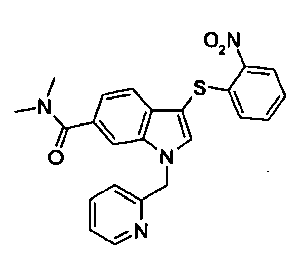

### a) 1-Pyridin-2-ylmethyl-1H-indole-6-carboxylic acid methyl ester.

Under a nitrogen atmosphere: to a solution of 1*H-*indole-6-carboxylic acid methyl ester (for synthesis see example 2a), 2.47 g, 14.1 mmol) in NMP (140 mL) was added Cs₂CO₃ (10.1 mg, 31 mmol) at 0 °C. After stirring for 15 min 2-picolyl chloride hydrochloride (2.77 g, 16.9 mmol) was added and the mixture was stirred at room temperature for 18 h. Ethyl acetate (200 mL) was added and the mixture was washed with saturated aqueous ammonium chloride (3x150 mL) and brine (200 mL). The organic phase was dried (Na₂SO₄) and concentrated. The crude product was purified using column chromatography (ethyl acetate/heptane 1:3) to give the title compound as a colourless oil, which slowly crystallised on standing (2.72 g, 10.2 mmol, 72%).

### b) 3-(2-Nitro-phenylsulfanyl)-1-pyridin-2-ylmethyl-1H-indole-6-carboxylic acid methyl ester, structure 13 of Scheme IV, where R¹ = 2-pyridyl, R² = 2-nitrophenyl, R³=R⁴=H.

To a solution of 1-pyridin-2-ylmethyl-1H indole-6-carboxylic acid methyl ester (2.72 g, 10.2 mmol) in dichloromethane (75 mL) was added at room temperature a solution of 2-nitrobenzenesulfenyl chloride (1.93 g, 10.2 mmol) in dichloromethane (75 mL). The mixture was stirred at room temperature for 24 hrs. The reaction mixture was concentrated and the crude product was purified using column chromatography (ethyl acetate/heptane 1:5 to 1:2) to give the title compound as a yellow solid (3.21 g, 7.65 mmol, 75%).

### c) 3 -(2-Nitro-phenylsulfanyl)-1-pyridin-2-ylmethyl-1H-indole-6-carboxylic acid, structure 14 of Scheme IV, where R¹ = 2 -pyridyl, R² = 2-nitrophenyl, R³=R⁴=H.

To a suspension of 3-(2-nitro-phenylsulfanyl)-1-pyridin-2-ylmethyl-1*H-*indole-6-carboxylic acid methyl ester (3.0 g, 6.58 mmol) in dioxane (65 mL) was added a solution of LiOH.H₂O (1.66 g, 39.6 mmol) in water (65 mL). The reaction mixture was stirred overnight at 60°C. The mixture was then acidified to pH 6 by addition of 15% aqueous HCl and the mixture was extracted with ethyl acetate (2 x 100 mL). The combined organic layers were dried over Na₂SO₄ and concentrated to give a yellow powder. The product was recrystallised from heptane/ethyl acetate to give the title compound as yellow/orange crystals (2.47 g, 6.09 mmol, 93%)

### General method 7: amidation of 6-carboxyl indoles of structure 14 to give 6-carboxamideindoles of structure 15 (Scheme IV).

Under nitrogen atmosphere: to a solution of 3-(2-nitro-phenylsulfanyl)-1-pyridin-2-ylmethyl-1*H-*indole-6-carboxylic acid (700 mg, 1.73mmol) in dry CH₂Cl₂ (70 mL) was added DIPEA (0.9 mL, 5.2 mmol), TBTU (840 mg, 2.6 mmol) and dimethylamine hydrochloride (420 mg, 5.1 mmol). The reaction mixture was stirred overnight at room temperature and then poured into saturated aqueous NaHCO₃ (100 mL). The organic layer was washed with brine (50 mL), dried (Na₂SO₄) and concentrated. The crude product was purified by column chromatography (ethyl acetate/MeOH 95:5) to afford compound **109** (540 mg, 1.25 mmol, 72%, yellow solid).
¹H-NMR (CDCl₃): δ 2.97 (s, 3H), 3.11 (s, 3H), 5.53 (s, 2H), 6.90 (dd, 1H, J1 = 7.6 Hz, J2 = 1.5 Hz), 6.95 (d, 1H, J = 7.6 Hz), 7.17-7.28 (m, 4H), 7.51 (d, 1H, J = 7.6 Hz), 7.54 (s, 1H), 7.61-7.66 (m, 2H), 8.28 (dd, 1H, J 1 = 8.4 Hz, J2 = 1.5 Hz), 8.61 (d, 1H, J = 4.6 Hz).

The following compounds were prepared using general procedure 7:

| Compound **110:** 3-(2-Nitro-phenylsulfanyl)-1-pyridin-2-ylmethyl-1*H-*indole-6-carboxylic acid methylamide, structure **15** of Scheme **IV,** where R¹ = 2-pyridyl, R² = 2-nitrophenyl, R³=R⁴=H, R⁸=H, R⁹=Me. | |
|---|---|
| 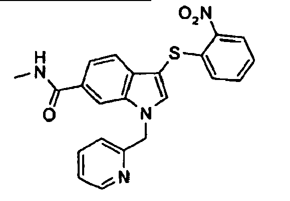 | ¹H-NMR (DMSO): δ 2.78 (d, 3H, J = 4.6 Hz), 5.70 (s, 2H), 6.88 (dd, 1H, J1 = 8.4 Hz, J2 = 1.5 Hz), 7.19 (d, 1H, J = 7.6 Hz), 7.30-7.37 (m, 2H), 7.41 (d, 1H, J = 8.4 Hz), 7.48-7.53 (m, 1H), 7.62 (dd, 1H, J1 = 7.6 Hz, J2 = 1.5 Hz), 7.79 (td, 1H, J1 = 7.6 Hz, J2 = 1.5 Hz), 8.13 (s, I H), 8.20 (s, 1H), 8.28 (dd, 1H, J1 = 8.4 Hz, J2 = 1.5 Hz), 8.38 (q, 1H, J = 4.6 Hz), 8.55 (s, 1H, J = 4.6 Hz). |

| Compound **111**: 3-(2-Nitro-phenylsulfanyl)-1-pyridin-2-ylmethyl-1*H-*indole-6-carboxylic acid amide, structure **15** of Scheme **IV,** where R¹ = 2-pyridyl, R² = 2-nitrophenyl, R³=R⁴=H, R⁸=R⁹=H. | |
|---|---|
| 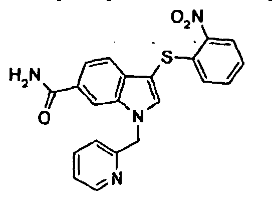 | ¹H-NMR (DMSO): δ 5.70 (s, 2H), 6.88 (dd, 1H, J1= 8.4 Hz, J2 = 1.5 Hz), 7.21 (d, 1H, J = 7.6 Hz), 7.28-7.37 (m, 3H), 7.39 (d, 1H, J = 7.6 Hz), 7.48-7.53 (m, 1H), 7.66 (dd, 1H, J 1 = 8.4 Hz, J2 = 1.5 Hz), 7.79 (td, 1H, J = 7.6 Hz, J2 = 1.5 Hz), 7.93 (s, 1H), 8.17 (s, 1H), 8.20 (s, 1H), 8.26 (dd, 1H, J1 = 7.6 Hz, J2 = 1.5 Hz), 8.55 (d, 1H, J = 4.6 Hz). |

| Compound **112**: 3-(2-Nitro-phenylsulfanyl)-1-pyridin-3-ylmethyl-1*H-*indole-6-carboxylic acid methylamide, structure **15** of Scheme **IV,** where R¹ = 3-pyridyl, R² = 2-nitrophenyl, R³=R⁴=H, R⁸=H, R⁹=Me. 3-Picolyl chloride hydrochloride was used instead of 2-picolyl chloride hydrochloride. | |
|---|---|
| 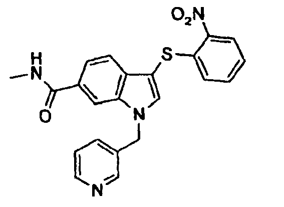 | ¹H-NMR (DMSO): δ 2.80 (d, 3H, J =4.6 Hz), 5.65 (s, 2H), 6.84 (dd, 1H, J1 = 7.6 Hz, J2 = 1.1 Hz), 7.33 -7.42 (m, 3H), 7.46-7.51 (m, 1H), 7.62 (dd, 1H, J1 = 8.4 Hz, J2 = 1.1 Hz), 7.68 (dt, 1H, J1 = 7.6 Hz, J2 = 2.2 Hz), 8.19 (s, 1H), 8.26 (s, 1H), 8.28 (dd, 1H, J1 = 7.6 Hz, J2 = 1.1 Hz), 8.41 (q, 1H, J = 4.6 Hz), 8.51 (dd, 1H, J1 = 4.6 Hz, J2 = 1.1 Hz), 8.64 (d, 1H, J = 3.0 Hz). |

| Compound **113**:3-(2-Nitro-phenylsulfanyl)-1-pyridin-3-ylmethyl-1*H-*indole-6-carboxylic acid ethylamide, structure **15** of Scheme **IV,** where R¹ = 3-pyridyl, R² =2-nitrophenyl, R³=R⁴=H, R⁸=H, R⁹=Et. 3-Picolyl chloride hydrochloride was used instead of 2-picolyl chloride hydrochloride. | |
|---|---|
| 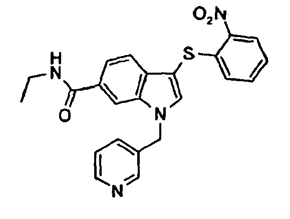 | ¹H-NMR (DMSO): δ 1.13 (t, 3H, J = 7.6 Hz), 3.30 (q, 2H, J = 7.6 Hz), 5.66 (s, 2H), 6.83 (dd, 1H, J 1 = 7.6 Hz, J2 = 1.1 Hz), 7.33-7.41 (m, 3H), 7.46-7.51 (m, 1H), 7.64 (dd, 1H, J 1= 7.6 Hz, J2 = 1.1 Hz), 7.67 (dt, 1H, J1 = 7.6 Hz, J2 = 2.2 Hz), 8.19 (s, 1H), 8.26 (s, 1H), 8.27 (dd, 1H, J1 = 7.6 Hz, J2 = 1.1 Hz), 8.45 (t, 1H, J = 5.7 Hz), 8.52 (dd, 1H, J 1 = 4.6 Hz, J2 = 1.5 Hz), 8.64 (d, 1H, J = 3.0 Hz). |

| Compound **114**: 3-(2-Nitro-phenylsulfanyl)-1-pyridin-3-ylmethyl-1*H-*indole-6-carboxylic acid cyclopropylamide, structure **15** of Scheme **IV**, where R¹ = 3 -pyridyl, R² = 2-nitrophenyl, R³=R⁴=H, R⁸=H, R⁹=cyclopropyl. 3-Picolyl chloride hydrochloride was used instead of 2-picolyl chloride hydrochloride. | |
|---|---|
| 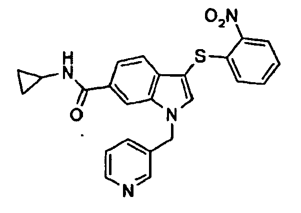 | ¹H-NMR (DMSO): δ 0.54-0.58 (m, 2H), 0.67-0.73 (m, 2H), 2.80-2.87 (m, 1H), 5.66 (s, 2H), 6.82 (d, 1H, J = 7.6 Hz), 7.33-7.41 (m, 3H), 7.46-7.50 (m, 1H), 7.61 (dd, 1H, J1 = 7.6 Hz, J2 = 1.1 Hz), 7.67 (dt, 1H, J1 = 7.6 Hz, J2 = 1.5 Hz), 8.17 (s, 1H), 8.25 (s, 1H), 8.28 (dd, 1H, J1 = 7.6 Hz, J2 = 1.1 Hz), 8.42 (t, 1H, J = 4.6 Hz), 8.52 (dd, 1H, J1 = 4.6 Hz, J2 = 1.5 Hz), 8.63 (d, 1H, J = 3.0 Hz). |

### Example 23

### Compound 118: N-[3-(2-Nitro-phenylsulfanyl)-1-pyrimidin-5-ylmethyl-1H-indol-6-yl]-formamide, structure 28 of Scheme VII, where R¹ = 3,5-pyrimidyl, R² = 2-nitrophenyl, R³=R⁴=H, Z = H

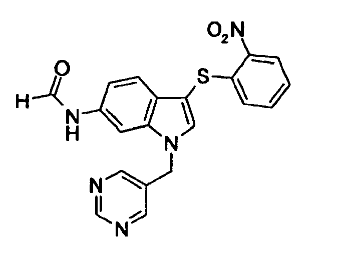

### a) 6-Nitro-1-pyrimidin-5-ylmethyl-1H-indole

5-(Hydroxymethyl)pyrimidine (16.2 g, 147 mmol) was dissolved in 500 mL of CH₂Cl₂ and cooled to -40°C. MsCl (16.0 g, 140 mmol) was added at -40 °C. The mixture was stirred for 1 hour allowing to reach -20°C. The solution was used as such.
6-Nitroindole-1*H*-indole (35.6 g, 220 mmol) was dissolved in 750 mL of DMF and NaH (50%, 12 g, 250 mmol) was added in portions and the mixture was stirred for I hour. The mixture was cooled to -40°C and the solution of the mesylate was added dropwise. The mixture was allowed to reach room temperature overnight. The mixture was quenched with water and extracted with ethyl acetate (2x). The combined organic layers were washed with water (2x) and brine and dried over Na₂SO₄. After concentration in *vacuo* the material was purified by means of column chromatography (silica, heptane/ethyl acetate (1:1)→(1:4)) affording compound the title compound as a yellow solid (14 g, 55 mmol, 39%).

### b) 1-Pyrimidin-5-ylmethyl-1H-indol-6-ylamine.

6-Nitro-1-pyrirmidin-5-ylmethyl-1*H-*indole (4.1 g, 16.1 mmol) was dissolved in 100 mL of THF and 100 mL of isopropanol. Pd/C (2 g) was added and the mixture was stirred under an H₂-atmosphere (balloon) for 2 hours. The mixture was filtered over Celite and washed with ethyl acetate affording the title compound after concentration (3.5 g, 15.6 mmol, 97 %).

### c) 1-Pyrimidin-5-ylmethyl-1H-indol-6-yl)-carbamic acid tert-butyl ester

1-Pyrimidin-5-ylmethyl-1*H*-indol-6-ylamine (5.5 g, 24.6 mmol) was dissolved in 100 mL of NMP and triethylamine (3.5 g, 35 mmol) and di-tert-butyl dicarbonate (7.0 g, 32 mmol) were added. The mixture was stirred overnight, quenched with NaHCO₃-sat and extracted with toluene (3x). The combined organic layers were washed with water (4x) and brine and dried over Na₂SO₄. Concentration of the layers afforded the title compound (3.1 g, 39%).

### d) [3-(2-Nitro-phenylsulfanyl)-1-pyrimidin-5-ylmethyl-1H-indol-6-yl]-carbamic acid tert-butyl ester

1-Pyrimidin-5-ylmethyl-1*H-*indol-6-yl)-carbamic acid tert-butyl ester (3.1 g, 9.6 mmol) was dissolved in 150 mL of CH₂Cl₂ and 2-nitrobenzene sulfenylchloride (1.86 g, 9.8 mmol) was added. The mixture was stirred for 18 hours and quenched with NaHCO₃-sat and extracted with ethyl acetate (2x). The combined organic layers were washed with brine and dried over Na₂SO₄. The product was purified by means of column chromatography (silica, heptane / ethyl acetate (1:1) which gave the title compound as a yellow solid (3.8 g, 83 %).

### e) N-[3-(2-Nitro-phenylsulfanyl)-1-pyrimidin-5-ylmethy]-1H-indol-6-yl]-formamide

[3-(2-Nitro-phenylsulfanyl)-1-pyrimidin-5-ylmethyl-1*H-*indol-6-yl]-carbamic acid tert-butyl ester (3.8 g, 8.0 mmol) was dissolved in 150 mL of formic acid and stirred for 4 hours. The solution was concentrated *in vacuo.* NaHCO₃-sat was added and the mixture was extracted with CH₂Cl₂ (2x) The combined organic layers were washed with brine and dried over Na₂SO₄. The solution was allowed to stand overnight. The formed solid was filtered affording a mixture of compound **118** and structure **23** of Scheme **VII,** where R¹ = 3,5-pyrimidyl, R²=2-nitrophenyl, R³=R⁴=H (2.4 g).
Compound **118:** ¹H NMR (CDCl₃ + drop MeOD) δ 5.47 (s, 2H), 6.91 (dd, 1H, J1 = 8.4 Hz, J2 = 1.1 Hz), 6.99 (dd, 1H, J1 = 8.4 Hz, J2 = 1.9 Hz), 7.19-7.23 (m, 1H), 7.27-7.31 (m, 1H), 7.33 (s, 1H), 7.42 (d, 1H, J = 8.4 Hz), 7.49 (s, 1H), 8.22 (d, 1H, J = 1.9 Hz), 8.27 (dd, 1H, J1 = 8.4 Hz, J2 = 1.1 Hz), 8.66 (s, 2H), 9.16 (s, 1H).

### Example 24

### Compound 119: 3-(2-Nitro-phenylsulfanyl)-1-pyridin-2-ylmethyl-1H-indole-6-carbonitrile structure 3 of scheme I, where R¹ =2-pyridyl, R² = 2-nitrophenyl_{.} R³ = R⁴ = H, R⁵ = CN.

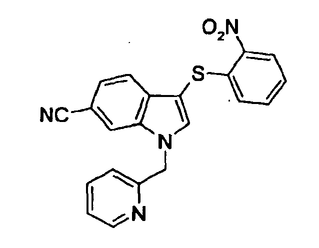

a) To a solution of 1*H-*indole-6-carbonitrile (1.00 g, 7.04 mmol) in diethyl ether (50 mL) was added at rt 2-nitrobenzenesulfenyl chloride (1.34 g, 7.04 mmol) and the reaction mixture was stirred for 18 hrs. The mixture was concentrated and the crude product was purified using column chromatography (ethyl acetate/heptane 1:4 → 4:1) to give 3-(2-Nitro-phenylsulfanyl)-1*H-*indole-6-carbonitrile (1.62 g, 5.49 mmol, 78%).
b) To a solution of 3-(2-nitro-phenylsulfanyl)-1*H-*indole-6-carbonitrile (200 mg, 0.68 mmol) in DMF (5 mL) was added at 0 °C Cs₂CO₃ (442 mg, 1.36 mmol) and the mixture was stirred for 20 min. Subsequently 2-picolylchloride hydrochloride (112 mg, 0.68 mmol) was added and the mixture was stirred for 18 hrs at room temperature. Aqueous saturated NaHCO₃ was added and the mixture was extracted with ethyl acetate (2x). The combined organic layers were washed with brine, dried (Na₂SO₄) and concentrated. The crude product was purified using column chromatography to afford compound **119** (223 mg, 0.58 mmol, 85%).
   ¹H NMR (CDCl₃) δ 5.54 (s, 2H), 6.86 (dd, 1H, J1 = 8.4 Hz, J2 = 1.1 Hz), 7.04 (d, 1H, J = 8.4 Hz), 7.19-7.23 (m, 1H), 7.26-7.31 (m, 2H), 7.40 (dd, 1H, J1 = 8.4 Hz, J2 = 1.1 Hz), 7.59 (d, 1H, J = 8.4 Hz), 7.70 (td, 1H, J1 = 8.4 Hz, J2 = 1.9 Hz), 7.76 (s, 1H), 7.78 (s, 1H), 8.29 (dd, 1H, J1 = 8.4 Hz, J2 = 1.1 Hz), 8.63 (d, 1H, J = 3.0 Hz).

According to **general method 3** the following compounds were prepared:

| Compound **121**: 3-(2-Nitro-phenylsulfanyl)-1-pyridin-3-ylmethyl- 1*H-*indole-6-carbonitrile, structure **3** of scheme **I**, where R¹=3 -pyridyl, R² = 2-nitrophenyl, R³ = R⁴ = H, R⁵ = CN. Cs₂CO₃ and DMF were used. | |
|---|---|
| 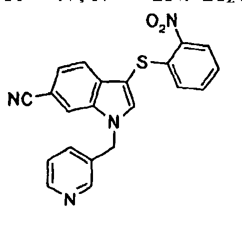 | ¹NMR (CDCl₃) δ 5.46 (s, 2H), 6.82 (dd, 1H, J1 = 7.6 Hz, J2 = 1.1 Hz), 7.21-7.25 (m, 1H), 7.27-7.31 (m, 1H), 7.34 (dd, 1H, J1 = 8.4 Hz, J2 = 4.6 Hz), 7.43 (dd, 1H, J1 =7.6Hz,J2=1.1 Hz),7.49(dt, 1H, J1 =8.4Hz, J2= 1.9 Hz), 7.61 (d, 1H, J = 8.4 Hz), 7.66 (s, 1H), 7.71 (s, 1H), 8.29 (dd, 1H, J1 = 7.6 Hz, J2 = 1.1 Hz), 8.55 (d, 1H, J = 3.0 Hz), 8.64 (dd, 1H, J1 = 8.4 Hz, J2 = 1.5 Hz). |

| Compound **122**: 3-(2-Nitro-phenylsulfanyl)-1-pyridin-4-ylmethyl-1*H-*indole-6-carbonitrile, structure **3** of scheme **I**, where R¹ = 4 -pyridyl, R² = 2-nitrophenyl, R³ = R⁴=H.R⁵=CN. | |
|---|---|
| 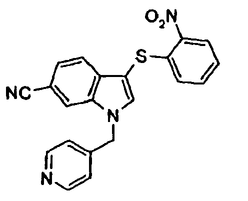 | ¹H NMR (CDCl₃) δ 5.46 (s, 2H), 6.84 (dd, 1H, J1 = 8.4 Hz, J2 = 1.1 Hz), 7.04 (d, 2H, J = 6.1 Hz), 7.22-7.26 (m, 1H), 7.28-7.32 (m, 1H), 7.44 (dd, 1H, J1 = 7.6 Hz, J2 = 1.1 Hz), 7.62 (d, 1H, J= 8.4 Hz), 7.64 (s, 1H), 7.68 (s, 1H), 8.30 (dd, 1H, J1 = 8.4 Hz, J2 = 1.1 Hz), 8.63-8.65 (m, 2H). Cs₂CO₃ and DMF were used. |

| Compound **123**: 3-(2-Nitro-phenylsulfanyl)-1-pyrimidin-5-ylmethyl-1*H-*indole-6-carbonitrile, structure **3** of scheme **I**, where R¹= 3,5-pyrimidyl, R² = 2-nitrophenyl, R³ = R⁴ = H, R⁵ = CN. NaH and DMF were used. | |
|---|---|
| 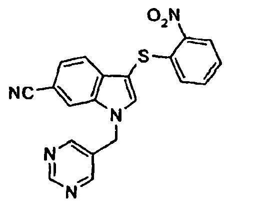 | ¹H NMR (CDCl₃) δ 5.48 (s, 2H), 6.81 (dd, 1H, J1 = 7.6 Hz, J2 = 1.1 Hz), 7.22-7.32 (m, 2H), 7.46 (dd, 1H, J1 = 7.6 Hz, J2 = 1.1 Hz), 7.63 (d, 1H, J = 8.4 Hz), 7.68 (s, 1H), 7.70 (s, 1H), 8.30 (dd, 1H, J1 = 8.4 Hz, J2 = 1.1 Hz), 8.63 (s, 2H), 9.25 (s, 1H). |

| Compound **124:** 3-(2-Nitro-phenylsulfanyl)-1-pyrimidin-4-ylmethyl-1*H-*indole-6-carbonitrile, structure **3** of scheme **I,** where R¹ = 2,4-pyrimidyl, R²= 2-nitrophenyl, R³ =R⁴= H, R⁵ = CN. Cs₂CO₃ and DMF were used. | |
|---|---|
| 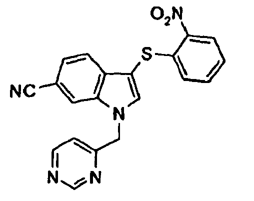 | ¹H NMR (CDCl₃) δ 5.52 (s, 2H), 6.86 (dd, 1H, J1 = 7.6 Hz, J2 = 1.1 Hz), 6.97 (dd, 1H, J1 = 4.6 Hz, J2 = 1.1 Hz), 7.22-7.32 (m, 2H), 7.44 (dd, 1H, J 1= 8.4 Hz, J2 = 1.1 Hz), 7.62 (d, 1H, J = 8.4 Hz), 7.70 (s, 1H), 7.75 (s, 1H), 8.30 (dd, 1H, J1 = 7.6 Hz, J2 = 1.1 Hz), 8.74 (d, 1H, J = 4.6 Hz), 9.24 (d, 1H, J = 1.1 Hz). |

| Compound **125:** 1-(3-Cyano-benzyl)-3-(2-nitro-phenylsulfanyl)-1*H-*indole-6-carbonitrile, structure **3** of scheme **I,** where R¹ = 3 -cyanophenyl, R²= 2-nitrophenyl, R³ = R⁴ = H, R⁵ = CN. Cs₂CO₃ and DMF were used. | |
|---|---|
| 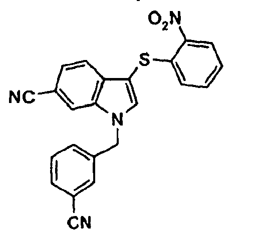 | ¹H NMR (CDCl₃) δ 5.48 (s, 2H), 6.83 (dd, 1H, J1 = 8.4 Hz, J2 = 1.1 Hz), 7.21-7.26 (m, 1H), 7.30-7.35 (m, 1H), 7.38-7.42 (m, 3H), 7.45 (d, 1H, J = 8.4 Hz), 7.53 (d, 1H, J = 7.6 Hz), 7.64 (d, 1H, J = 6.1 Hz), 7.65 (d, 1H, J = 3.7 Hz), 7.66-7.69 (m, 1H), 8.30 (dd, 1H, J1 = 8.4 Hz, J2 = 1.1 Hz). |

### Example 25

### Compound 120: 3-(2-Nitro-phenylsulfanyl)-1-pyridin-2-ylmethyl-1H-indole-6-carbonitrile monohydrochloride

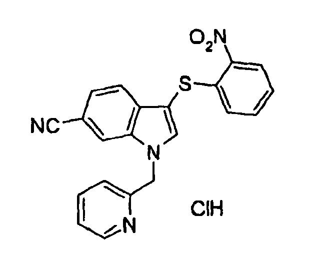

Concentrated hydrochloric acid (0.0225 ml) was added to a solution of compound **119** (0.093 g) in dioxane. The mixture was stirred for 20 min. and then concentrated under reduced pressure, to give compound **120** (0.096 g).
¹H NMR (MeOD) δ 6.01 (s, 2H), 6.98 (dd, 1H, J1 = 7.6 Hz, J2 = 1.1 Hz), 7.28-7.32 (m, 1H), 7.36-7.41 (m, 1H), 7.48 (dd, 1H, J 1 = 7.6 Hz, J2 = 1.1 Hz), 7.53 (d, 1H, J = 8.4 Hz), 7.60 (d, 1H, J = 8.4 Hz), 7.93 (t, 1H, J = 6.1 Hz), 8.08 (s, 1H), 8.10 (s, 1H), 8.27 (dd, 1H, J1 = 8.4 Hz, J2 = 1.1 Hz), 8.44 (td, 1H, J 1 = 8.4 Hz, J2 = 1.1 Hz), 8.85 (dd, 1H, J1 = 6.1 Hz, J2 = 1.9Hz).

Compounds were tested for their Androgen Receptor activity in a transactivation assay and in a binding assay.

The (anti-)androgenic activity of test compounds (EC50 and intrinsic activity) was determined in an *in vitro* bioassay of Chinese hamster ovary (CHO) cells stably transfected with the human androgen receptor expression plasmid and a reporter plasmid in which the MMTV-promoter is linked to the luciferase reporter gene. The cell-line CHO-AR-pMMTV-LUC 1G12-AS-CA is described in Schoonen et al. (2000), Journal of Steroid Biochemistry and Molecular Biology 74(4):213-222. The antiandrogenic activity of a test compound was determined by the inhibition of the transactivation via the androgen receptor of the enzyme luciferase in the presence of 1 nM DHT (5 α-dihydrotestosterone, 17β-hydroxy-5α-androstan-3-one). Intrinsic activity of antiandrogenic activity was determined in the presence of the reference antiandrogen 2-Hydroxy-2-methyl-*N-*(4-nitro-3-trifluoromethyl-phenyl)-propionamide (Hydroxyflutamide), and set at 100%. For androgenic activity, maximal intrinsic activity in the presence of 100 nM DHT was set at 100%. The results are presented in Table 4.

**Table 4 Androgen receptor activity**

| **Cpd. number** | **X** | **R¹** | **R²** | **R³** | **R⁴** | **R⁵** | **ago EC50** | **ago efficacy** | **ant EC50** | **ant efficacy** |
|---|---|---|---|---|---|---|---|---|---|---|
| 1 | S | 2,5-difluorophenyl | 2-nitrophenyl | H | H | OMe | ++ | + | - | - |
| 2 | S | 2,fluoro,3-methylphenyl | 2-nitrophenyl | H | H | OMe | + | + | - | - |
| 3 | S | 2-chlorophenyl | 2-nitrophenyl | H | H | OMe | + | + | - | - |
| 4 | S | 2-cyanophenyl | 2-nitrophenyl | H | H | OMe | + | ± | ++ | ± |
| 5 | S | 2-fluorophenyl | 2-nitrophenyl | H | H | OMe | + | + | - | - |
| 6 | S | 2-methyl,3 -nitrophenyl | 2-nitrophenyl | H | H | OMe | + | + | - | - |
| 7 | S | 2-methylphenyl | 2-nitrophenyl | H | H | OMe | ++ | ++ | - | - |
| 8 | S | 2-nitrophenyl | 2-nitrophenyl | H | H | OMe | ++ | + | - | - |
| 9 | S | 2-pyridyl | 2-nitrophenyl | H | H | OMe | +++ | + | +++ | ± |
| 10 | S | 2-pyridyl HCl salt | 2-nitrophenyl | H | H | OMe | ++ | + | - | - |
| 11 | S | 2-tetrahydropyranyl | 2-nitrophenyl | H | H | OMe | ++ | + | - | - |
| 12 | S | 2-trifluoromethylphenyl | 2-nitrophenyl | H | H | OMe | + | + | - | - |
| 13 | S | 3-(5-methylisoxazolyl) | 2-nitrophenyl | H | H | OMe | + | ± | - | - |
| 14 | S | 3,4-dichloropherryl | 2-nitrophenyl | H | H | OMe | + | + | - | - |
| 15 | S | 3,5-dichlorophenyl | 2-nitrophenyl | H | H | OMe | ++ | ++ | - | - |
| 16 | S | 3,5-difluorophenyl | 2-(hydroxymethylphenyl) | H | H | OMe | ++ | + | ++ | ± |
| 17 | S | 3,5-difluorophenyl | 2-(N-methylbenzarnide) | H | H | OMe | - | - | + | + |
| 18 | S | 3,5-difluorophenyl | 2-benzamide | H | H | OMe | - | + | ++ | ± |
| 19 | S | 3,5-difluorophenyl | 2-benzoic acid methyl ester | H | H | OMe | + | + | - | - |
| 20 | S | 3,5-difluorophenyl | 2-cyanophenyl | H | H | OMe | ++ | ++ | - | - |
| 21 | S | 3,5-difluorophenyl | 2-methoxyphenyl | H | H | OMe | ++ | | - | - |
| 22 | S | 3,5-difluorophenyl | 2-nitrophenyl | H | H | Br | ++ | ++ | - | - |
| 23 | S | 3,5-difluorophenyl | 2-nitrophenyl | H | H | CF3 | +++ | ++ | - | - |
| 24 | S | 3,5-difluorophenyl | 2-nitrophenyl | H | H | CH2OH | ++ | ++ | - | - |
| 25 | S | 3,5-difluorophenyl | 2-nitrophenyl | H | H | Cl | +++ | ++ | - | - |
| 26 | S | 3,5-difluorophenyl | 2-nitrophenyl | H | H | CN | +++ | ++ | - | - |
| 27 | S | 3,5-difluorophenyl | 2-nitrophenyl | H | H | CO(1-pyrrolidinyl) | ++ | + | - | - |
| 28 | S | 3,5-difluorophenyl | 2-nitrophenyl | H | H | CO(4-morpholinyl) | ++ | + | - | - |
| 29 | S | 3,5-difluorophenyl | 2-nitrophenyl | H | H | CO[1-(4-methylpiperazinyl)] | ++ | ++ | - | - |
| 30 | S | 3,5-difluorophenyl | 2-nitrophenyl | H | H | CO2H | + | + | - | - |
| 31 | S | 3,5-difluorophenyl | 2-nitrophenyl | H | H | CO2Me | +++ | + | - | - |
| 32 | S | 3,5-difluorophenyl | 2-nitrophenyl | H | H | CONEt2 | ++ | + | + | ± |
| 33 | S | 3,5-difluorophenyl | 2-nitrophenyl | H | H | CONH2 | +++ | ++ | - | - |
| 34 | S | 3,5-difluorophenyl | 2-nitrophenyl | H | H | CONHCH2(2-furanyl) | ++ | + | - | - |
| 35 | S | 3,5-difluorophenyl | 2-nitrophenyl | H | H | CONHCH2(3-pyridyl) | ++ | ± | - | - |
| 36 | S | 3,5-difluorophenyl | 2-nitrophenyl | H | H | CONHCH2CH2Nme2 | ++ | + | - | - |
| 37 | S | 3,5-difluorophenyl | 2-nitrophenyl | H | H | CONHCH2CH2OH | +++ | ++ | - | - |
| 38 | S | 3,5-difluorophenyl | 2-nitrophenyl | H | H | CONHCH2CH2OMe | +++ | ++ | - | - |
| 39 | S | 3,5-difluorophenyl | 2-nitrophenyl | H | H | CONHCH2CO2Me | ++ | ++ | - | - |
| 40 | S | 3,5-difluorophenyl | 2-nitrophenyl | H | H | CONHCH2CONMe2 | ++ | + | - | - |
| 41 | S | 3,5-difluorophenyl | 2-nitrophenyl | H | H | CONHCH2cPr | +++ | + | - | - |
| 42 | S | 3,5-difluorophenyl | 2-nitrophenyl | H | H | CONHCH2iPr | ++ | + | - | - |
| 43 | S | 3,5-difluorophenyl | 2-nitrophenyl | H | H | CONHCH2Ph | ++ | ± | + | ± |
| 44 | S | 3,5-difluorophenyl | 2-nitrophenyl | H | H | CONHcPr | +++ | + | - | - |
| 45 | S | 3,5-difluorophenyl | 2-nitrophenyl | H | H | CONHEt | +++ | ++ | - | - |
| 46 | S | 3,5-difluorophenyl | 2-nitrophenyl | H | H | CONHiPr | ++ | + | - | ± |
| 47 | S | 3,5-difluorophenyl | 2-nitrophenyl | H | H | CONHMe | +++ | + | - | - |
| 48 | S | 3,5-difluorophenyl | 2-nitrophenyl | H | H | CONHnPr | ++ | + | + | ± |
| 49 | S | 3,5-difluorophenyl | 2-nitrophenyl | H | H | CONMe2 | +++ | + | - | - |
| 50 | S | 3,5-difluorophenyl | 2-nitrophenyl | H | H | F | ++ | ++ | - | - |
| 51 | S | 3,5-difluorophenyl | 2-nitrophenyl | F | H | H | ++ | ++ | - | - |
| 52 | S | 3,5-difluorophenyl | 2-nitrophenyl | H | H | H | +++ | ++ | - | - |
| 53 | S | 3,5-difluorophenyl | 2-nitrophenyl | H | F | H | ++ | ++ | - | - |
| 54 | S | 3,5-difluorophenyl | 2-nitrophenyl | Cl | H | H | ++ | ++ | - | - |
| 55 | S | 3,5-difluorophenyl | 2-nitrophenyl | Me | H | H | ++ | + | - | - |
| 56 | S | 3,5-difluorophenyl | 2-nitrophetryl | H | OH | H | +++ | - | - | - |
| 57 | S | 3,5-difluorophenyl | 2-nitrophenyl | H | H | NH2 | + | + | - | - |
| 58 | S | 3,5-difluorophenyl | 2-nitrophenyl | H | H | NHAc | +++ | ++ | - | - |
| 59 | S | 3,5-difluorophenyl | 2-nitrophenyl | H | H | NHCOCF3 | +++ | + | - | - |
| 60 | S | 3,5-difluorophenyl | 2-nitrophenyl | H | H | NO2 | +++ | ++ | - | - |
| 61 | S | 3,5-difluorophenyl | 2-nitrophenyl | H | H | OH | +++ | | - | - |
| 62 | SO2 | 3,5-difluorophenyl | 2-nitrophenyl | H | H | OMe | +++ | ++ | - | - |
| 63 | S | 3,5-difluorophenyl | 2-nitrophenyl | H | H | OMe | +++ | ++ | - | - |
| 64 | SO | 3,5-difluorophenyl | 2-nitrophenyl | H | H | OMe | - | ± | + | + |
| 65 | S | 3,5-difluorophenyl | 2-pyridyl | H | H | OMe | ++ | ++ | - | - |
| 66 | S | 3,5-difluorophenyl | 2-pyridyl-N-oxide | H | H | OMe | + | + | ++ | ± |
| 67 | S | 3-chlorophenyl | 2-nitrophenyl | H | H | OMe | ++ | ++ | - | - |
| 68 | S | 3-cyanophenyl | 2-nitrophenyl | H | H | OMe | +++ | ++ | - | - |
| 69 | S | 3-fluoro,5-trifluoromethylphenyl | 2-nitrophenyl | H | H | OMe | ++ | ++ | - | - |
| 70 | S | 3-fluoro,6-trifluoromethylphenyl | 2-nitrophenyl | H | H | OMe | ++ | + | - | - |
| 71 | S | 3-fluorophenyl | 2-nitrophenyl | H | H | OMe | +++ | ++ | - | - |
| 72 | S | 3-methoxyphenyl | 2-nitrophenyl | H | H | OMe | ++ | + | - | - |
| 73 | S | 3-methylphenyl | 2-nitrophenyl | H | H | OMe | ++ | ++ | - | - |
| 74 | S | 3-nitrophenyl | 2-nitrophenyl | H | H | OMe | +++ | ++ | - | - |
| 75 | S | 3-pyridyl | 2-nitrophenyl | H | H | OMe | +++ | ++ | - | - |
| 76 | S | 3-trifluoromethyl,4-chlorophenyl | 2-nitrophonyl | H | H | OMe | + | + | - | - |
| 77 | S | 3-trifluoromethyloxyphenyl | 2-nitrophenyl | H | H | OMe | ++ | ++ | - | - |
| 78 | S | 3-trifluoromethylphenyl | 2-nitrophenyl | H | H | OMe | ++ | ++ | - | - |
| 79 | S | 4-(2-methylthiazolyl) | 2-nitrophenyl | H | H | OMe | ++ | + | - | - |
| 80 | S | 4-(3,5-dimethylisoxazolyl) | 2-nitrophenyl | H | H | OMe | + | ± | - | ± |
| 81 | S | 4-chlorophenyl | 2-nitrophenyl | H | H | OMe | + | + | +++ | + |
| 82 | S | 4-cyanophenyl | 2-nitrophenyl | H | H | OMe | ++ | + | - | - |
| 83 | S | 4-fluorophenyl | 2-nitrophenyl | H | H | OMe | ++ | ++ | - | - |
| 84 | S | 4-methoxyphenyl | 2-nitrophenyl | H | H | OMe | + | ± | ± | ++ |
| 85 | S | 4-methylphenyl | 2-nitrophenyl | H | H | OMe | + | + | - | ± |
| 86 | S | 4-morpholinyl | 2-nitrophenyl | H | H | OMe | ± | ± | + | + |
| 87 | S | 5-(2-chlorothiazolyl) | 2-nitrophenyl | H | H | OMe | ++ | + | - | - |
| 88 | S | 5-(2-chlorothiophenyl) | 2-nitrophenyl | H | H | OMe | ++ | ++ | - | - |
| 89 | S | Cyclohexyl | 2-nitrophenyl | H | H | OMe | ++ | + | +++ | ± |
| 90 | S | Phenyl | 2-nitrophenyl | H | H | H | + | + | - | - |
| 91 | S | Phenyl | 2-nitrophenyl | H | OMe | H | - | - | ± | ++ |
| 92 | S | Phenyl | 2-nitrophenyl | H | H | OMe | +++ | ++ | - | - |

| | | | | | | | | | | |
|---|---|---|---|---|---|---|---|---|---|---|
| note 1: EC50 scale: +++ <5 nM; ++ between 5 and 100 nM; + <1000 nM; ± between 1000 and 10000 nM; - > 10000 nM note 2: efficacy scale (maximal intrinsic activity, i.e. intrinsic activity observed in the presence of 100 nM DHT, was set at 1.00): ++ ≥0.80; + between 0.50 and 0.80; ± between 0.20 and 0.50; - <0.20 | | | | | | | | | | |

The androgenic activity (EC50, potency, and efficacy) of the compounds in Table 5 was determined in an *in vitro* bioassay of Chinese hamster ovary (CHO) cells stably transfected with the human androgen receptor expression plasmid and a reporter plasmid in which the MMTV promotor is linked to the luciferase reporter gene. The cell line CHO-AR-pMMTV-LUC 1G12-A5-CA is described in Schoonen et al. (J. Steroid Biochem. Molec. Biol. 2002; 74: 213-222). The androgen receptor activity of compounds was determined in the presence of 0.1 µmol/l onapristone. The maximal efficacy in the presence of 100 nmol/l DHT was set as 100%. The potencies are expressed as percentage of DHT activity.

**Table 5 Androgen receptor activity**

| **Cpd. number** | **X** | **R¹** | **R²** | **R³** | **R⁴** | **R⁵** | **ago potency** | **ago efficacy** |
|---|---|---|---|---|---|---|---|---|
| 93 | S | 4-pyridyl | 2-nitrophenyl | H | H | OMe | ± | + |
| 94 | S | 3,4,5-trifluorophenyl | 2-nitrophenyl | H | H | OMe | + | ++ |
| 95 | S | 2,3,5-trifluorophenyl | 2-nitrophenyl | H | H | OMe | ± | ++ |
| 96 | S | 4-chloro-3-pyridyl | 2-nitrophenyl | H | H | OMe | ± | ± |
| 97 | S | 3,5-difluorophenyl | 2-nitrophenyl | H | H | NHCOCH2F | + | ++ |
| 98 | S | 3,5-difluorophenyl | 2-nitrophenyl | H | H | NHCOCHF2 | ++ | + |
| 99 | S | 3,5-difluorophenyl | 2-nitrophenyl | H | H | NHCOEt | ± | + |
| 100 | S | 2-pyridyl | 2-nitrophenyl | H | H | NHCOCH2F | + | ++ |
| 101 | S | 2-pyridyl | 2-nitrophenyl | H | H | NHCOMe | + | + |
| 102 | S | 2-pyridyl | 2-nitrophenyl | H | H | NHCOEt | + | + |
| 103 | S | 3-pyridyl | 2-nitrophenyl | H | H | NHCOMe | + | + |
| 104 | S | 3-pyridyl | 2-nitrophenyl | H | H | NHCOCH2F | + | - |
| 105 | S | 3-pyridyl | 2-nitrophenyl | H | H | NHCOEt | + | ± |
| 106 | S | 3,5-difluorophenyl | 2-nitrophenyl | H | H | NHSO2Me | + | ++ |
| 107 | S | 3,5-difluorophenyl | 2-nitrophenyl | H | H | NHSO2-(3-thiophene) | + | ++ |
| 108 | S | 3,5-difluorophenyl | 2-nitrophenyl | H | H | NHSO2-(1-Me-4-imidazolyl | + | ++ |
| 109 | S | 2-pyridyl | 2-nitrophenyl | H | H | CONMe2 | + | ++ |
| 110 | S | 2-pyridyl | 2-nitrophenyl | H | H | CONHMe | + | + |
| 111 | S | 2-pyridyl | 2-nitrophenyl | H | H | CONH2 | ± | ± |
| 112 | S | 3-pyridyl | 2-nitrophenyl | H | H | CONHMe | + | ± |
| 113 | S | 3-pyridyl | 2-nitrophenyl | H | H | CONHEt | + | + |
| 114 | S | 3-pyridyl | 2-nitrophenyl | H | H | CONHcyclopropyl | + | ++ |
| 115 | S | 3,5-pyrimidyl | 2-nitrophenyl | H | H | OMe | ++ | + |
| 116 | S | 2,4-pyrimidyl | 2-nitrophenyl | H | H | OMe | + | + |
| 117 | S | 2,5-pyrazyl | 2-nitrophenyl | H | H | OMe | + | + |
| 118 | S | 3,5-pyrimidyl | 2-nitrophenyl | H | H | NHCHO | ++ | + |
| 119 | S | 2-pyridyl | 2-nitrophenyl | H | H | CN | ++ | ++ |
| 120 | S | 2-pyridyl HCl salt | 2-nitrophenyl | H | H | CN | ++ | ++ |
| 121 | S | 3-pyridyl | 2-nitrophenyl | H | H | CN | + | ++ |
| 122 | S | 4-pyridyl | 2-nitrophenyl | H | H | CN | + | + |
| 123 | S | 3,5-pyrimidyl | 2-nitrophenyl | H | H | CN | ++ | ++ |
| 124 | S | 2,4-pyrimidyl | 2-nitrophenyl | H | H | CN | ++ | ++ |
| 125 | S | 3-cyanophenyl | 2-nitrophenyl | H | H | CN | + | ++ |

| | | | | | | | | |
|---|---|---|---|---|---|---|---|---|
| note 1: potency scale: ++ >5%; + between 1% and 5%; ± between 0.1 % and 1%. note 2: efficacy scale (maximal intrinsic activity, i.e. intrinsic activity observed in the presence of 100 nM DHT, was set at 1.00): ++ ≥0.80; + between 0.50 and 0.80; ± between 0.20 and 0.50; - <0.20. | | | | | | | | |

Determination of competitive binding to cytoplasmic human androgen receptor from recombinant CHO cells is used to estimate the relative binding affinity (RBA) of a test compound for androgen receptors present in the cytosol prepared of the recombinant CHO cell-line, CHO-AR-pMMTV-LUC 1G12-A5-CA.
Compound 63 and compound 92 were tested in this binding assay and both compounds were found to have a relative binding affinity >1% relative to DHT.

## Claims

1. A compound having the formula 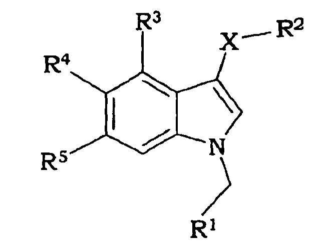 wherein
X is S, SO or SO₂;
R¹ is a 5- or 6-membered monocyclic, hetero- or homocyclic, saturated or unsaturated ring structure optionally substituted with one or more substituents selected from the group consisting of halogen, CN, (1C-4C)fluoroalkyl, nitro, (1C-4C)alkyl, (1C-4C)alkoxy or (1C-4C)fluoroalkoxy;
R² is 2-nitrophenyl, 2-cyanophenyl, 2-hydroxymethyl-phenyl, pyridin-2-yl, pyridin-2-yl-N-oxide, 2-benzamide, 2-benzoic acid methyl ester or 2-methoxyphenyl;
R³ is H, halogen or (1C-4C)alkyl;
R⁴ is H, OH, (1C-4C)alkoxy, or halogen;
R⁵ is H, OH, (1C-4C)alkoxy, NH₂, CN, halogen, (1C-4C)fluoroalkyl, NO₂, hydroxy(1C-4C)alkyl, CO₂H, CO₂(1C-6C)alkyl, or
R⁵ is NHR⁶, wherein R⁶ is (1C-6C)acyl optionally substituted with one or more halogens, S(O)₂(1C-4C)alkyl, or S(O)₂aryl optionally substituted with (1C-4C)alkyl or one or more halogens, or
R⁵ is C(O)N(R⁸R⁹), wherein R⁸ and R⁹ each independently are H, (3C-6C)cycloalkyl, or CH₂R¹⁰, wherein R¹⁰ is H, (1C-5C)alkyl, (1C-5C)alkenyl, hydroxy(1C-3C)alkyl, (1C-4C)alkylester of carboxy(1C-4C)alkyl, (1C-3C)alkoxy(1C-3C)alkyl, (mono- or di(1C-4C)alkyl)aminomethyl, (mono- or di(1C-4C)alkyl)aminocarbonyl, or a 3-, 4-, 5- or 6-membered monocyclic, homo- or heterocyclic, aromatic or non-aromatic ring, or R⁸ and R⁹ form together with the N a heterocyclic 5- or 6-membered saturated or unsaturated ring optionally substituted with (1C-4C)alkyl;
or a salt or hydrate form thereof.

2. A compound according to claim 1, **characterised in that**
R¹ is a 5- or 6-membered monocyclic, hetero- or homocyclic, saturated or unsaturated ring structure optionally substituted with one or more substituents selected from the group consisting of halogen, CN, CF₃, nitro, methoxy, trifluoromethoxy or methyl;
R² is 2-nitrophenyl, 2-cyanophenyl, 2-hydroxymethyl-phenyl, pyridin-2-yl, pyridin-2-yl-N-oxide, 2-benzamide, 2-benzoic acid methyl ester or 2-methoxyphenyl;
R³ is H, halogen or (1C-2C)alkyl;
R⁴ is H or F.

3. A compound according to claim 2, **characterised in that**
R⁵ is H, OH, (1C-4C)alkoxy, CN, halogen, (1C-4C)fluoroalkyl, NO₂, hydroxy(1C-4C)alkyl, CO₂(1C-6C)alkyl, or
R⁵ is NHR⁶, wherein R⁶ is (1C-6C)acyl optionally substituted with one or more halogens, S(O)₂(1C-4C)alkyl, or S(O)₂aryl optionally substituted with (1C-4C)alkyl or one or more halogens, or
R⁵ is C(O)N(R⁸,R⁹), wherein R⁸ and R⁹ each independently are H, (3C-6C)cycloalkyl, or CH₂R¹⁰, wherein R¹⁰ is H, (1C-5C)alkyl, (1C-5C)alkenyl, hydroxy(1C-3C)alkyl, (1C-4C)alkylester of carboxy(1C-4C)alkyl, (1C-3C)alkoxy(1C-3C)alkyl, (mono- or di(1C-4C)alkyl)aminomethyl, (mono- or di(1C-4C)alkyl)-aminocarbonyl, or a 3-, 4-, 5- or 6-membered monocyclic, homo- or heterocyclic, aromatic or non-aromatic ring, or R⁸ and R⁹ form together with the N a heterocyclic 5- or 6-membered saturated or unsaturated ring optionally substituted with (1C-4C)alkyl.

4. A compound according to claim 3, **characterised in that**
R³ is H or halogen;
R⁴ is H;
R⁵ is H, OH, (1C-4C)alkoxy, CN, F, Cl, CF₃, NO₂, hydroxy(1C-4C)alkyl, CO₂(1C-6C)alkyl, or
R⁵ is NHR⁶, wherein R⁶ is (1C-3C)acyl optionally substituted with one or more halogens or
R⁵ is C(O)N(R⁸,R⁹), wherein R⁸ and R⁹ each independently are H, (3C-5C)cycloalkyl, or CH₂R¹⁰, wherein R¹⁰ is H, (1C-5C)alkyl, (1C-5C)alkenyl, hydroxy(1C-3C)alkyl, (1C-2C)alkylester of carboxy(1C-2C)alkyl, (1C-3C)alkoxy(1C-3C)alkyl, (mono- or di(1C-4C)alkyl)aminomethyl, (mono- or di(1C-4C)alkyl)aminocarbonyl, (3C-5C)cycloalkyl, or a 5-membered heterocyclic ring.

5. A compound according to claim 4, **characterised in that**
X is S or SO₂;
R² is 2-nitrophenyl, 2-hydroxymethyl-phenyl, 2-benzamide, 2-methoxyphenyl, 2-cyanophenyl or pyridin-2-yl;
R³ is H or F;
R⁵ is H, OH, (1C-2C)alkoxy, CN, F, Cl, CF₃, NO₂, hydroxy(1C-4C)alkyl, CO₂(1C-4C)alkyl, or
R⁵ is NHR⁶, wherein R⁶ is formyl, acetyl, fluoroacetyl, difluoroacetyl, or trifluoroacetyl, or
R⁵ is C(O)N(R⁸,R⁹), wherein R⁸ is H, and R⁹ is H, cyclopropyl or
R⁹ is CH₂R¹⁰, wherein R¹⁰ is H, (1C-2C)alkyl, hydroxy(1C-2C)alkyl, methoxy(1C-2C)alkyl, cyclopropyl.

6. A compound according to claim 5, **characterised in that**
X is S;
R¹ is 3,5-difluorophenyl, pyridin-2-yl, pyridin-3-yl, pyrimidin-5-yl, pyrimidin-4-yl, pyrazin-2-yl, 3-fluorophenyl, 3-cyanophenyl, or 3-nitrophenyl;
R² is 2-nitrophenyl, 2-hydroxymethyl-phenyl, 2-methoaryphenyl, 2-cyanophenyl or pyridin-2-yl;
R³ is H;
R⁵ is OH, (1C-2C)alkoxy, CN, CF₃, NO₂, hydroxy(1C-4C)alkyl, CO₂(1C-4C)alkyl, or NHR⁶, wherein R⁶ is formyl, acetyl, fluoroacetyl, difluoroacetyl, or trifluoroacetyl.

7. A compound according to claim 6, **characterised in that**
R¹ is 3,5-difluorophenyl, pyridin-2-yl, pyridin-3-yl, pyrimidin-5-yl, pyrimidin-4-yl, or pyrazin-2-yl;
R² is 2-nitrophenyl, or 2-hydroxymethyl-phenyl;
R⁵ is OH, (1C-2C)alkoxy, CN, hydroxy(1C-4C)alkyl, or NHR⁶, wherein R⁶ is formyl, acetyl, fluoroacetyl, difluoroacetyl, or trifluoroacetyl.

8. A compound according to claim 7, **characterised in that**
R¹ is 3,5-difluorophenyl, pyridin-2-yl, pyridin-3-yl, pyrimidin-5-yl, or pyrimidin-4-yl;
R² is 2-nitrophenyl;
R⁵ is OH, (1C-2C)alkoxy, CN, or NHR⁶, wherein R⁶ is formyl, acetyl, fluoroacetyl, difluoroacetyl, or trifluoroacetyl.

9. A compound according to claim 8 selected from the group consisting of 6-Methoxy-3-(2-nitro-phenylsulfanyl)-1-pyrimidin-5-ylmethyl-1*H*-indole, 3-(2-Nitro-phenylsulfanyl)-1-pyridin-2-ylmethyl-1*H*-indole-6-carbonitrile, 3-(2-Nitrophenylsulfanyl)-1-pyridin-2-ylmethyl-1*H*-indole-6-carbonitrile-hydrochloride, 3-(2-Nitro-phenylsulfanyl)-1-pyrimidin-5-ylmethyl-1*H*-indole-6-carbonitrile, 3-(2-Nitro-phenylsulfanyl)-1-pyrimidin-4-ylmethyl-1*H*-indole-6-carbonitrile, *N*-[1-(3,5-Difluoro-benzyl)-3-(2-nitro-phenylsulfanyl)-1*H*-indol-6-yl]-2-fluoroacetamide, and *N*-[3-(2-Nitro-phenylsulfanyl)-1-pyrimidin-5-ylmethyl-1*H*-indol-6-yl]-formamide.

10. A compound according to claim 5, **characterised in that**
X is S;
R¹ is 3,5-difluorophenyl, pyridin-2-yl, pyridin-3-yl, 3-fluorophenyl, 3-cyanophenyl, or 3-nitrophenyl;
R² is 2-nitrophenyl, 2-hydroxymethyl-phenyl, 2-methoxyphenyl, 2-cyanophenyl or pyridin-2-yl;
R³ is H;
R⁵ is C(O)N(R⁸,R⁹), wherein R⁸ is H, and R⁹ is H, or CH₂R¹⁰, wherein R¹⁰ is H, (1C-2C)alkyl, hydroxy(1C-2C)alkyl, or methoxy(1C-2C)alkyl.

11. A compound according to claim 10, **characterised in that**
R¹ is 3,5-difluorophenyl, pyridin-2-yl, or pyridin-3-yl;
R² is 2-nitrophenyl, or 2-hydroxymethyl-phenyl;
R⁵ is C(O)N(R⁸,R⁹), wherein R⁸ is H, and R⁹ is CH₂R¹⁰, wherein R¹⁰ is H, or (1C-2C)alkyl.

12. A compound according to claim 11 which is 1-(3,5-Difluoro-benzyl)-3-(2-nitrophenylsulfanyl)-1*H*-indole-6-carboxylic acid methylamide.

13. A compound according to claim 4, **characterised in that**
X is S;
R¹ is 3,5-difluorophenyl, pyridin-2-yl, pyridin-3-yl, 3-fluorophenyl, 3-cyanophenyl, or 3-nitrophenyl;
R² is 2-nitrophenyl, 2-hydroxymethyl-phenyl, 2-methoxyphenyl, 2-cyanophenyl or pyridin-2-yl;
R³ is H;
R⁵ is C(O)N(R⁸,R⁹), wherein R⁸ and R⁹ each independently are H, or CH₂R¹⁰, wherein R¹⁰ is H, (1C-5C)alkyl, (1C-5C)alkenyl, hydroxy(1C-3C)alkyl, (1C-3C)alkoxy(1C-3C)alkyl, or (mono- or di(1C-4C)alkyl)aminomethyl.

14. A compound according to claim 13, **characterised in that**
R¹ is 3,5-difluorophenyl, pyridin-2-yl, or pyridin-3-yl;
R² is 2-nitrophenyl, or 2-hydroxymethyl-phenyl;
R⁵ is C(O)N(R⁸,R⁹), wherein R⁸ and R⁹ each independently are H, or CH₂R¹⁰, wherein R¹⁰ is H, (1C-5C)alkyl, hydroxy(1C-3C)alkyl, or (1C-3C)alkoxy(1C-3C)alkyl.

15. A compound according to claim 14 which is 1-(3,5-Difluoro-benzyl)-3-(2-nitrophenylsulfanyl)-1*H*-indole-6-carboxylic acid dimethylamide.

16. The compound of any one of claims 1 - 15 for use in therapy.

17. A pharmaceutical composition comprising a compound according to any one of claims 1-15 and a pharmaceutically acceptable carrier.

18. A pharmaceutical composition according to claim 17 for the treatment of a disorder selected from the group consisting of an androgen-receptor related disorder, an androgen related disorder and androgen insufficiency.

19. A use of a compound according to any one of claims 1-15 for the manufacture of a medicament for the treatment of androgen-receptor related disorders, androgen related disorders and androgen insufficiency.

## Patentansprüche

1. Verbindung mit der Formel 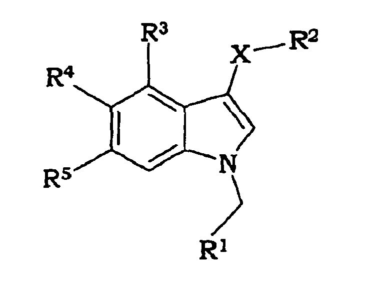 wobei
X S, SO oder SO₂ ist;
R¹ eine 5- oder 6-elementige monozyklische, hetero- oder homozyklische, gesättigte oder ungesättigte Ringstruktur ist, optional substituiert mit einem oder mehreren Substituenten ausgewählt aus der Gruppe bestehend aus einem Halogen, CN, (1C-4C) Fluoralkyl, Nitro, (1C-4C) Alkyl, (1C-4C) Alkoxy oder (1C-4C) Fluoralkoxy;
R² 2-Nitrophenyl, 2-Cyanophenyl, 2-Hydroxymethyl-phenyl, Pyridin-2-yl, Pyridin- 2-yl-N-oxid, 2-Benzamid, 2-Benzoesäuremethylester oder 2-Methoxyphenyl ist;
R³ H, ein Halogen oder (1C-4C) Alkyl ist;
R⁴ H, OH, (1C-4C) Alkoxy, oder ein Halogen ist;
R⁵ H, OH, (1C-4C) Alkoxy, NH₂, CN, ein Halogen, (1C-4C) Fluoralkyl, NO₂, Hydroxy (1C-4C) alkyl, CO₂H, CO₂ (1C-6C) Alkyl ist, oder
R⁵ NHR⁶ ist, wobei R⁶ (1C-6C) Acyl ist, optional substituiert mit einem oder mehreren Halogenen, S(O)₂ (1C-4C) Alkyl, oder S(O)₂ Aryl optional substituiert mit (1C-4C) Alkyl oder einem oder mehreren Halogenen, oder
R⁵ C(O)N (R⁸,R⁹) ist, wobei R⁸ und R⁹ jeweils unabhängig H, (3C-6C) Cycloalkyl, oder CH₂R¹⁰ sind, wobei R¹⁰ H, (1C-5C) Alkyl, (1C-5C) Alkenyl, Hydroxy (1C-3C) alkyl, (1C-4C) Alkylester von Carboxy (1C-4C) alkyl, (1C-3C) Alkoxy (1C-3C) alkyl, (mono- oder di-(1C-4C) Alkyl) aminomethyl, (mono- oder di-(1C-4C) Alkyl) aminocarbonyl ist, oder ein 3-, 4-, 5- oder 6- elementiger monozyklischer, homo- oder heterozyklischer, aromatischer oder nicht-aromatischer Ring, oder R⁸ und R⁹ zusammen mit einem N einen heterozyklischen 5- oder 6-elementigen gesättigten oder ungesättigten Ring bilden, optional substituiert mit (1C-4C) Alkyl; oder eine Salz- oder Hydratform davon.

2. Verbindung gemäss Anspruch 1, **dadurch gekennzeichnet, dass**
R¹ eine 5- oder 6-elementige monozyklische, hetero- oder homozyklische, gesättigte oder ungesättigte Ringstruktur ist, optional substituiert mit einem oder mehreren Substituenten ausgewählt aus der Gruppe bestehend aus einem Halogen, CN, CF₃, Nitro, Methoxy, Trifluormethoxy oder Methyl;
R² 2-Nitrophenyl, 2-Cyanophenyl, 2-Hydroxymethyl-phenyl, Pyridin-2-yl, Pyridin-2-yl-N-oxid, 2-Benzamid, 2-Benzoesäuremethylester oder 2-Methoxyphenyl ist;
R³ H, ein Halogen oder (1C-2C) Alkyl ist;
R⁴ H oder F ist.

3. Verbindung gemäss Anspruch 2, **dadurch gekennzeichnet, dass**
R⁵ H, OH, (1C-4C) Alkoxy, CN, ein Halogen, (1C-4C) Fluoralkyl, NO₂, Hydroxy (1C-4C) Alkyl, CO₂ (1C-6C) Alkyl ist, oder
R⁵ NHR⁶ ist, wobei R⁶ (1C-6C) Acyl ist, optional substituiert mit einem oder mehreren Halogenen, S(O)₂ (1C-4C) Alkyl, oder S(O)₂ Aryl optional substituiert mit (1C-4C) Alkyl oder einem oder mehreren Halogenen, oder
R⁵ C(O)N(R⁸,R⁹) ist, wobei R⁸ und R⁹ jeweils unabhängig H, (3C-6C) Cycloalkyl, oder CH₂ R¹⁰ sind, wobei R¹⁰ H, (1C-5C) Alkyl, (1C-5C) Alkenyl, Hydroxy (1C-3C) alkyl, (1C-4C) Alkylester von Carboxy (1C-4C) alkyl, (1C-3C) Alkoxy (1C-3C) alkyl, (mono- oder di-(1C-4C) Alkyl) aminomethyl, (mono- oder di-(1C-4C) Alkyl)-aminocarbonyl ist, oder ein 3-, 4-, 5- oder 6- elementiger monozyklischer, homo- oder heterozyklischer, aromatischer oder nicht-aromatischer Ring, oder R⁸ und R⁹ zusammen mit dem N einen heterozyklischen 5- oder 6-elementigen gesättigten oder ungesättigten Ring bilden, optional substituiert mit (1C-4C) alkyl.

4. Verbindung gemäss Anspruch 3, **dadurch gekennzeichnet, dass**
R³ H oder ein Halogen ist;
R⁴ H ist;
R⁵ H, OH, (1C-4C) Alkoxy, CN, F, Cl, CF₃, NO₂, Hydroxy (1C-4C) alkyl, CO₂ (1C-6C) Alkyl ist, oder
R⁵ NHR⁶ ist, wobei R⁶ (1C-3C)Acyl ist, optional substituiert mit einem oder mehreren Halogenen oder
R⁵ C(O)N(R⁸,R⁹) ist, wobei R⁸ und R⁹ jeweils unabhängig H, (3C-5C) Cycloalkyl, oder CH₂ R¹⁰ sind, wobei R¹⁰ H, (1C-5C) Alkyl, (1C-5C) Alkenyl, Hydroxy (1C-3C) alkyl, (1C-2C) Alkylester von Carboxy (1C-2C) alkyl, (1C-3C) Alkoxy (1C-3C) alkyl, (mono- oder di-(1C-4C) Alkyl) aminomethyl, (mono- oder di-(1C-4C) Alkyl) aminocarbonyl, (3C-5C) Cycloalkyl, oder ein 5-elementiger heterozyklischer Ring ist.

5. Verbindung gemäss Anspruch 4, **dadurch gekennzeichnet, dass**
X S oder SO₂ ist;
R² 2-Nitrophenyl, 2-Hydroxymethyl-phenyl, 2-Benzamid, 2-Methoxyphenyl, 2-Cyanophenyl oder Pyridin-2-yl ist;
R³ H oder F ist;
R⁵ H, OH, (1C-2C) Alkoxy, CN, F, Cl, CF₃, NO₂, Hydroxy (1C-4C) Alkyl, CO₂ (1C-4C) Alkyl ist, oder
R⁵ NHR⁶ ist, wobei R⁶ Formyl, Acetyl, Fluoracetyl, Difluoracetyl, oder Trifluoracetyl ist, oder
R⁵ C(O)N (R⁸,R⁹) ist, wobei R⁸ H ist, und R⁹ H, Cyclopropyl ist oder R⁹ CH₂ R¹⁰ ist, wobei R¹⁰ H, (1C-2C) Alkyl, Hydroxy (1C-2C) alkyl, Methoxy (1C-2C) alkyl, Cyclopropyl ist.

6. Verbindung gemäss Anspruch 5, **dadurch gekennzeichnet, dass**
X S ist;
R¹ 3,5-Difluorphenyl, Pyridin-2-yl, Pyridin-3-yl, Pyrimidin-5-yl, Pyrimidin-4-yl, Pyrazin-2-yl, 3-Fluorphenyl, 3-Cyanophenyl, oder 3-Nitrophenyl ist;
R² 2-Nitrophenyl, 2-Hydroxymethyl-phenyl, 2-Methoxyphenyl, 2-Cyanophenyl oder Pyridin-2-yl ist;
R³ H ist;
R⁵ OH, (1C-2C) Alkoxy, CN, CF₃, NO₂, Hydroxy (1C-4C) alkyl, CO₂ (1C-4C) Alkyl, oder NHR⁶ ist, wobei R⁶ Formyl, Acetyl, Fluoracetyl, Difluoracetyl, oder Trifluoracetyl ist.

7. Verbindung gemäss Anspruch 6, **dadurch gekennzeichnet, dass**
R¹ 3,5-Difluorphenyl, Pyridin-2-yl, Pyridin-3-yl, Pyrimidin-5-yl, Pyrimidin-4-yl, oder pyrazin-2-yl ist;
R² 2-Nitrophenyl, oder 2-Hydroxymethyl-phenyl ist;
R⁵ OH, (1C-2C) Alkoxy, CN, Hydroxy (1C-4C) alkyl, oder NHR⁶ ist, wobei R⁶ Formyl, Acetyl, Fluoracetyl, Difluoracetyl, oder Trifluoracetyl ist.

8. Verbindung gemäss Anspruch 7, **dadurch gekennzeichnet, dass**
R¹ 3,5-Difluorphenyl, Pyridin-2-yl, Pyridin-3-yl, Pyrimidin-5-yl, oder Pyrimidin-4-yl ist;
R² 2-Nitrophenyl ist;
R⁵ OH, (1C-2C) Alkoxy, CN, oder NHR⁶ ist, wobei R⁶ Formyl, Acetyl, Fluoracetyl, Difluoracetyl, oder Trifluoracetyl ist.

9. Verbindung gemäss Anspruch 8, ausgewählt aus der Gruppe bestehend aus 6- Methoxy-3-(2-nitro-phenylsulfanyl)-]-pyrinlidin-5-ylmethyl-1*H*-indol, 3-(2-Nitro-phenylsulfanyl)-1-pyridin-2-ylmethyl-1-indol-6-carbonitril, 3-(2-Nitro-phenylsulfanyl)-1-pyridin-2-ylmethyl-1*H*-indol-6-carbonitril-hydrochlorid, 3-(2-Nitro-phenyl sulfanyl)-1-pyrklidin-5-ylmethy]-1*H*-indol-6-carbonitril, 3-(2-Nitro-phenylsulfanyl)-1-pyrimidin-4-ylmethyl-1*H-*indol-6-carbonitril, N-[1-(3,5-Difluorbenzyl)-3-(2-nitro-phenylsulfanyl)-1*H-*indol-6-yl]-2-fluor-acetamid, und N-[3-(2-Nitrophenylsulfanyl)-1-pyrimidin-5-ylmethyl-1*H*-indol-6-yl]-formamid ist.

10. Verbindung gemäss Anspruch 5, **dadurch gekennzeichnet, dass**
X S ist;
R¹ 3,5-Difluorphenyl, Pyridin-2-yl, Pyridin-3-yl, 3-Fluorphenyl, 3-Cyanophenyl, oder 3-Nitrophenyl ist;
R² 2-Nitrophenyl, 2-Hydroxymethyl-phenyl, 2-Methoxyphenyl, 2-Cyanophenyl oder Pyridin-2-yl ist;
R³ H ist;
R⁵ C(O)N(R⁸,R⁹) ist, wobei R⁸ H ist, und R⁹ H, oder CH₂R¹⁰ ist, wobei R¹⁰ H, (1C-2C) Alkyl, Hydroxy (1C-2C) alkyl, oder Methoxy (1C-2C) alkyl ist.

11. Verbindung gemäss Anspruch 10, **dadurch gekennzeichnet, dass**
R¹ 3,5-Difluorphenyl, Pyridin-2-yl, oder Pyridin-3-yl ist;
R² 2-Nitrophenyl, oder 2-Hydroxymethyl-phenyl ist;
R⁵ C(O)N(R⁸,R⁹) ist, wobei R⁸ H ist, und R⁹ CH₂R¹⁰ ist, wobei R¹⁰ H ist, oder (1C-2C) Alkyl ist.

12. Verbindung gemäss Anspruch 11, welche 1-(3,5-Difluor-benzyl)-3-(2-nitrophenylsulfanyl)-1*H*-indol-6-Carboxylsäuremethylamid ist.

13. Verbindung gemäss Anspruch 4, **dadurch gekennzeichnet, dass**
X S ist;
R¹ 3,5-Difluorphenyl, Pyridin-2-yl, Pyridin-3-yl, 3-Fluorphenyl, 3-Cyanophenyl, oder 3-Nitrophenyl ist;
R² 2-Nitrophenyl, 2-Hydroxymethyl-phenyl, 2-Methoxyphenyl, 2-Cyanophenyl oder Pyridin-2-yl ist;
R³ H ist;
R⁵ C(O)N(R⁸,R⁹) ist, wobei R⁸ und R⁹ jeweils unabhängig H, oder CH₂R¹⁰ sind, wobei R¹⁰ H, (1C-5C) Alkyl, (1C-5C) Alkenyl, Hydroxy (1C-3C) alkyl, (1C-3C) Alkoxy (1C-3C) alkyl, oder (mono- oder di-(1C-4C) Alkyl) aminomethyl ist.

14. Verbindung gemäss Anspruch 13, **dadurch gekennzeichnet, dass**
R¹ 3,5-Difluorphenyl, Pyridin-2-yl, oder Pyridin-3-yl ist;
R² 2-Nitrophenyl, oder 2-Hydroxymethyl-phenyl ist;
R⁵ C(O)N(R⁸,R⁹) ist, wobei R⁸ und R⁹ jeweils unabhängig H, oder CH₂ R¹⁰ sind, wobei R¹⁰ H, (1C-5C) Alkyl, Hydroxy (1C-3C) alkyl, oder (1C-3C) Alkoxy (1C-3C) alkyl ist.

15. Verbindung gemäss Anspruch 14, welche 1-(3,5-Difluor-benzyl)-3-(2-nitrophenylsulfanyl)-1*H*-indol-6-Carboxylsäuredimethylamid ist.

16. Verbindung gemäss irgendeinem der Ansprüche 1 - 15 für die Verwendung in einer Therapie.

17. Pharmazeutische Zusammensetzung umfassend eine Verbindung gemäss irgendeinem der Ansprüche 1-15 und einen pharmazeutisch verträglichen Träger.

18. Pharmazeutische Zusammensetzung gemäss Anspruch 17 für die Behandlung einer Störung, ausgewählt aus der Gruppe bestehend aus einer Androgenrezeptor bezogenen Störung, einer Androgen bezogenen Störung und Androgeninsuffizienz.

19. Verwendung einer Verbindung gemäss irgendeinem der Ansprüche 1 - 15 für die Herstellung eines Medikamentes für die Behandlung einer Androgenrezeptor bezogenen Störung, einer Androgen bezogenen Störung und einer Androgeninsuffizienz.

## Revendications

1. Un composé, ou un sel ou hydrate en dérivant, présentant la formule: 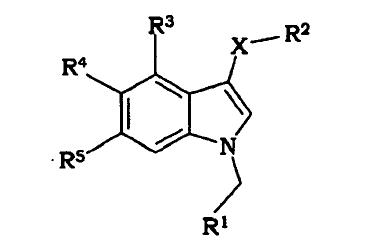 dans laquelle
- X est S, SO ou SO₂;
- R¹ est une structure cyclique monocyclique, hétéro- ou homocyclique comprenant 5 ou 6 atomes, saturée ou insaturée, éventuellement substituée par un ou plusieurs substituants choisis dans le groupe formé par les halogènes, CN, (C₁-C₄)-fluoroalkyl, nitro, (C₁-C₄)-alkyl, (C₁-C₄)-alkoxy ou (C₁-C₄)-fluoroalkoxy;
- R² est un radical 2-nitrophényl, 2-cyanophényl, 2-hydroxyméthyl-phényl, pyridin-2-yl, pyridin-2-yl-N-oxyde, 2-benzamide, ester méthylique de l'acide 2-benzoïque ou 2-méthoxyphényl;
- R³ est un atome d'hydrogène, d'halogène ou un radical (C₁-C₄)-alkyl;
- R⁴ est un atome d'hydrogène, OH, (C₁-C₄)-alkoxy, ou un halogène;
- R⁵ est un atome d'hydrogène, OH, (C₁-C₄)-alkoxy, NH₂, CN, halogène, (C₁-C₄)-fluoroalkyl, NO₂, hydroxy-(C₁-C₄)-alkyl, CO₂H, CO₂(C₁-C₆)-alkyl,
ou
R⁵ est NHR⁶, où R⁶ est (C₁-C₆)-acyl éventuellement substitué par un ou plusieurs halogènes, S(O)₂(C₁-C₄)-alkyl, ou S(O)₂-aryl éventuellement substitué par un radical (C₁-C₄)-alkyl ou un ou plusieurs halogènes,
ou
- R⁵ est C(O)N(R⁸,R⁹), où R⁸ et R⁹ chacun, indépendamment l'un de l'autre, sont hydrogène, (C₃-C₆)-cycloalkyl, ou CH₂R¹⁰, où R¹⁰ est un atome d'hydrogène, un radical (C₁-C₅)-alkyl, (C₁-C₅)-alkényl, hydroxy-(C₁-C₃)-alkyl, ester (C₁-C₄)-alkylique de carboxy-(C₁-C₄)-alkyl, (C₁-C₃)-alkoxy-(C₁-C₃)-alkyl, [mono-
ou di-(C₁-C₄)-alkyl]-aminométhyl, [mono- ou di-(C₁-C₄)-alkyl]-aminocarbonyl, ou un noyau monocyclique, homo- ou hétérocyclique, aromatique ou non-aromatique comprenant 3, 4, 5 ou 6-atomes, ou R⁸ et R⁹ forment ensemble avec l'atome de N un noyau hétérocyclique saturé ou insaturé comprenant 5 ou 6 atomes, éventuellement substitué par un radical (C₁-C₄)-alkyl.

2. Un composé selon la revendication 1, **caractérisé en ce que**
- R¹ est une structure cyclique monocyclique, hétéro- ou homocyclique comprenant 5 ou 6 atomes, saturée ou insaturée, éventuellement substitué par un ou plusieurs substituants choisis dans le groupe formé par les halogènes, CN, CF₃, nitro, méthoxy, trifluorométhoxy ou méthyl;
- R² est 2-nitrophényl, 2-cyanophényl, 2-hydroxyméthyl-phényl, pyridin-2-yl, pyridin-2-yl-N-oxyde, 2-benzamide, ester méthylique de l'acide 2-benzoique ou 2-méthoxy-phényl;
- R³ est un atome d'hydrogène, d'halogène ou (C₁-C₂)-alkyl;
- R⁴ est un atome de H ou de F.

3. Un composé selon la revendication 2, **caractérisé en ce que**
- R⁵ est un atome d'hydrogène, OH, (C₁-C₄)-alkoxy, CN, halogène, (C₁-C₄)-fluoroalkyl, NO₂, hydroxy-(C₁-C₄)-alkyl, CO₂(C₁-C₆)-alkyl,
ou
- R⁵ est NHR⁶, où R⁶ est (C₁-C₆)-acyl éventuellement substitué par un ou plusieurs halogènes, S(O)₂(C₁-C₄)-alkyl, ou S(O)₂-aryl éventuellement substitué par (C₁-C₄)-alkyl ou un ou plusieurs halogènes,
ou
- R⁵ est C(O)N(R⁸,R⁹), où R⁸ et R⁹ chacun, indépendamment l'un de l'autre, sont l'hydrogène, (C₃-C₆)-cycloalkyl, ou CH₂R¹⁰, dans laquelle R¹⁰ est un atome d'hydrogène, (C₁-C₅)-alkyl, (C₁-C₅)-alkényl, hydroxy-(C₁-C₃)-alkyl, ester (C₁-C₄)-alkylique de carboxy-(C₁-C₄)-alkyl, (C₁-C₃)-alkoxy-(C₁-C₃)-alkyl, [mono- ou di-(C₁-C₄)-alkyl]-aminométhyl, [mono- ou di(C₁-C₄]-alkyl)-aminocarbonyl, ou un noyau monocyclique, homo- ou hétérocyclique, aromatique ou non-aromatique comportant 3, 4, 5 ou 6-atomes,
ou
- R⁸ et R⁹ forment ensemble avec l'atome de N un noyau hétérocyclique saturé ou insaturé comportant 5 ou 6 atomes éventuellement substitué par un radical (C₁-C₄)-alkyl.

4. Un composé selon la revendication 3, **caractérisé en ce que**
- R³ est un atome d'hydrogène ou d'halogène;
- R⁴ est un atome d'hydrogène;
- R⁵ est un atome d'hydrogène, OH, (C₁-C₄)-alkoxy, CN, F, Cl, CF₃, NO₂, hydroxy-(C₁-C₄)-alkyl, CO₂(C₁-C₆)-alkyl, ou
- R⁵ est NHR⁶, où R⁶ est (C₁-C₃)-acyl éventuellement substitué par un ou plusieurs halogènes
ou
- R⁵ est C(O)N(R⁸,R⁹), où R⁸ et R⁹ chacun, indépendamment l'un de l'autre, sont un atome d'hydrogène, (C₃-C₅)-cycloalkyl, ou CH₂R¹⁰, dans laquelle R¹⁰ est un atome d'hydrogène, (C₁-C₅)-alkyl, (C₁-C₅)-alkényl, hydroxy-(C₁-C₃)-alkyl, (C₁-C₂)-alkylester de carboxy-(C₁-C₂)-alkyl, (C₁-C₃)-alkoxy-(C₁-C₃)-alkyl, [mono- ou di-(C₁-C₄)-alkyl]-aminométhyl, [mono- ou di-(C₁-C₄)-alkyl]-aminocarbonyl, (C₃-C₅)-cycloalkyl, ou un noyau hétérocyclique comportant 5 atomes.

5. Un composé selon la revendication 4, **caractérisé en ce que**
- X est S ou SO₂;
- R² est un radical 2-nitrophényl, 2-hydroxyméthyl-phényl, 2-benzamide, 2-méthoxyphényl, 2-cyanophényl ou pyridin-2-yl;
- R³ est un atome d'hydrogène ou de fluor;
- R⁵ est un atome d'hydrogène, OH, (C₁-C₂)-alkoxy, CN, F, Cl, CF₃, NO₂, hydroxy-(C₁-C₄)-alkyl, CO₂(C₁-C₄)-alkyl, ou
- R⁵ est NHR⁶, où R⁶ est un radical formyl, acétyl, fluoroacétyl, difluoroacétyl, ou trifluoroacétyl,
ou
- R⁵ est C(O)N(R⁸,R⁹), où R⁸ est un atome d'hydrogène et R⁹ est un atome d'hydrogène ou un radical cyclopropyl
ou
- R⁹ est CH₂R¹⁰, où R¹⁰ est un atome d'hydrogène ou un radical (C₁-C₂)-alkyl, hydroxy-(C₁-C₂)-alkyl, méthoxy-(C₁-C₂)-alkyl, cyclopropyl.

6. Un composé selon la revendication 5, **caractérisé en ce que**
- X est S;
- R¹ est un radical 3,5-difluorophényl, pyridin-2-yl, pyridin-3-yl, pyrimidin-5-yl, pyrimidin-4-yl, pyrazin-2-yl, 3-fluorophényl, 3-cyanophényl, ou 3-nitrophényl;
- R² est un radical 2-nitrophényl, 2-hydroxyméthyl-phényl, 2-méthoxyphényl, 2-cyanophényl ou pyridin-2-yl;
- R³ est un atome d'hydrogène;
- R⁵ est OH, (C₁-C₂)-alkoxy, CN, CF₃, NO₂, hydroxy-(C₁-C₄)-alkyl- CO₂(C₁-C₄)-alkyl, ou NHR⁶, où R⁶ est un radical formyl, acétyl, fluoroacétyl, difluoroacétyl ou trifluoroacétyl.

7. Un composé selon la revendication 6, **caractérisé en ce que**
- R¹ est un radical 3,5-difluorophényl, pyridin-2-yl-, pyridin-3-yl-, pyrimidin-5-yl-, pyrimidin-4-yl, ou pyrazin-2-yl;
R² est un radical 2 -nitrophényl, ou 2-hydroxyméthyl-phényl;
R⁵ est OH, (C₁-C₂)-alkoxy, CN, hydroxy-(C₁-C₄)-alkyl, ou NHR⁶ où R⁶ est un radical formyl, acétyl, fluoroacétyl, difluoroacetyl ou trifluoroacétyl.

8. Un composé selon la revendication 7, **caractérisé en ce que**
- R¹ est un radical 3,5-difluorophényl, pyridin-2-yl, pyridin-3-yl- pyrimidin-5-yl, ou pyrimidin-4-yl;
R² est 2-nitrophényl;
R⁵ est OH, (C₁-C₂)-alkoxy, CN, ou NHR⁶, où R⁶ est un radical formyl, acétyl, fluoroacétyl, difluoroacétyl ou trifluoroacétyl.

9. Un composé selon la revendication 8, choisi dans le groupe consistant en les 6-méthoxy-3-(2-nitro-phénylsulfanyl)-1-pyrimidin-5-ylméthyl-1H-indole 3-(2nitro-phénylsulfanyl)-1-pyridin-2-ylméthyl-1H-indole-6-carbonitrile, chlorhydrate de 3-(2-nitro-phénylsulfanyl)-1-pyridin-2-ylméthyl-1H-indole-6-carbonitrile, 3-(2-nitro-phénylsulfanyl)-1-pyrimidin-5-ylméthyl-1H-indole-6-carbonitrile, 3-(2-nitro-phénylsulfanyl)-1-pyrimidin-4-ylméthyl-1H-indole-6-carbonitrile, N-[1-(3,5-difluoro-benzyl)-3-(2-nitro-phénylsulfanyl)-1H-indol-6-yl)-2-fluoroacétamide, et N-[3-(2-nitro-phénylsulfanyl)-1-pyrimidin-5-ylméthyl-1H-indol-6-yl]-formamide.

10. Un composé selon la revendication 5, **caractérisé en ce que**
- X est S;
- R¹ est un radical 3,5-difluorophényl, pyridin-2-yl, pyridin-3-yl, 3-fluorophényl, 3-cyanophényl ou 3-nitrophényl;
- R² est 2-nitrophényl, 2-hydroxyméthyl-phényl, 2-méthoxyphényl, 2-cyanophényl ou pyridin-2-yl;
- R³ est H;
- R⁵ est C(O)N(R⁸, R⁹) où R⁸ est un atome d'hydrogène, et R⁹ est un atome d'hydrogène, ou CH₂R¹⁰, où R¹⁰ est un atome d'hydrogène, (C₁C₂)-alkyl, hydroxy-(C₁-C₂)-alkyl ou méthoxy-(C₁-C₂)-alkyl.

11. Un composé selon la revendication 10, **caractérisé en ce que**
- R¹ est un radical 3,5-difluorophényl, pyridin-2-yl ou pyridin-3-yl;
- R² est 2-nitrophényl ou 2-hydroxyméthyl-phényl;
- R⁵ est C(O)N(R⁸,R⁹), où R⁸ est un atome d'hydrogène, et R⁹ est CH₂R¹⁰, où
- R¹⁰ est un atome d'hydrogène ou un radical (C₁-C₂)-alkyl.

12. Un composé selon la revendication 11, consistant en l'acide 1-(3,5-difluorobenzyl)-3-(2-nitrophénylsulfanyl)-1H-indole-6-carboxylique méthylamide.

13. Un composé selon la revendication 4, **caractérisé en ce que**
- X est S;
- R¹ est un radical 3,5-difluorophényl, pyridin-2-yl, pyridin-3-yl, 3-fluorophényl, 3-cyanophényl ou 3-nitrophényl;
- R² est un radical 2-nitrophényl, 2-hydroxyméthyl-phényl, 2-méthoxyphényl, 2-cyanophényl ou pyridin-2-yl;
- R³ est H;
- R⁵ est C(O)N(R⁸,R⁹), où R⁸ et R⁹ chacun, indépendamment l'un de l'autre, sont un atome d'hydrogène, ou CH₂R¹⁰, où R¹⁰ est un atome d'hydrogène, (C₁-C₅)-alkyl, (C₁-C₅)-alkényl, hydroxy-(C₁-C₃)-alkyl, (C₁-C₃)-alkoxy-(C₁-C₃)-alkyl ou [mono- ou di-(C₁-C₄)-alkyl)]-aminométhyl.

14. Un composé selon la revendication 13, **caractérisé en ce que**
- R¹ est un radical 3,5-difluorophényl, pyridin-2-yl ou pyridin-3-yl;
- R² est un radical 2-nitrophényl ou 2-hydroxyméthyl-phényl;
- R⁵ est C(O)N(R⁸,R⁹), où R⁸ et R⁹ chacun, indépendamment l'un de l'autre, sont un atome d'hydrogène, ou CH₂R¹⁰, où R¹⁰ est un atome d'hydrogène, (C₁-C₅)-alkyl, hydroxy-(C₁-C₃)-alkyl ou (C₁-C₃)-alkoxy-(C₁-C₃)-alkyl.

15. Un composé selon la revendication 14, consistant en l'acide 1-(3,5-Difluorobenzyl)-3-(2-nitrophénylsulfanyl)-1H-indole-6-carboxylique diméthylamide.

16. Le composé selon l'une quelconque des revendications 1 à 15 pour un usage en thérapie.

17. Une composition pharmaceutique comprenant un composé selon l'une quelconque des revendications 1 à 15 et un support pharmaceutiquement acceptable.

18. Une composition pharmaceutique selon la revendication 17 pour le traitement d'une affection du groupe formé par les désordres provoqués par les récepteurs androgènes, désordres provoquées par les androgènes et insuffisances d'androgènes.

19. L'utilisation d'un composé selon l'une quelconque des revendications 1 à 15 pour la fabrication d'un médicament pour le traitement des désordres provoqués par les récepteurs androgènes, des désordres provoquées par les androgènes et les insuffisances d'androgènes.
